# EUROPEAN PATENT APPLICATION

(11) **EP 4 821 507 A1**
(43) Date of publication of application: **14.10.2026**
(21) Application number: 25382366.0
(22) Date of filing: 08.04.2025
(51) Int. Cl.: A61K 40/11, A61K 40/31, A61K 40/42

(54) **CAR-T CELLS WITH IMPROVED ANTI-CANCER PROPERTIES**

(71) Applicant: Fundació de Recerca Clínic Barcelona-Institut d'Investigacions Biomèdiques August Pi I Sunyer, 08036 Barcelona (ES)
(72) Inventor: BARBAO CARRASCO, Paula, 08036 Barcelona (ES); GUEDÁN CARRIÓ, Sonia, 08036 Barcelona (ES)
(74) Representative: Hoffmann Eitle

(57) **Abstract**

The present invention refers to an immune T cell, or a population thereof expressing an antigen receptor, wherein the antigen receptor comprises an antigen-binding domain, and the immune T cell has been modified to have reduced or eliminated the function and/or the expression of the one or more of the target genes ZC3H12C, UCP3, TG, or ITGB8, or of the proteins they encode.

## Description

### TECHNICAL FIELD

The present invention provides CAR-T cells with improved anti-cancer properties. In particular, the present invention provides CAR-T cells that target different cancer types and uses thereof.

### BACKGROUND ART

Chimeric antigen receptor (CAR) T cell therapy has shown sustained complete responses in a subset of patients with relapsed/refractory hematologic malignancies. The first clinical applications of CAR T cell therapy were approved in 2017 by the US Food and Drug Administration (FDA), directed to CD19 to treat B-cell acute lymphoblastic leukemia (ALL) and diffuse large B-cell lymphoma (DLBCL). Other CAR T cells products against CD19 and B-cell maturation antigen (BCMA) have been approved since then.

However, although CAR-T therapy is a valuable tool against cancer, various multifactorial factors still contribute to CAR-T cell failure. A common characteristic of treatment failure is limited proliferation and persistence of the CAR-T cells. This may be attributed partially to the pre-existing exhausted state of manufactured T cells, acquisition of CAR T-cell hypofunction after infusion, or a reduction in stem cell memory/central memory differentiation and premature senescence. In some cases, tumor cells lose or downregulate antigen expression to escape and evade CAR-T cell recognition. In other cases, chronic exposure to high levels of antigen expressed by the tumor cells leads to a state of T cell exhaustion.

Compared to haematological malignancies, CAR-T therapy encounters additional hurdles in solid tumors. First, CAR-T cells must arrive at tumor sites and penetrate stromal barriers to execute tumor-specific cytotoxicity. Supposing that trafficking and infiltration are successful, the toxic tumor microenvironment comprising metabolic alterations, inhibitory soluble factors and cytokines, increased frequencies of suppressive immune cells that secrete these mediators and overexpress ligands for negative immune checkpoint receptors causes T cells to become dysfunctional.

To improve the survival and function of CAR-T cells, approaches such as optimization of CAR constructs (scFv or costimulatory endodomains), prevention of tonic signaling, inhibition of transcription factors driving T cell exhaustion (such as NR4A or TOX) and overexpression of c-Jun have been developed. A successful approach to maintain T cell function has been checkpoint blockade, and inhibitors that target negative T cell regulators like CTLA-4, PD-1, LAG-3 and TIM-3 are being investigated in the clinical setting to prevent or ameliorate exhaustion.

Since intermittent CAR T-cell 'rest' mitigates exhaustion and enhances antitumor potency in association with epigenetic reprogramming, engineering loss-of-function or modulation of genetic and epigenetic targets that potentiate T-cell exhaustion using CRISPR-Cas9 technology has the potential to prevent and perhaps even reverse CAR T-cell dysfunction (Dimitri, Herbst and Fraietta, 2022 doi: 10.1186/s12943-022-01559-z). The first-in-human trial of multiplex CRISPR-Cas9 edited (i.e., TRAC, TRBC and PDCD1 knockout) T-cells with a transgenic TCR specific for tumor-associated antigen NY-ESO-1 in patients with myeloma and sarcoma was conducted, demonstrating the feasibility and short-term safety of treating-patients with CRISPR-edited human T-cells.

However, despite the strategies mentioned above, there is still an urgent need to improve the anti-tumor effectiveness of CAR-T cell therapies thereby avoiding T cell dysfunction.

### BRIEF DESCRIPTION OF THE DRAWINGS

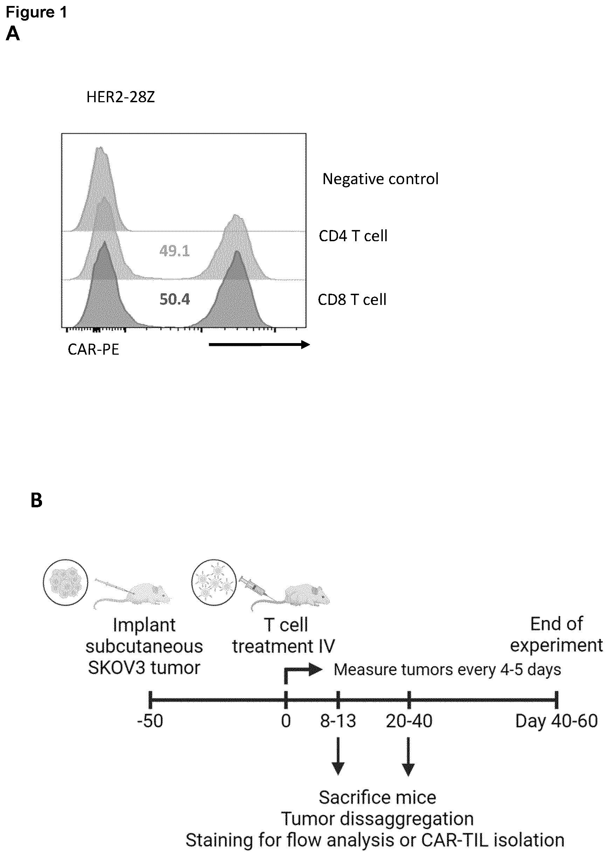

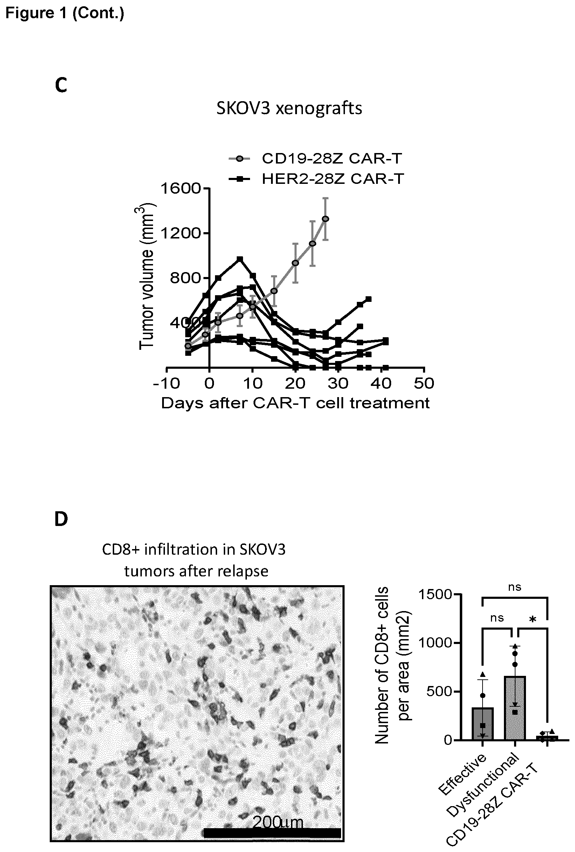

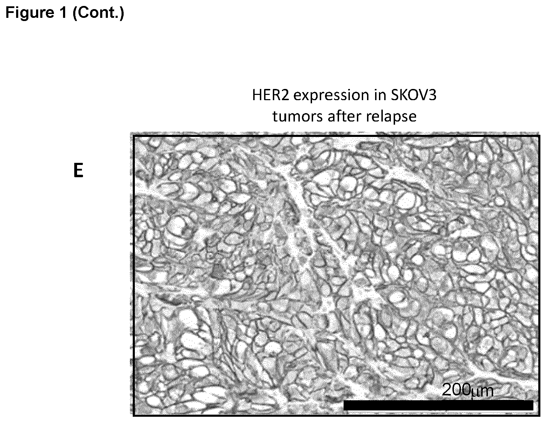

**Figure 1****. Xenograft tumors escape to HER2-28Z CAR-T therapy despite infiltration and antigen persistence in SKOV3 solid tumors. A)** Flow cytometry analysis showing CAR expression by representative histogram. The percentage of CAR-positive cells for CD4+ (light color) and CD8+ (dark color) is indicated. Untransduced T cells were used as negative control. **B)** Diagrammatic illustration of the experimental design followed for the execution of *in vivo* experiments to test antitumor effect of anti-HER2-CAR-T against SKOV3 xenografts. **C)** NSG mice bearing subcutaneous SKOV3 tumors were treated with a single dose of 2x10⁶ anti-HER2 CD28-based CAR+ T cells or control anti-CD19 CD28-based CAR+ T cells (Control CAR-T). Data is plotted as mean ±SEM of tumor volume or as individual tumors. Representative of >5 experiments using different healthy donors. D) IHC analysis for SKOV3 tumors treated with anti-HER2 CAR-T cells showed CD8+ infiltration after tumor escape. Scale bar, 200µm (left). The number of CD8+ per area of tumor was quantified from brightfield images using QuPath software. Data shows mean ±SD of positive cells in tumors treated with HER2-CAR-T cells at effective and dysfunctional phase, or control CAR-T cells. N=4 different healthy donors. *p<0.05 by One-way ANOVA with Tukey's multiple comparisons test. E) Persistence of HER2 antigen in tumors after relapse was analyzed using IHC. Scale bar, 200µm.

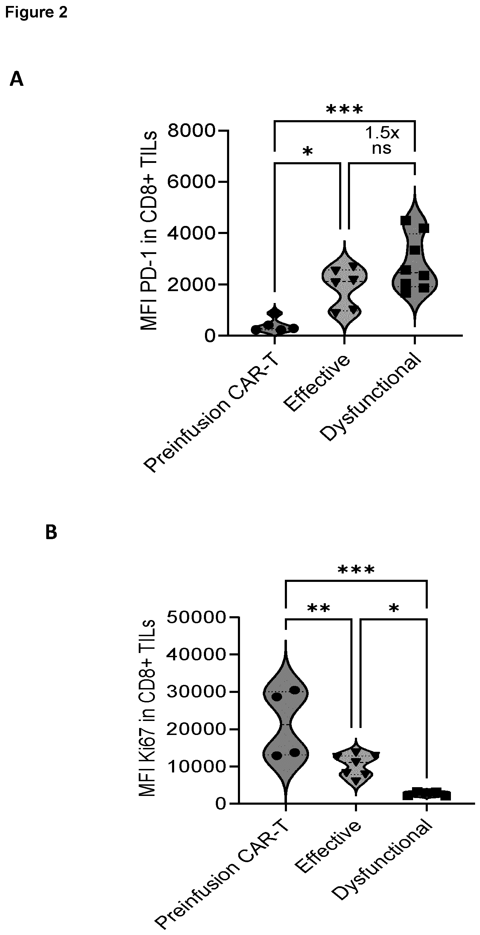

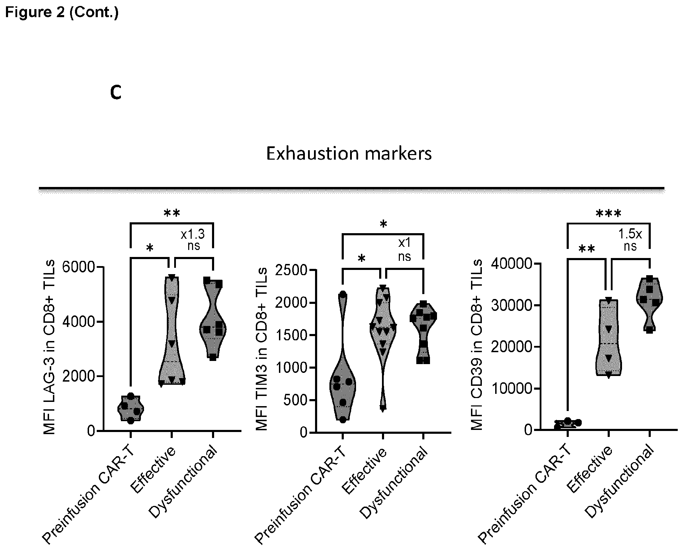

**Figure 2****. Dysfunctional CAR-TILs maintain PD-1 expression but lose Ki67 proliferation marker. A-C)** Violin plots showing min to max the mean fluorescence intensity (MFI) of PD-1 **(A),** Ki67 **(B)** and exhaustion markers **(C)** expression in tumor infiltrating CAR-T cells at effective and dysfunction phase compared to preinfusion anti-HER2-CAR-T cells. Each dot represents a tumor, N=4-5 in **(A** and **B)** and N=3-6 in **(C)** healthy donors. *p<0.05, **p<0.01, ***p<0.001 by one-way ANOVA and Tukey's multiple comparisons test. Fold change of expression between dysfunctional and effective is indicated.

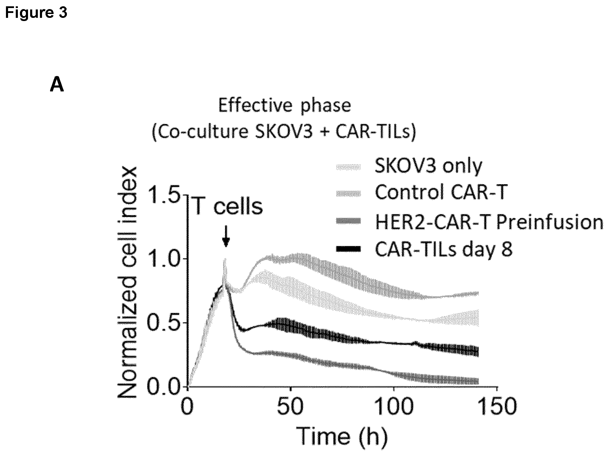

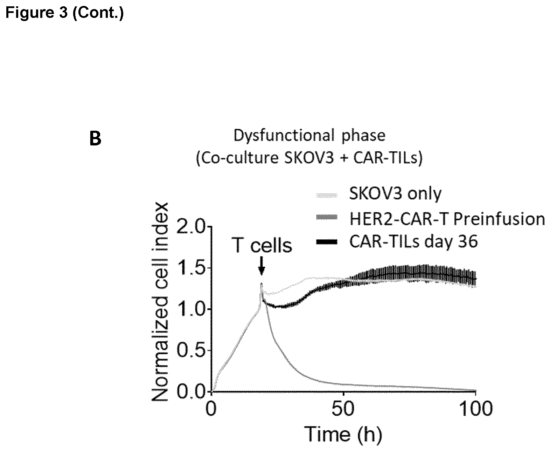

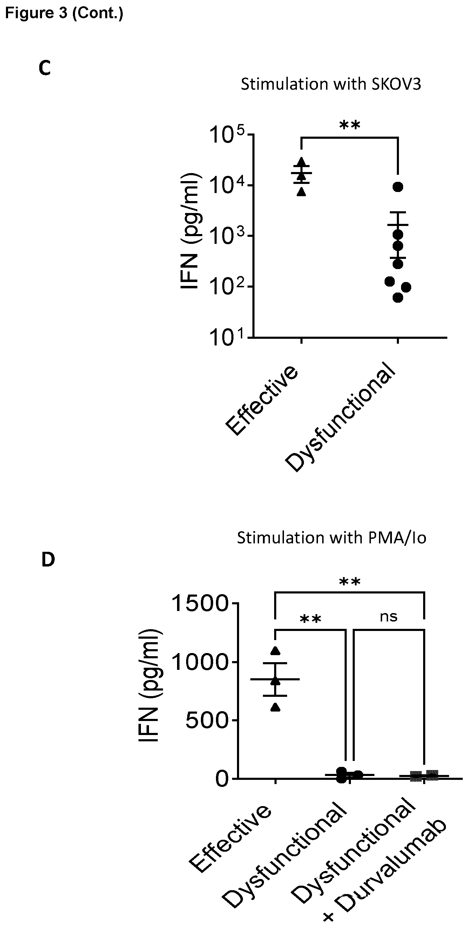

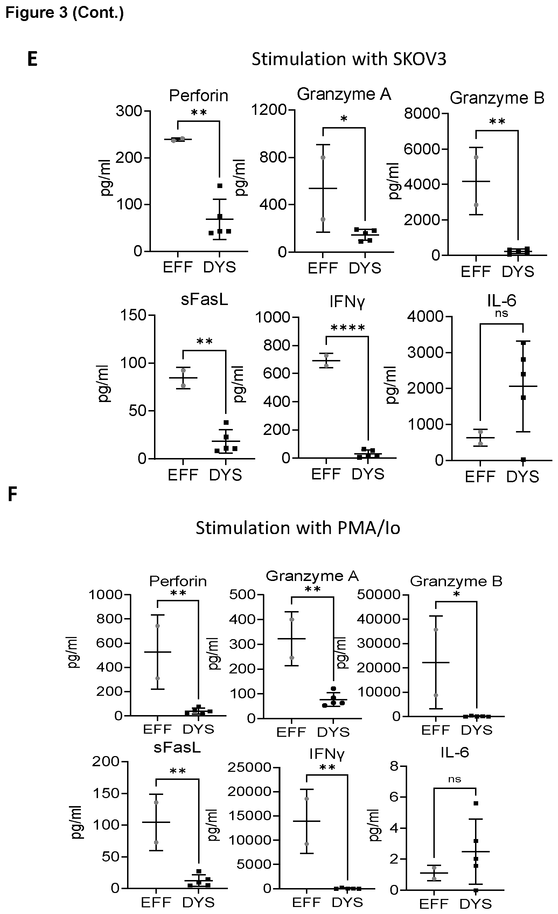

**Figure 3****. CAR-TILs at dysfunctional phase display impaired cytotoxic activity and cytokine production against SKOV3 tumor cells.** CAR-TILs were isolated from tumors on day 8 (effective phase) and days 25-40 (dysfunctional phase for downstream analysis. **A-B)** Real-time cytotoxicity of isolated CAR-TILs at effective **(A)** and dysfunctional **(B)** phases was *ex vivo* analyzed using xCELLingence against SKOV3 tumor cells compared to preinfusion CAR-T cells. Data shows mean ±SEM of normalized cell index. Time when T cells were added is indicated by an arrow. Tumor cells or irrelevant CAR-T cells (CD19-CAR-T cells) were used as negative control. **C)** Analysis of IFN-γ production by effective and dysfunctional CAR-TILs after *in vitro* co-culture with SKOV3 cells. Data shows mean ±SEM of IFN-γ production. Each dot represents a tumor used for isolating CAR-TILs. N=3 healthy donors. **p<0.01 by unpaired T-test. **D)** Analysis of IFN-γ production by CAR-TILs during CAR-independent stimulation using PMA/lonomycin (PMA/lo). The PD-1/PD-L1 axis was inhibited by addition of PD-L1 blocking antibody Durvalumab. Data shows mean ±SEM. Each dot represents a tumor used for isolating CAR-TILs N=3 healthy donors for effective and dysfunctional, N=2 healthy donors for dysfunctional + durvalumab. **p<0.01 by unpaired T-test. **E-F)** Cytokine release by (EFF) and dysfunctional (DYS) CAR-TILs was measured after 24-h of co-culture with SKOV3 tumor cells (E) or after overnight stimulation with PMA/lo **(F)** using LegendPlex. Data are mean ±SD of 2 healthy donors. Each dot represents a tumor. *p<0.05, **p<0.01, ****p<0.0001 by unpaired T-test.

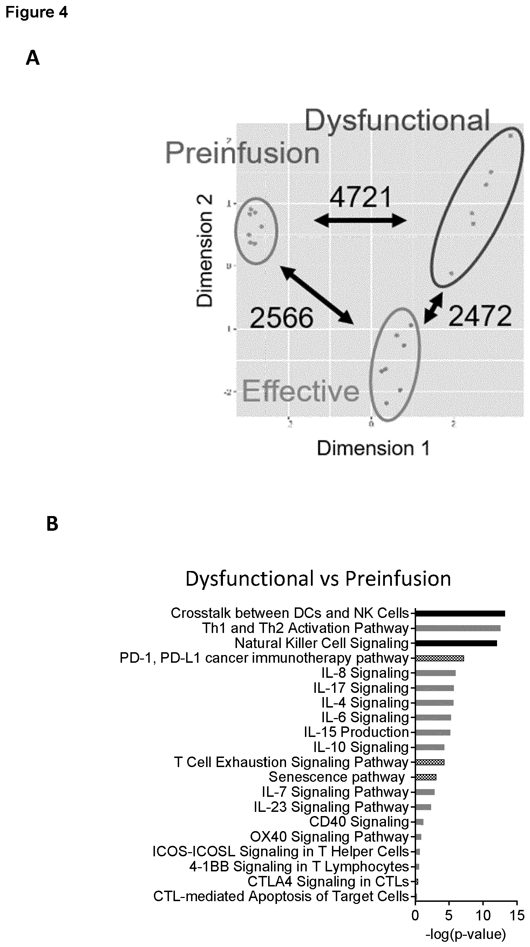

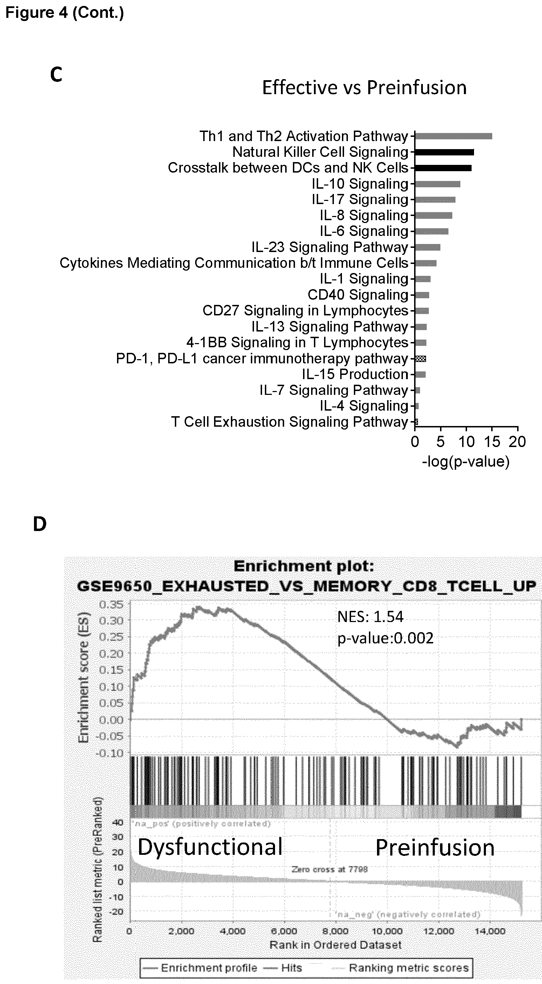

**Figure 4****. RNA sequencing reveals distinct transcriptomic profiles across effective and dysfunctional CAR-TILs. A)** Principal component analysis of preinfusion, effective and dysfunctional CAR-TILs samples included in the RNA-seq. Number of differentially expressed genes between CAR-TILs identified using limma software is indicated (p-adjusted value<0.05 and logFc<1 or logFc<-1). Preinfusion N= 7 samples from 7 healthy donors; Effective N= 7 samples from 6 healthy donors; Dysfunctional N=6 samples from 5 healthy donors. **B-C)** Upregulated genes from differential expression analysis (p-adjusted value<0.05 and logfc>1) from dysfunctional vs preinfusion **(B)** and effective vs preinfusion **(C)** comparisons were used to perform Ingenuity Pathway Analysis (IPA). For each gene set of upregulated genes, selected pathways were ranked by -log (p-value) of enrichment. Black bars denote NK cell-related pathways, patterned bars denote pathways of T cell exhaustion and grey bars indicate other enriched pathways.**D**) GSEA plot showing upregulation of exhausted vs memory CD8 T cell gene signature in CAR-TILs at dysfunctional phase compared to preinfusion. P-value is nominal p-value given by the GSEA program.

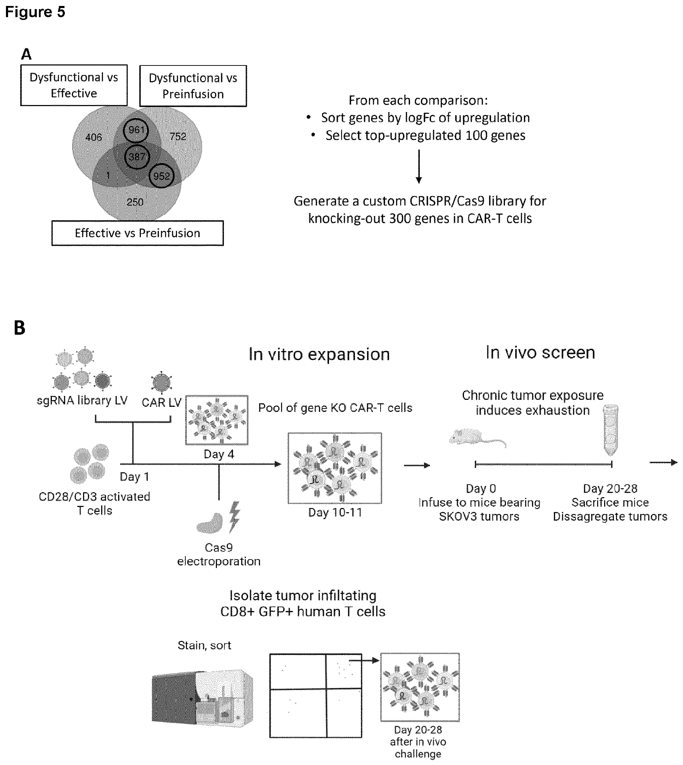

**Figure 5****. A custom CRISPR /Cas9 library was used to screen 300 candidate gene knockouts to prevent CAR-T cell dysfunction *in vivo.* A)** Overlap of upregulated genes in dysfunctional versus effective, dysfunctional versus preinfusion and effective versus preinfusion comparisons from differential expression analysis. Upregulated genes were selected in each comparison with p-adjusted value<0.05 and logFc>1. The 300 most upregulated genes between at least two comparisons were selected to generate a library of sgRNAs. **B)** A pool of CRISPR-edited HER2-28Z CAR-T cells was generated from 3 healthy donors, expanded *in vitro,* and then injected to mice bearing SKOV3 tumors for screening of gene KO enhancing CAR-T function. Amplicon-Sequencing was used to analyze enrichment and depletion of sgRNAs after *in vitro* expansion of CAR-T cells or in T cells isolated from tumors after 20-28 days, compared to T cells after transduction but before KO. Grey boxes indicate which samples were compared to identify candidate genes.

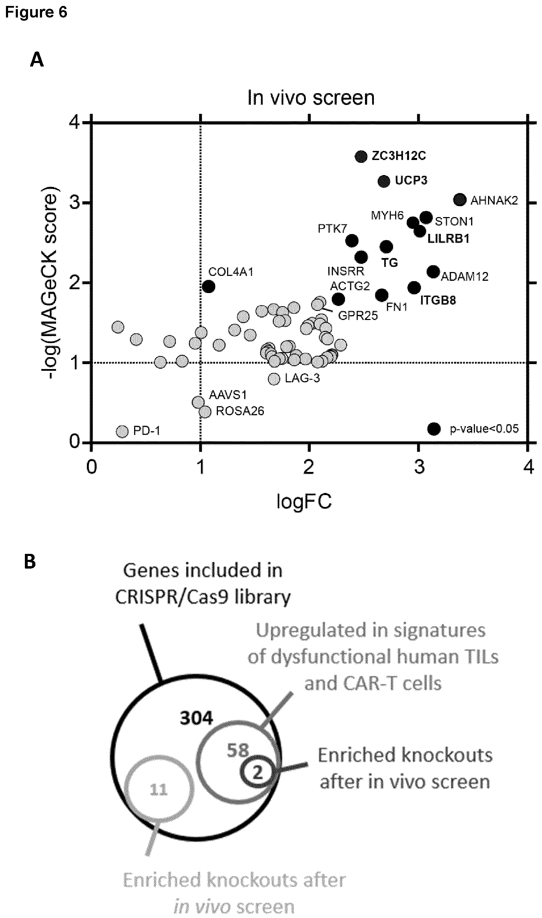

**Figure 6****. *In vivo* CRISPR-based screen identifies gene candidates with a possible role in mediating CAR-T cell dysfunction. A)** Amplicon-seq data for pooled KO CAR-T cells after *in vivo* screen was analyzed using MAGeCK algorithm to identify enriched gene knockouts in CAR-T cells after prolonged exposure to tumors compared to T cells before KO. Significantly enriched candidates were considered with -log(MAGeCK score)>1 and p-value<0.05. AAVS1, GPR25, ROSA26, PD-1 and LAG-3 are also depicted. **B)** Overlap analysis between candidate genes included in the CRISPR library and previously published signatures of dysfunctional human TILs and CAR-T cells.

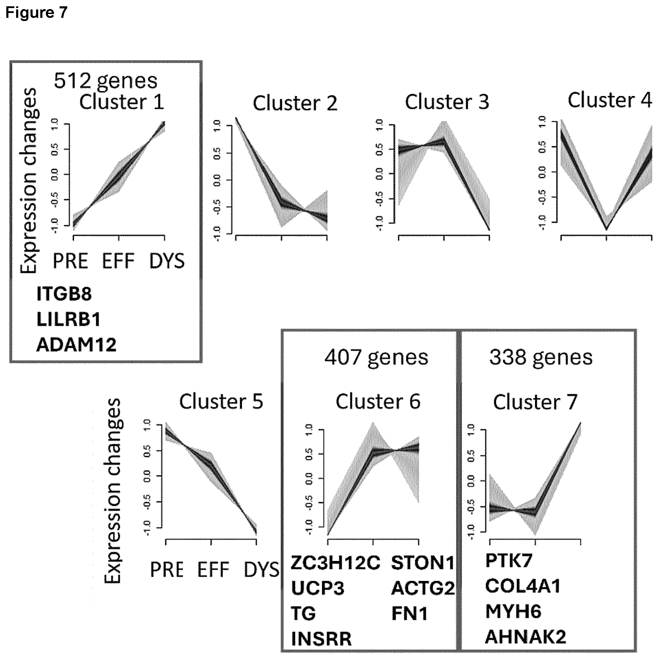

**Figure 7****. Candidate genes are categorized into three groups based on their kinetic pattern of expression in CAR-TILs.** Soft clustering was performed on log counts of RNA-sequencing data from preinfusion, effective and dysfunctional CAR-TILs, including genes with a variance>0.1. For clusters 1, 6, and 7, the total number of genes in each cluster is shown at the top, and the candidate genes are highlighted in bold.

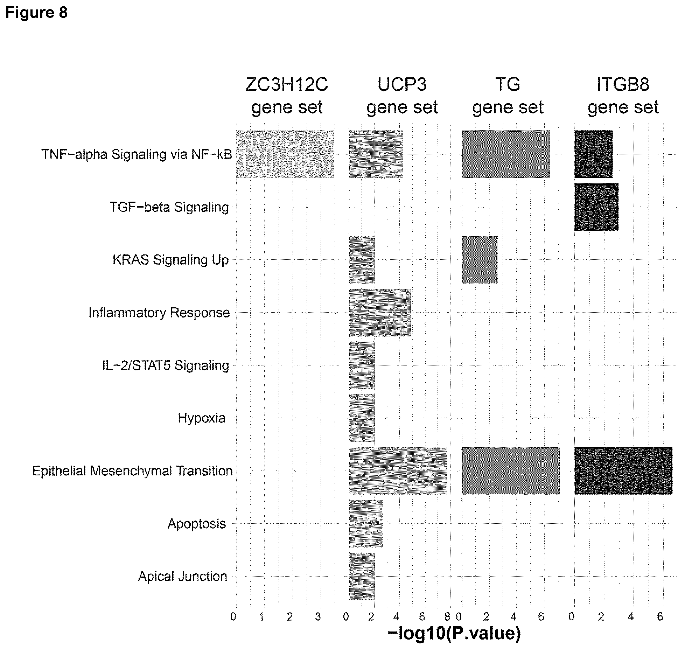

**Figure 8****. ZC3H12C, UCP3, TG and ITGB8 co-express with genes from the TNF-α signaling pathway.** Bar plots depicting the statistically significant enriched pathways obtained by over-representation analysis of the subsets of genes that co-express positively (r >0.08) and significantly (p.value <0.05) with each individual gene in the dysfunctional samples from the RNA-sequencing data. The co-expression analysis was performed using the Pearson correlation and the enrichment analysis using the enrichR package v3.2 in R with the Hallmark gene set database.

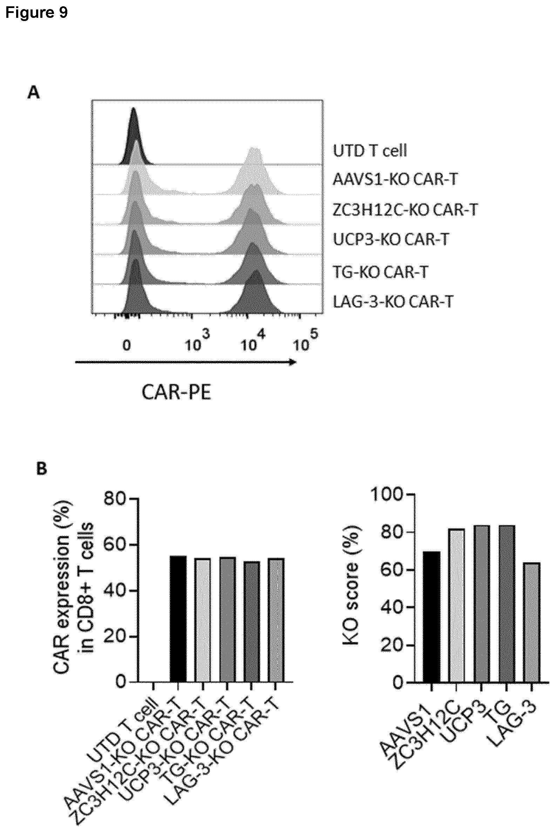

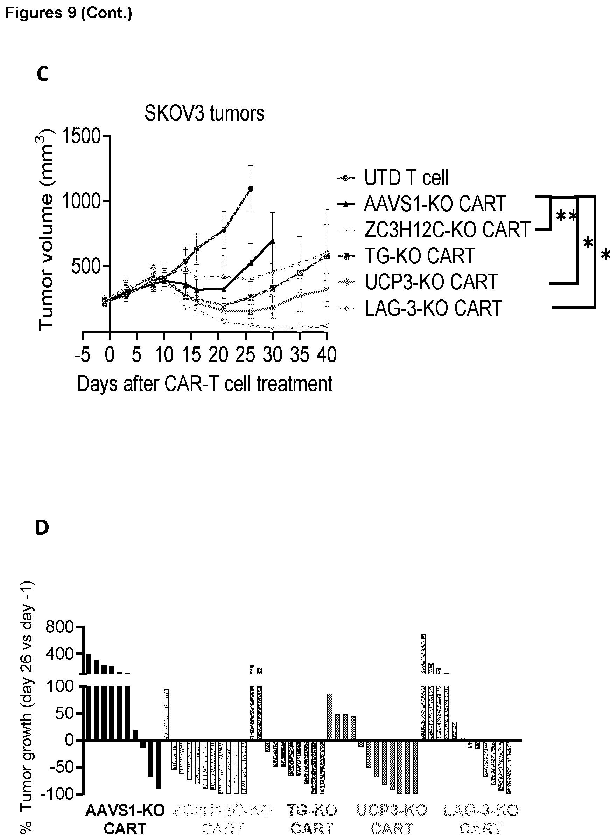

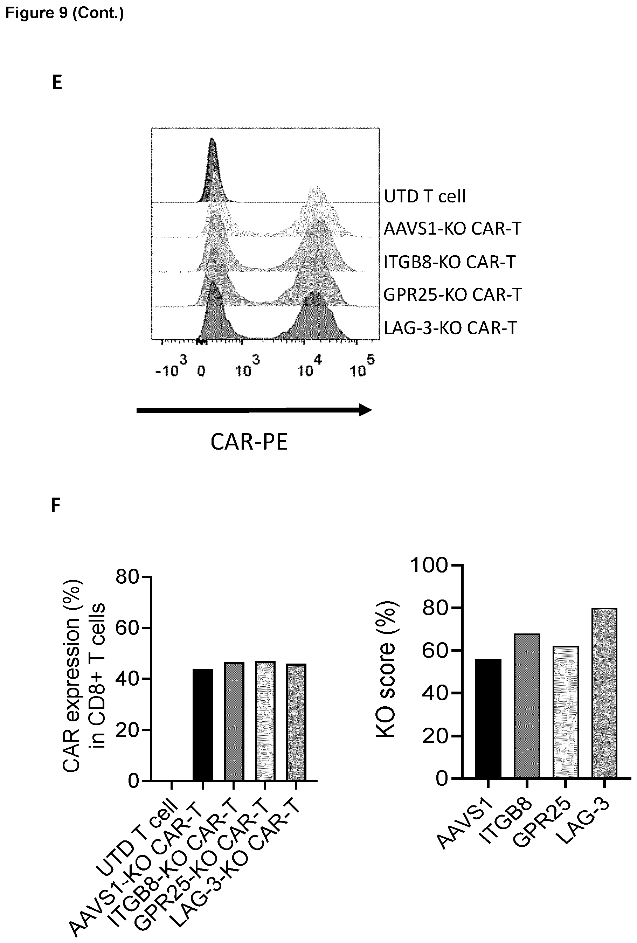

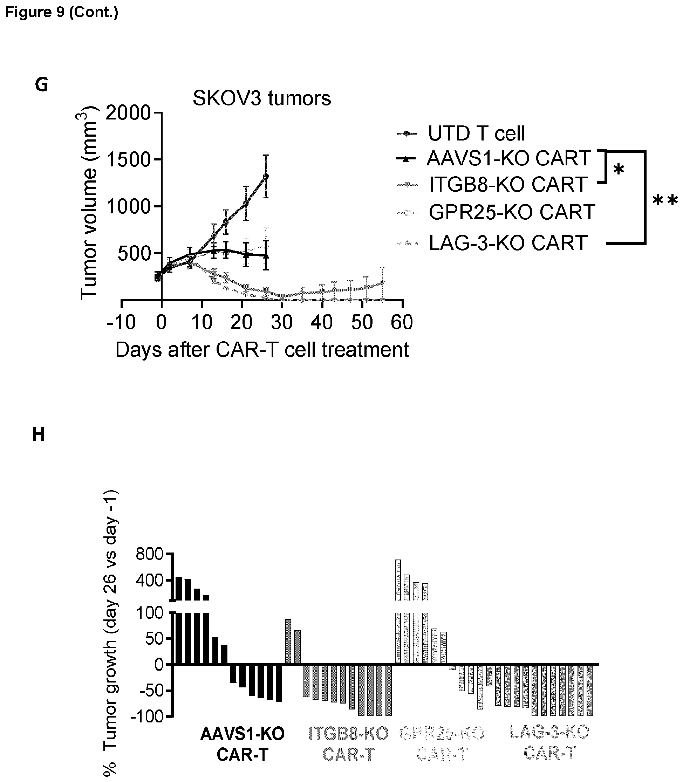

**Figure 9****. Gene KO candidates enhance antitumor efficacy of CAR-T cells in an *in vivo* model of solid tumor. A-D)** NSG bearing pre-established SKOV3 tumors were treated with a single dose of with 2x10⁶ non-transduced T cells, anti-HER2-28Z CAR-T cells KO for candidate ZC3H12C, UCP3, TG genes, or control LAG-3-KO or AAVS1-KO CAR-T cells. **A)** Flow cytometry analysis showing CAR expression by histogram and percentage in CD8+ T cells with KO for gene candidates used for *in vivo* assessment of antitumor effect. **B)** CRISPR/Cas9 efficiency represented as percentage of KO score at day 10 of T cell expansion. **C)** Tumor volume of mice treated with KO CAR-T cells was analyzed at indicated time points. **D)** Change in tumor volume on day 26 after CAR-T therapy versus baseline is plotted for individual tumors (N=10-12 tumors per group). **E-H)** In a second experiment, the anti-tumor efficacy of ITGB8-KO CAR-T was evaluated as in (A-D). GPR25, LAG-3 or AAVS1-KO CAR-T cells were added as controls. Representative flow cytometry analysis of CAR-T cells used for this experiment showing histogram of CAR expression, percentage of CAR expression **(E)** and KO score **(F),** tumor volume of treated mice at different timepoints **(G)** and change in tumor volume at day 26 after CAR-T therapy treatment (N=10-12 tumors per group) **(H). C** and **G)** Data are plotted as mean tumor volume ±SEM (N=10-12 tumors per group). *p<0.05, **p<0.01 by two-way ANOVA and Turkey comparison test at day 26 after CAR-T therapy.

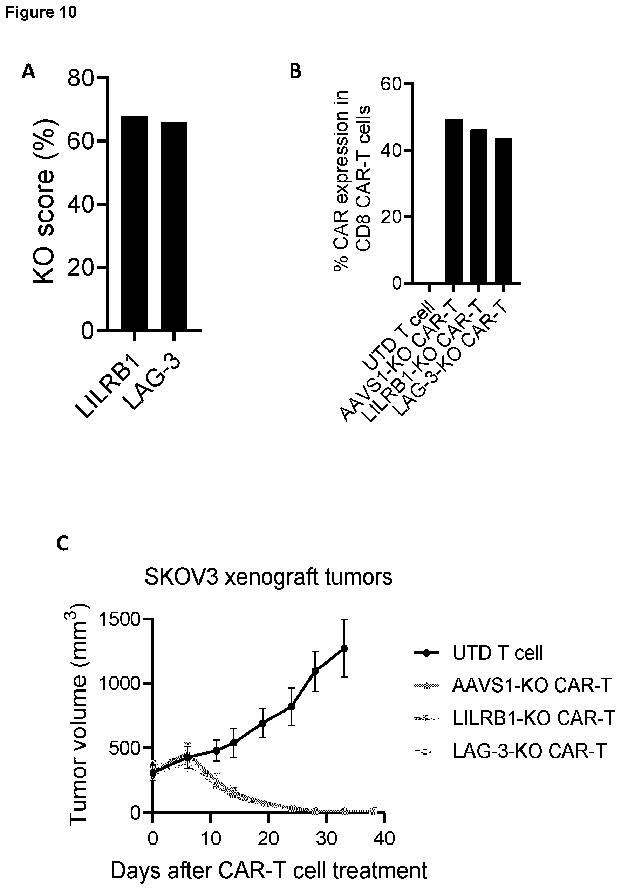

**Figure 10****. LILRB1-KO CAR-T cells show high antitumor efficacy in an *in vivo* model of solid tumor.** NSG bearing pre-established SKOV3 tumors were treated with a single dose of with 2x10⁶ non-transduced T cells (UTD), anti-HER2-28Z CAR-T cells KO for candidate LILRB1, or control LAG-3-KO or AAVS1-KO CAR-T cells. **A)** CRISPR/Cas9 efficiency represented as percentage of KO score on CD8+ CAR-T cells used in the *in vivo* experiment. **B)** Percentage of CAR expression analyzed in gene-edited CAR-T cells on day 9 after primary *in vitro* expansion on CD8+ T cell population. **C)** Tumor volume of mice treated with KO CAR-T cells was analyzed at indicated time points. Data are plotted as mean tumor volume ±SEM (N=1 healthy donor, 6-10 tumors per group).

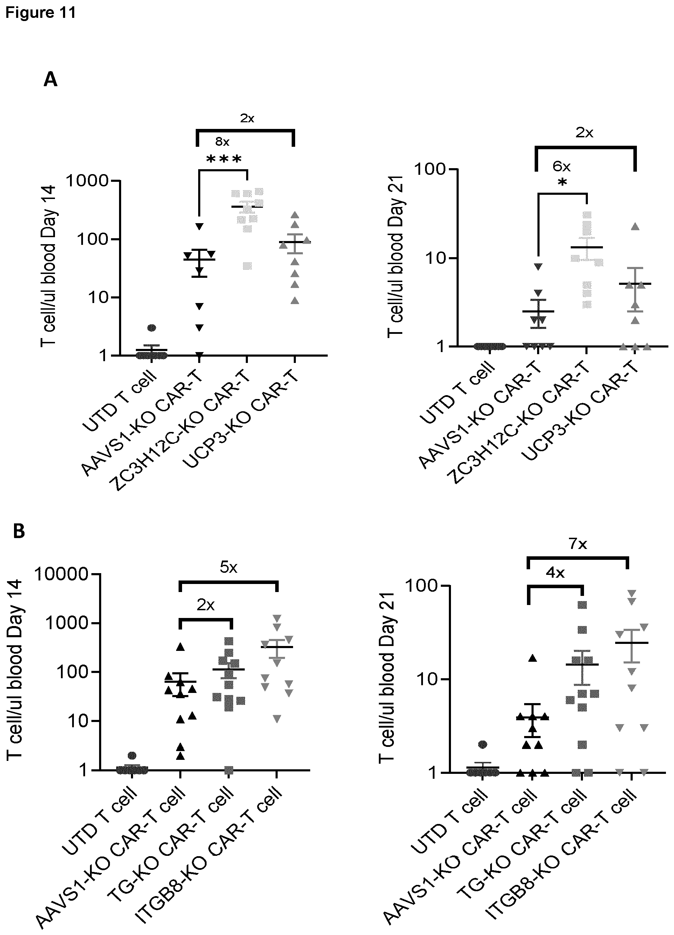

**Figure 11****. Genetic deletion of candidate genes ZC3H12C, UCP3, TG and ITGB8 improves T cell persistence in mice with SKOV3 tumors. A-B)** The concentration of total T cells was determined in the blood of animals treated with ZC3H12C-KO, UCP3-KO **(A),** TG-KO, ITGB8-KO **(B),** control AAVS1-KO CAR-T or UTD T cells at day 14 and 21 after T cell injection. Data is plotted as mean ± SEM. Each dot represents a mouse. Fold change compared to AAVS1-KO group is indicated. *p<0.05; ***p<0.001 by one-way ANOVA with Tukey's multiple comparisons test.

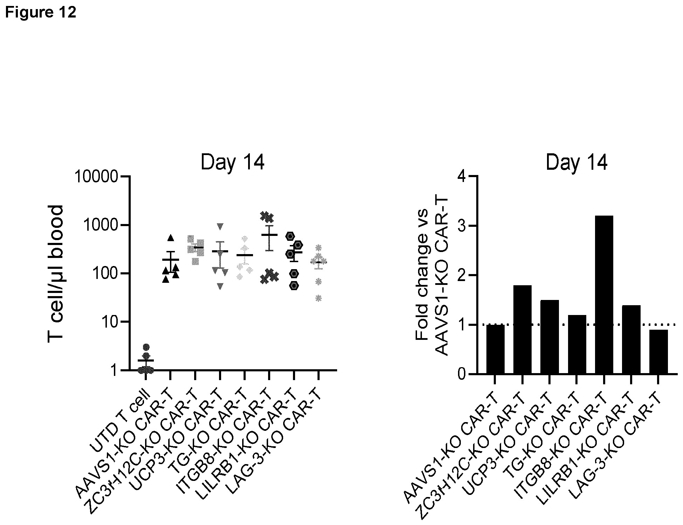

**Figure 12****. Validation of T cell persistence of candidate genes ZC3H12C, UCP3, TG, ITGB8 and LILRB1 in an *in vivo* model of L55-HER2t+ xenograft tumors.** NSG mice bearing subcutaneous L55-HER2t tumors were treated with a single dose of 4x10⁶ ZC3H12C-KO, UCP3-KO, TG-KO, ITGB8-KO, LILRB1-KO, LAG-3-KO or AAVS1-KO HER2 CAR+ T cells, or untransduced T cells (UTD) as control. Blood was collected on day 14 and T cell persistence was analyzed as concentration (left panel) or as fold change compared to AAVS1-KO CAR-T control (right panel).

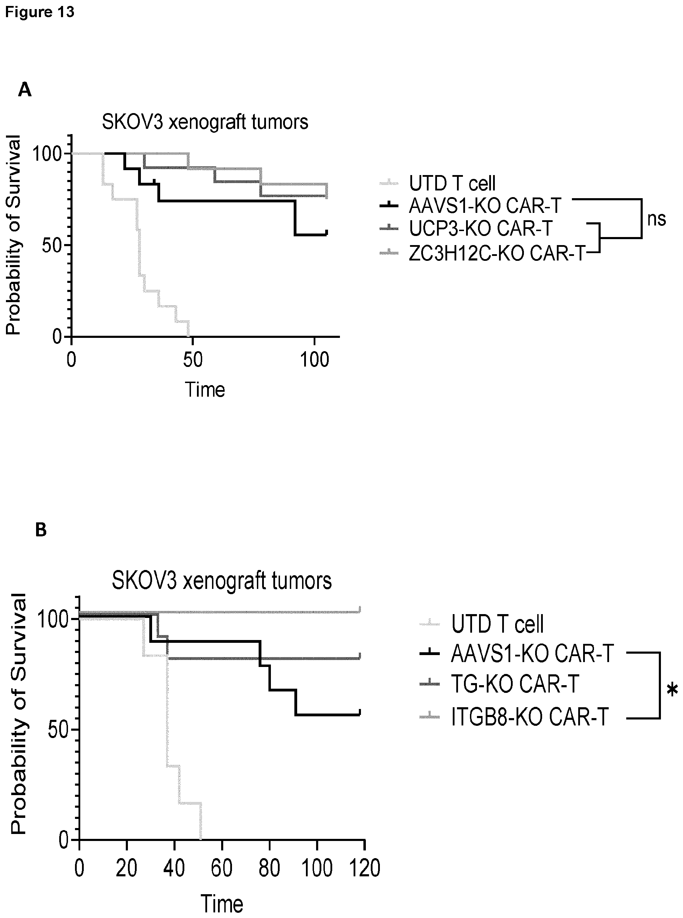

**Figure 13****. Genetic deletion of candidate genes ZC3H12C, TG and ITGB8 improves survival of mice with SKOV3 tumors. A-B)** NSG mice bearing SKOV3 xenograft tumors were treated with a single dose of ZC3H12C-KO, UCP3-KO, AAVS1-KO CAR-T cells or UTD T cells (A) and TG-KO, ITGB8-KO, AAVS1-KO CAR-T cells or UTD T cells **(B).** Animals were sacrificed when tumor volume reached 1500mm³. **A)** Shows 2 independent experiments using 3 healthy donors (N=9-12 tumors per group). **B)** Shows one experiment using 2 healthy donors. (N=9-10 tumors group) *p<0.05, by log-rank Mantel-Cox test (N=9-10).

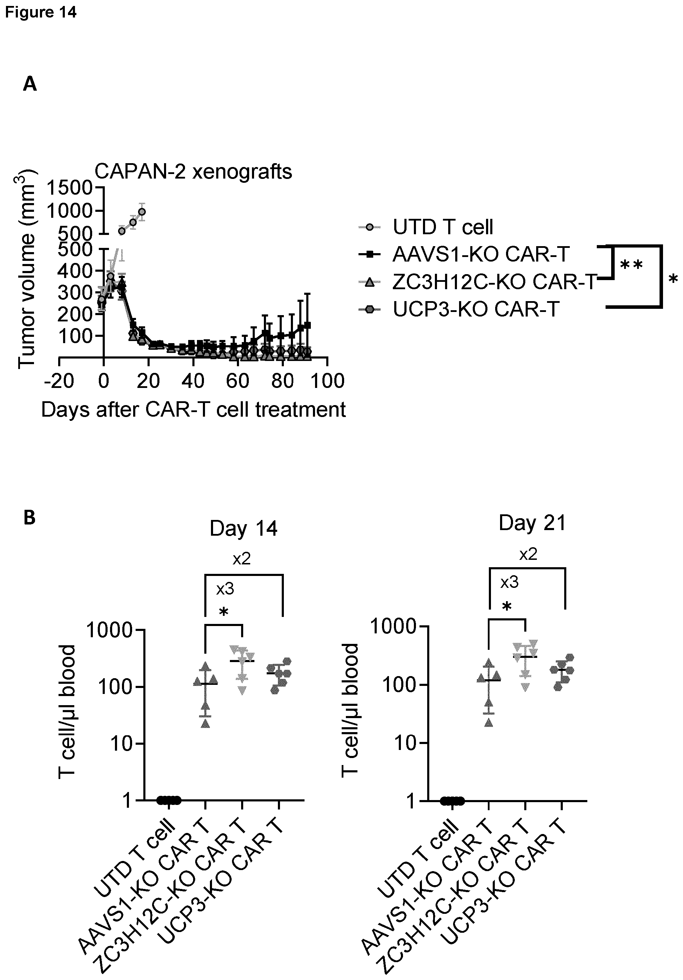

**Figure 14****. Gene KO candidates CAR-T eliminate pancreatic xenografts. A)** NSG mice bearing pre-established CAPAN-2 tumors were treated with a single dose of 2x10⁶ anti-Mesothelin-28Z CAR+ T cells with genetic ablation of ZC3H12C, UCP3 or for the safe harbour AAVS1. Data are plotted as mean ± SEM tumor volume (N=2 healthy donors, 8-10 tumors per group). *p<0.05, **p<0.01 by two-way ANOVA with Tukey's multiple comparisons test. **B)** The concentration of total T cells was determined in the blood of animals treated with ZC3H12C-KO, UCP3-KO or control AAVS1-KO CAR-T cells at day 14 (left panel) and day 21 (right panel). Data are plotted as mean ±SD (N=1 healthy donor, 4-5 animals per group). *p<0.05 by one-way ANOVA with Tukey's multiple comparisons test.

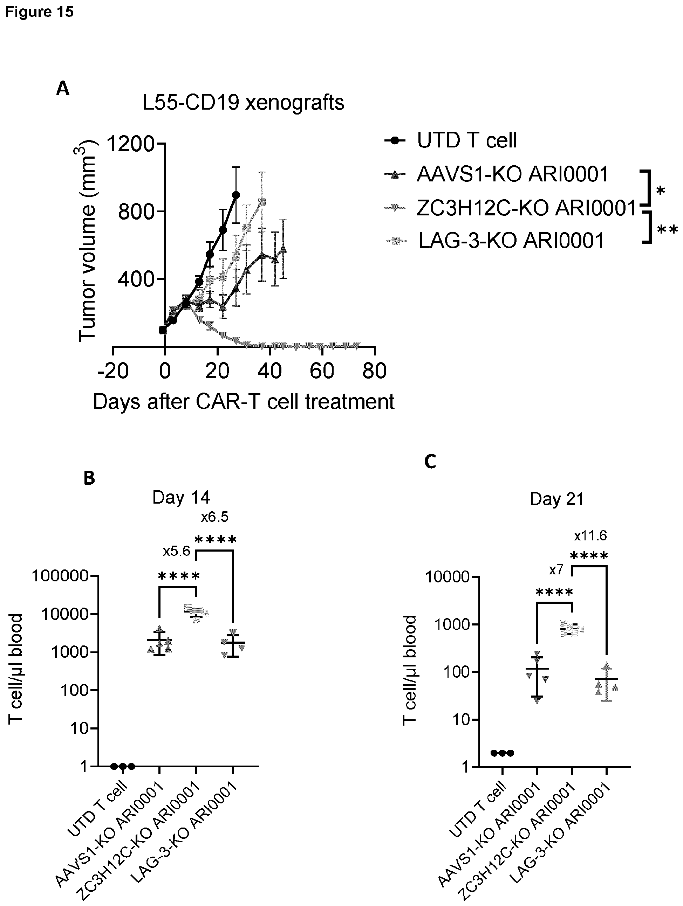

**Figure 15****. The genetic ablation of ZC3H12C enhances ARI0001 CAR-T antitumor effect and persistence in an *in vivo* model of lung xenografts overexpressing CD19.**
   **A)** NSG mice bearing subcutaneous L55-CD19 tumors were treated with a single dose of 4x10⁶ ZC3H12C-KO, LAG-3-KO, AAVS1-KO ARI0001 CAR+ T cells or untransduced T cells (UTD) as control. Tumors were measured at indicated time-points. Data are plotted as mean tumor volume ±SEM (N=1 healthy donor, 8-10 tumors per group). *p<0.05; **p< 0.01 by two-way ANOVA with Tukey's multiple comparisons test. **B-C)** The concentration of total T cells was determined in the blood of animals treated with ZC3H12C-KO, LAG-3-KO or control AAVS1-KO ARI0001 CAR-T cells at day 14 **(B)** and day 21 **(C).** Fold change compared to AAVS1-KO and LAG-3-KO group is indicated. Data are plotted as mean ±SD (N=1 healthy donor, 8-10 tumors per group). ****p<0.0001 by one-way ANOVA with Tukey's multiple comparisons test.

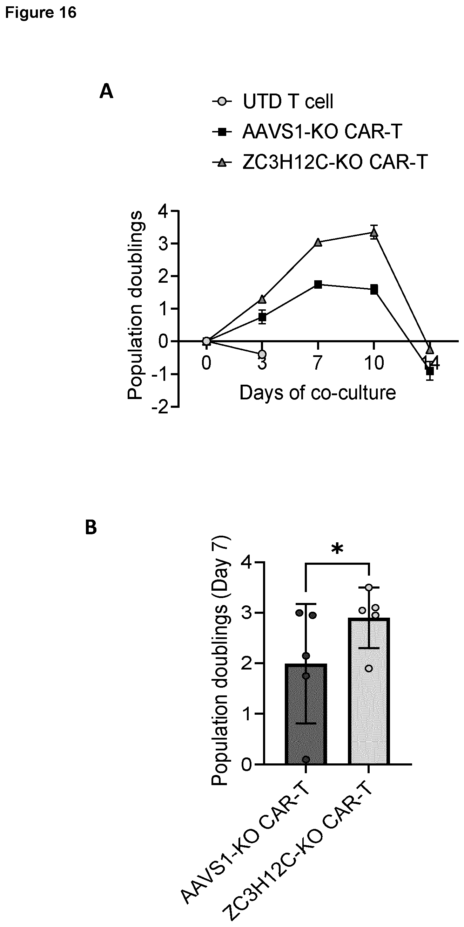

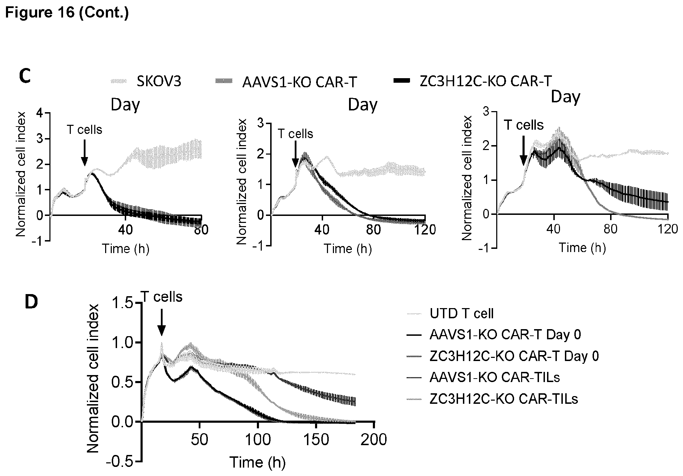

**Figure 16****. ZC3H12C-KO CAR-T cells show superior proliferation and cytotoxicity upon chronic antigen stimulation.**
   **A-C)** Control T cells (untransduced) and AAVS1- and ZC3H12C-KO CAR-T cells were *in vitro* co-cultured with HCC1954 breast tumor cells at an effector-to-target ratio of 3:1. Every 3-4 days, T cells were counted and plated with fresh tumor cells. **A)** T cell proliferation was assessed by plotting population doublings for each group overtime. Data are plotted as mean population doublings ± SD (N=1 healthy donor). Representative of 5 experiments using different healthy donors. **B)** Population doublings on day 7 of the *in vitro* restimulation assay using CAR-T cells generated from five healthy donors. Data are plotted as mean population doublings ±SD (N=5). *p<0.05 by paired T-test. C) The ability of CAR-T cells to kill HCC1954 tumor cells was assess every 7 days during the restimulation assay at an effector-to-target ratio of 1:1 using a real-time assay. Data are plotted as mean ±SD of two duplicates (N=1 healthy donor). Representative of 5 experiments using different healthy donors. **D)** NSG animals bearing SKOV3 tumors were treated with ZC3H12C-KO or AAVS1-KO CAR-T cells. CAR-TILs were isolated 19 days after treatment, and their ability to kill SKOV3 tumor cells ex *vivo* was assess using a real-time cytotoxicity assay. Day 0 ZC3H12C-KO and AAVS1-KO CAR-T cells were included as controls. Data are plotted as mean ±SD of two duplicates at an effector-to-target ratio of 1:1 using SKOV3 tumor cells as target (N=1 healthy donor).

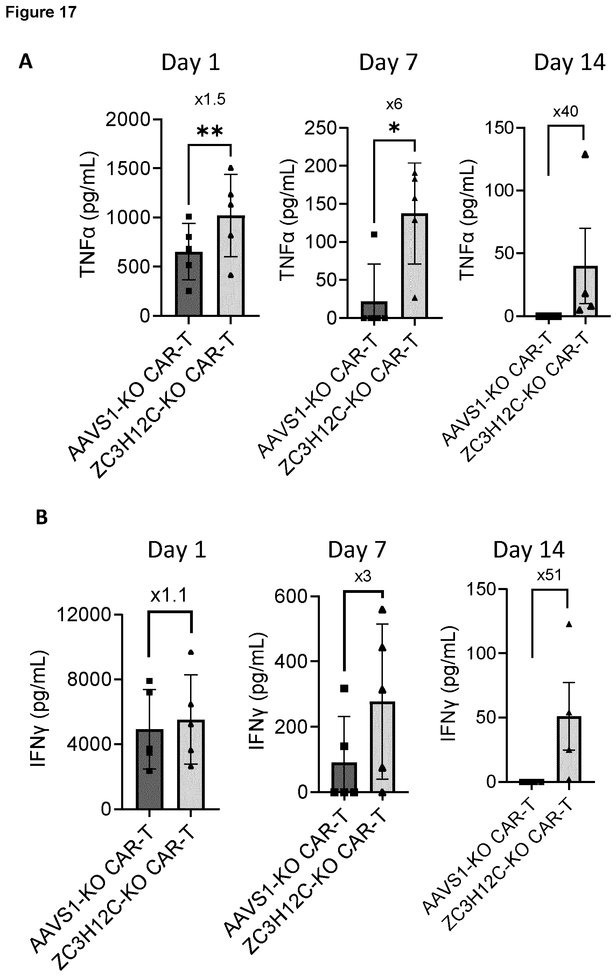

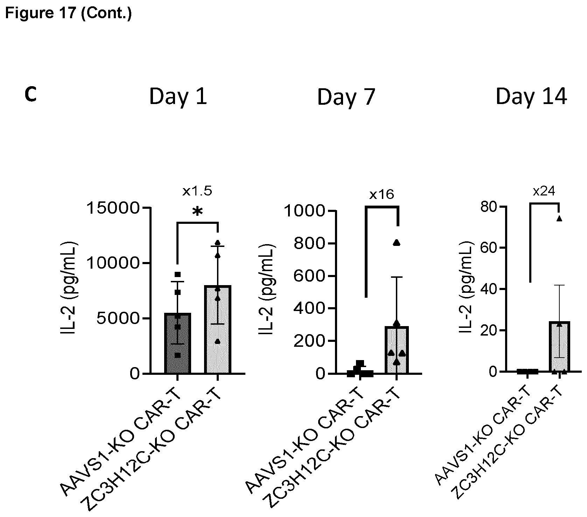

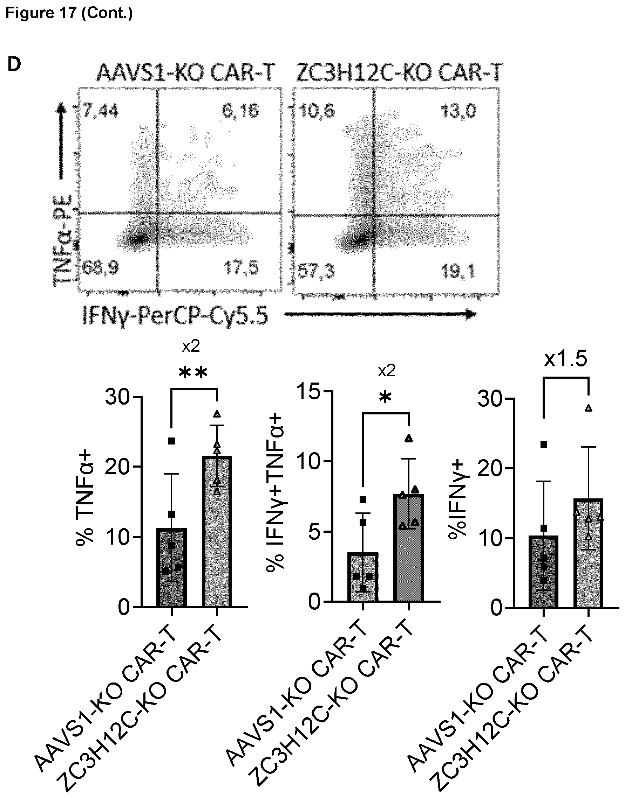

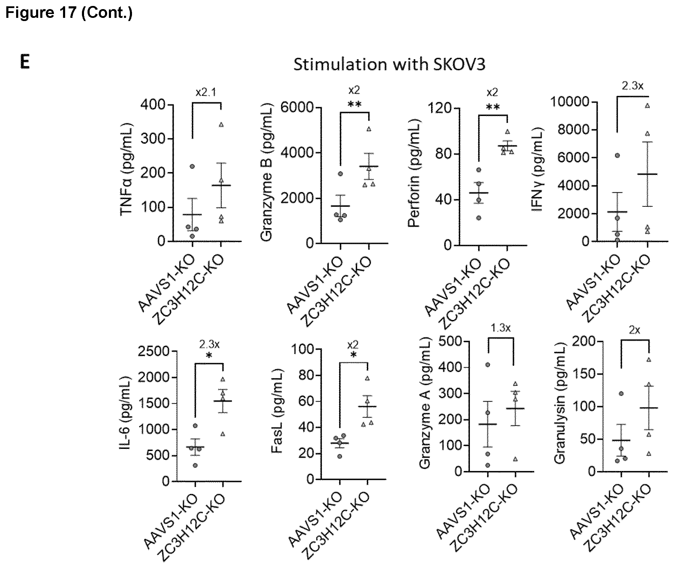

**Figure 17****. ZC3H12C-KO CAR-T cells show enhanced cytokine production upon chronic antigen stimulation.**
   Control T cells (untransduced) and AAVS1- and ZC3H12C-KO CAR-T cells were *in vitro* co-cultured with HCC1954 breast tumor cells at an effector-to-target ratio of 3:1. Every 3-4 days, T cells were counted and plated with fresh tumor cells. **A-C)** TNF-α **(A),** IFN-γ **(B)** and IL-2 (C) release by CAR-T cells was measured every 7 days during the restimulation assay in the supernatant of 24h co-cultures with fresh tumor cells by ELISA. Data are plotted as mean ±SD (N=5 healthy donors for day 1 and 7, N=4 healthy donor for day 14). *p<0.05, **p<0.01 by paired t-test. **D)** Representative flow cytometry plots of TNF-α+IFN-γ+ T-cells (gated on live/CD45+) of intracellular cytokine staining for TNF-α and IFN-γ in AAVS1-KO and ZC3H12C-KO CAR-T cells at day 7 after co-culture with HCC1954 tumor cells (left panel). Percentage of TNF-α+, IFN-γ+ or TNF-α+ IFN-γ+ T-cells (gated on live/CD45+) in AAVS1-KO or ZC3H12C-KO CAR-T cells after 7 days of restimulation with HCC1954 tumor cells *in vitro* as determined by intracellular staining. Data are plotted as mean ±SD (N=5 healthy donors). *p<0.05, **p<0.01 by paired T-test. E) NSG animals bearing SKOV3 tumors were treated with ZC3H12C-KO or AAVS1-KO CAR-T cells. CAR-TILs were isolated 19 days after treatment, and their ability to release cytokines after a 24h co-culture with SKOV3 tumour cells was analysed. Data are plotted as mean ±SEM of 4 healthy donors. *p<0.05, **p<0.01 by paired T-test. Fold change between AAVS1-KO and ZC3H12C-KO groups is indicated.

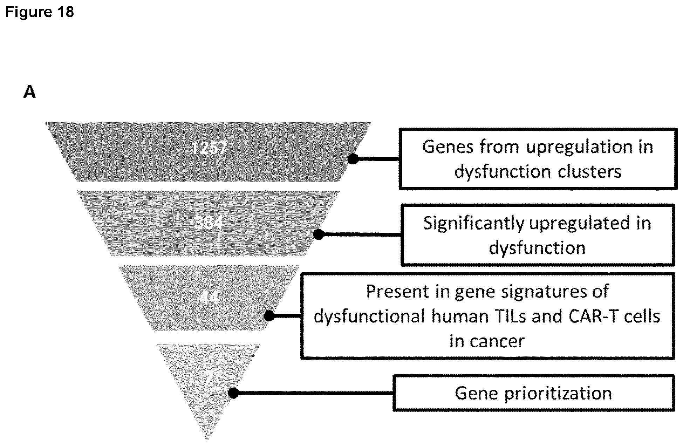

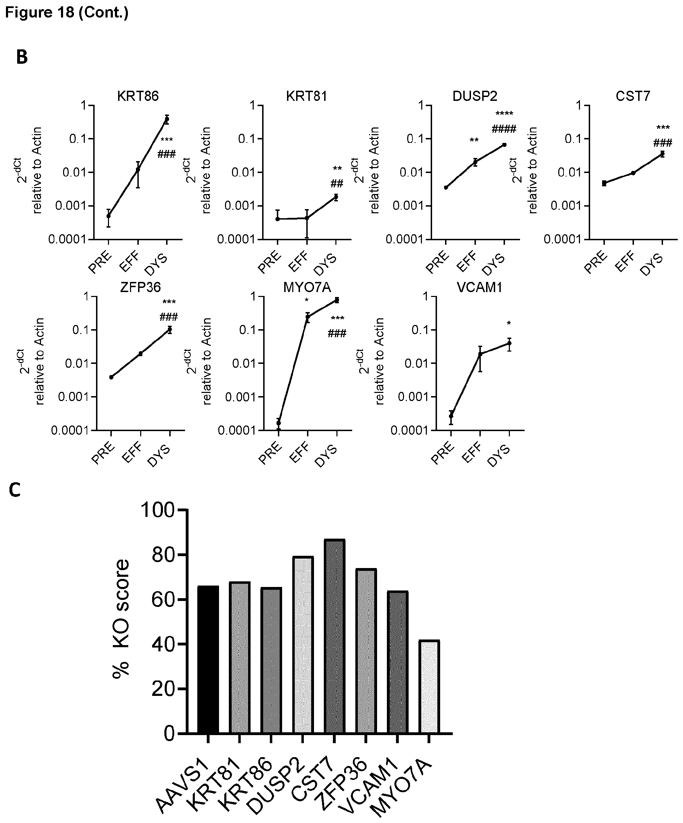

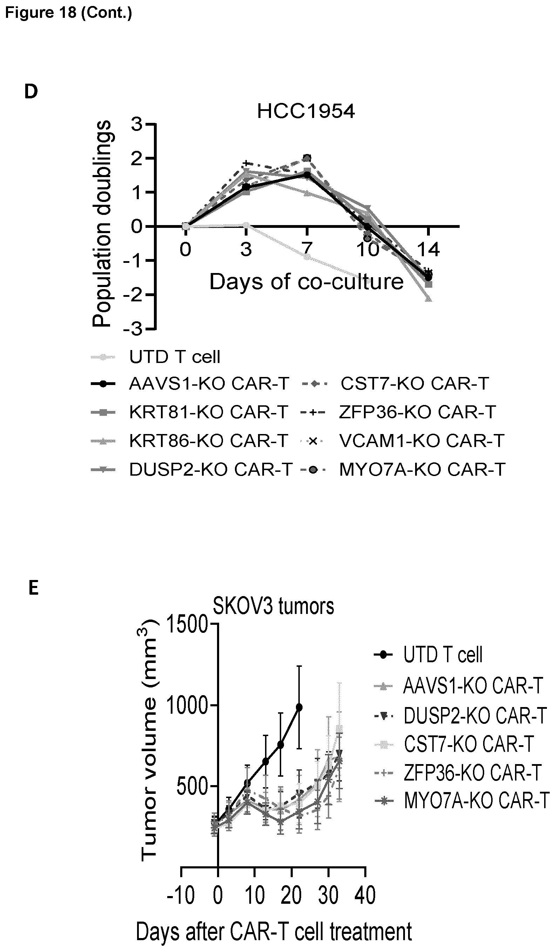

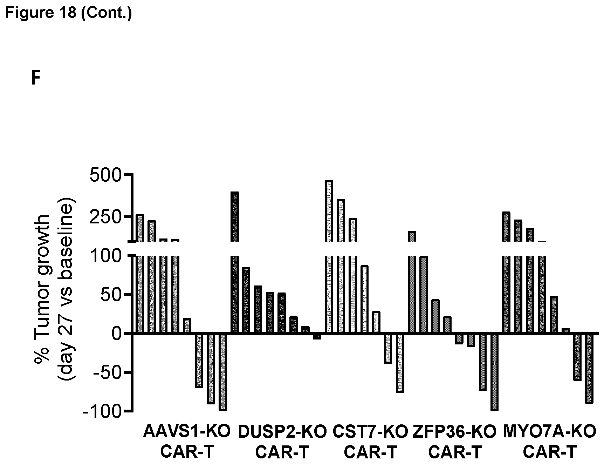

**Figure 18****. Knocking-out other candidate genes upregulated at the dysfunctional phase does not enhance CAR-T cell function. A)** Pipeline for the selection of candidate genes for individual validation. Briefly, genes were selected based on their significant upregulation in dysfunctional clusters and their presence in eight previously published gene signatures of dysfunctional human TILs and CAR-T cells in cancer. Gene prioritization included the highest log fold change of upregulation, a previously described role in T cell function, and highest level of expression in dysfunctional vs effective phase, according to logCPM values. **B)** Expression of gene candidates was assessed in CAR-TILs from preinfusion (PRE), effective (EFF) and dysfunctional (DYS) phase using RT-qPCR. Expression of selected candidate genes is shown as mean ±SD of 3 healthy donors. All genes were normalized using actin-b as housekeeping gene. Differences were calculated using a one-way ANOVA between effective or dysfunctional with preinfusion (indicated as *, *p<0.05, **p<0.01 and ***p<0.001) or between effective and dysfunctional (indicated as #, ^{##}p<0.01, ^{###}p<0.001 and ^{####}p<0.0001). C) Percentage of KO in CAR-T cells from one healthy donor is represented at day 10 after primary expansion. D) Control AAVS1-KO CAR-T cells and KO-CART cells for candidate genes were chronically stimulated in vitro with HCC1954 tumor cells every 3-4 days in absence of cytokines at a 3:1 effector to target cell ratio. Data shows population doublings throughout the restimulation assay. **E)** NSG mice bearing pre-established SKOV3 tumors were treated with a single dose of 2x10⁶ control AAVS1-KO or candidate gene-KO CAR-T cells. Tumors were measured at indicated timepoints using a digital calliper. Data is represented as mean ±SEM of tumor volume (N=8 tumors per group). F) Change in tumor volume on day 27 after CAR-T therapy versus baseline is plotted for individual tumors (N=10-12 tumors per group).

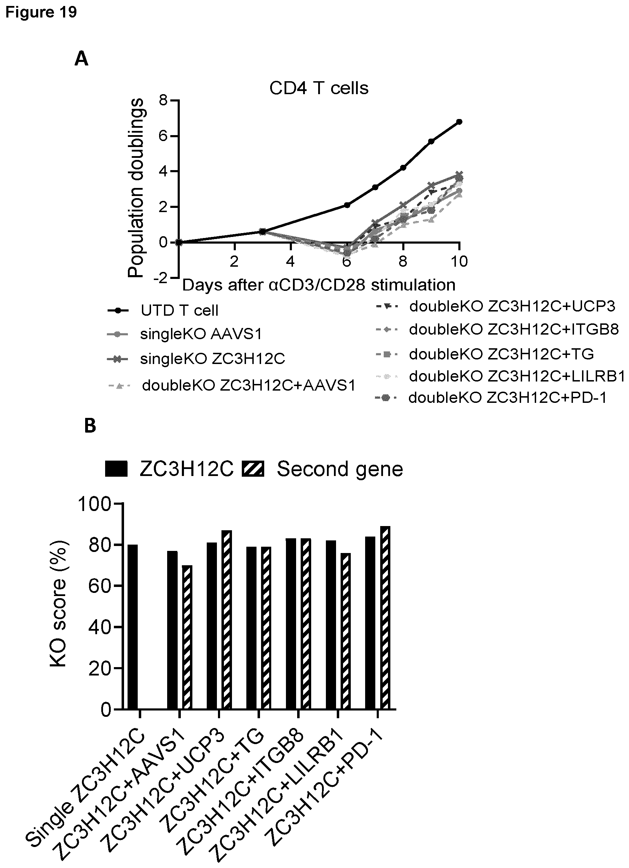

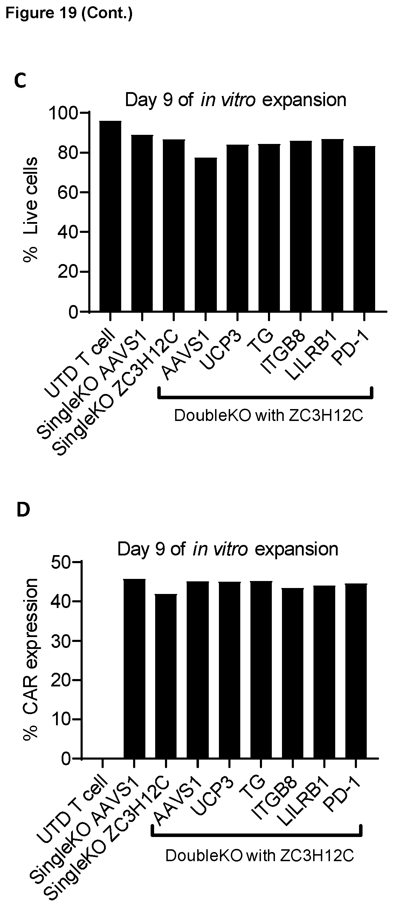

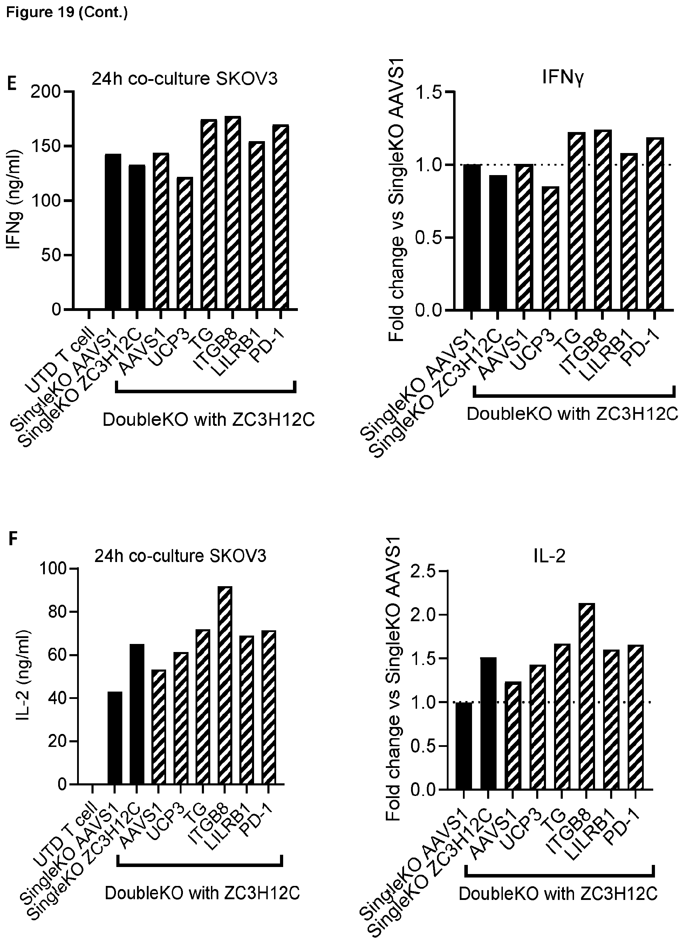

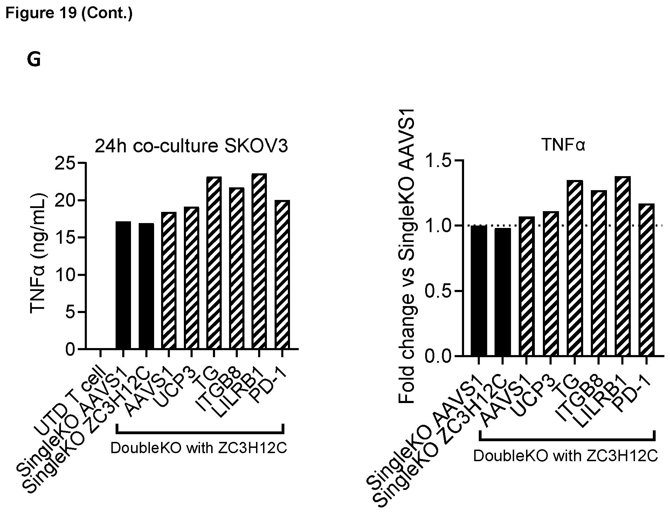

**Figure 19****. The double genetic ablation of ZC3H12C with other gene candidates results in enhanced cytokine production *in vitro.* A)** Single and double KO CAR-T cells were expanded and counted *in vitro* for ten days. Data for CD4+ T cells are plotted as population doublings overtime (N=1 healthy donor). Similar results were obtained for CD8+ T cells. **B)** Genome editing efficiency using the CRISPR/Cas9 system is represented as percentage of KO score at day 10 of primary T cell expansion. The second gene knocked-out in combination with ZC3H12C is indicated on the x-axis for each group. **C-D)** Percentage of **C)** live cells and **D)** CAR expression was analyzed in gene-edited CAR-T cells on day 9 after primary expansion on CD8+ T cells. **E-F)** Single and double KO CAR-T cells were *in vitro* co-cultured with SKOV3 tumor cells at an effector-to-target ratio of 3:1. **E)** IFN-y, **F)** IL-2 and **G)** TNF-α production was measured after 24 hours of coculture using ELISA (N=1 healthy donor). Left panels show concentration levels and right panels show fold change of each group versus AAVS1-KO CAR-T control.

### DETAILED DESCRIPTION OF THE INVENTION

### General definitions

Terms not specifically defined herein should be given the meanings that would be given to them by one of skill in the art in light of the disclosure and the context. As used in the specification, however, unless specified to the contrary, the following terms have the meaning indicated and the following conventions are adhered to.

The use of the word **"a"** or **"an"** may mean **"one,"** but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words **"comprising"** (and any form of comprising, such as "comprise" and "comprises"), **"having"** (and any form of having, such as "have" and "has"), **"including"** (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the term **"consists of".** As used herein, the phrase "consisting of" excludes any element, step, or ingredient not specified in the claim except for, e.g., impurities ordinarily associated with the element or limitation.

The expression **"or combinations thereof"** as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, **"about", "around", "approximately"** refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, 10, 11, 12, 13, 14 or 15%. Preferably the term "about" means exactly the indicated value (± 0%).

The term **"encoding"** refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

The term **"antigen"** or "Ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequence or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to elicit the desired immune response. Moreover, a skilled artisan will understand that an antigen need not be encoded by a "gene" at all. It is readily apparent that an antigen can be generated synthesized or can be derived from a biological sample. Such a biological sample can include, but is not limited to a tissue sample, a tumor sample, a cell or a biological fluid.

An **"antigen receptor"** is a molecule that binds antigens. It recognizes specific molecular structures and recruit adaptors through their effector domains, triggering trans-membrane transduction signaling pathway to exert immune response. Examples of antigen receptors can be the T cell receptor or TCR or a chimeric antigen receptor or CAR.

As used herein, the term **"T cell receptor"** or **"TCR"** refers to a complex of membrane proteins that participate in the activation of T cells in response to the presentation of antigen. The TCR comprises an antigen-binding domain that is responsible for recognizing antigens bound to major histocompatibility complex molecules. TCR is composed of a heterodimer of an alpha (α) and beta (β) chain, although in some cells the TCR consists of gamma and delta (γ/δ) chains. TCRs may exist in alpha/beta and gamma/delta forms, which are structurally similar but have distinct anatomical locations and functions.

A **"chimeric antigen receptor"** or **"CAR"** refers to an artificial immune cell receptor that is engineered to recognize and bind to an antigen, preferably a tumour associated antigen, expressed by undesired cells, for example, disease cells such as cancer cells. A T cell of the invention that expresses a CAR polypeptide is referred to as a CAR T cell

In the context of a protein sequence or polypeptide, a **"domain"** refers to a functional and/or structural unit or region in a protein or polypeptide. Domains are usually responsible for a particular function or interaction, contributing to the overall role of a protein. A domain may vary in length but is preferably between 25 amino acids to up to 500 amino acids long.

The term **"affibody"** refers to a protein that is derived from the Z domain of protein A and that been engineered to bind to a specific target.

The term **"anticalin"** refers to a protein that is derived from the lipocalin and that been engineered to bind to a specific target.

The term **"repebody"** refers to a protein that is derived from a leucine-rich repeat module and that been engineered to bind to a specific target.

The term **"designed ankyrin repeat proteins"** or "DARPin" refers to a protein that is derived from an ankyrin repeat that has been engineered to bind to a specific target.

The term **"nanobody"** refers to a protein comprising the soluble single antigen-binding V-domain of a heavy chain antibody, preferably a camelid heavy chain antibody.

The term **"peptide aptamer"** refers to a short, 5-20 amino acid residue sequence, that can bind to a specific target.

The term **"fynomer"** refers to a protein that is derived from the SH3 domain of human Fyn kinase that has been engineered to bind to a specific target. The term **"monobody"** refers to a protein that is derived from a fibronectin type II domain that has been engineered to bind to a specific target.

As used herein, the term **"antibody"** encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site.

The term **"antigen-binding fragment"** or **"Fab"** refers to an antibody fragment comprising one constant and one variable domain of each of the heavy and light chain. A Fab fragment may be obtained by digesting an intact monoclonal antibody with papain.

The term **"single-chain antigen-binding fragment"** or **"scFab"** refers to a fusion protein comprising one variable and one constant domain of the light chain of an antibody attached to one variable and one constant domain of the heavy chain of an antibody, wherein the heavy and light chains are linked together through a short peptide.

The term **"single-chain variable fragment"** or **"scFv"** refers to a fusion protein comprising the variable domains of the heavy chain and light chain of an antibody linked to one another with a peptide linker. The term also includes a disulfide stabilized Fv (dsFv). In some embodiments, the antigen-binding domain can be a single-chain variable fragment (scFv) that binds a tumor antigen as disclosed herein. The scFv may comprise an antibody heavy chain variable region (V_{H}) and an antibody light chain variable region (V_{L}), which optionally may be connected via a flexible peptide linker. In some embodiments, the scFv may have the V_{H} to V_{L} orientation (from N-terminus to C-terminus). Alternatively the scFv may have the V_{L} to V_{H} orientation (from N-terminus to C-terminus).

The term **"sequence identity"** refers to a percentage value obtained when two sequences are compared using a pairwise sequence alignment tool. In the present case, the sequence identity is preferably obtained using a local alignment tool, preferably a Basic Local Alignment Search Tool (BLAST) tool.

A **"lentivirus"** as used herein refers to a genus of the *Retroviridae* family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses. Vectors derived from lentiviruses offer the means to achieve significant levels of gene transfer *in vivo.*

The term **"anti-tumor effect"** or **"anti-tumor capacity"** as used herein, refers to a biological effect which can be manifested by a decrease in tumor volume, a decrease in the number of tumor cells, a decrease in the number of metastases, an increase in life expectancy, or amelioration of various physiological symptoms associated with the cancerous condition.

The term **"cancer"** as used herein is defined as disease characterized by the rapid and uncontrolled growth of aberrant cells. Cancer cells can spread locally or through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

As used herein **"endogenous"** refers to any material from or produced inside an organism, cell, tissue or system.

As used herein, the term **"exogenous"** refers to any material introduced from or produced outside an organism, cell, tissue or system.

The term **"expression"** as used herein is defined as the transcription and/or translation of a particular nucleotide sequence driven by its promoter.

The term **"treatment"** as used herein refers to the prophylactic and/or therapeutic treatment. The term **"therapeutic treatment"** as used herein refers to bringing a body from a pathological state or disease back to its normal, healthy state. Specifically, unless otherwise indicated, includes the amelioration, cure, and/or maintenance of a cure (i.e., the prevention or delay of relapse) of a disease or disorder. Treatment after a disorder has started aims to reduce, alleviate, ameliorate or altogether eliminate the disorder, and/or its associated symptoms, to prevent it from becoming worse, to slow the rate of progression, or to prevent the disorder from re-occurring once it has been initially eliminated (i.e., to prevent a relapse).

### Detailed description

The authors of the present invention hypothesized that some genes that are significantly enriched in dysfunctional T cells in comparison to pre-infusion T cells may have a role in orchestrating T cell dysfunction and thus can be the target for improved cells for use in immunotherapy. Particularly, the ablation of specific genes may be a tool to revert or delay CAR-T cell exhaustion and therefore enhance CAR-T antitumor efficacy *in vivo.* To test this, the authors of the present invention performed a series of analysis, explained in the Example section, that resulted in the selection of 14 genes, which were found to be enriched gene knockouts after *in vivo* screen compared to T cells before the knockout, see Fig. 6A and Table 3. These genes were further divided in three clusters based on their kinetic pattern of expression in CAR-TILs, see Fig. 7.

Next, the authors of the present invention selected 5 of them, ZC3H12C, UCP3, TG, ITGB8, and LILRB1 as a proof of concept to demonstrate that CAR-T cells KO for each gene would have improved anti-tumor properties. It is noted that the choice of the 5 genes was not based on the best-performing ones according to the ranking of Table 3, but the authors decided to choose 2 of the top list according to the score (ZC3H12C, UCP3), two in the middle (TG and LILRB1), and gene ITGB8 positioned with a score of 12. However, despite in their differences in the score, the five of them demonstrated being potential targets for generating improved CAR-T cells, as their silencing improved the anti-tumor efficacy, cell persistence, survival, and cytokine production of the CAR-T cells (see, e.g., Figs. 9 - 17). Importantly, it is noted that the genetic ablation of other genes differentially expressed in dysfunctional CAR-TILs but selected based on a different strategy, such as KRT86, KRT81, DUSP2, CST7, ZFP36, MYO7A and VCAM1 did not enhance or impair the antitumor effect of CART cells and the majority of tumors escaped CAR-T cell therapy (see Fig. 18).

Altogether, this indicates that not all of the genes with distinct kinetic patterns of gene expression during the preinfusion, effector, and dysfunctional phases are potential target for improved anti-tumor T cells. The 14 candidates described herein share a single general inventive concept due to their involvement in a common pathway or disease mechanism which is their role in T cell dysfunction. Hence, their unifying technical feature is their involvement in T cell dysfunction so that, when they are silenced or inhibited, the cell acquires improved anti-tumor properties.

Additionally, it was found that genes ZC3H12C, UCP3, TG and ITGB8 co-express with genes from the TNF-α signaling pathway, see Fig. 8. This indicates that these four genes also share a single inventive concept due to their involvement in common mechanisms of action of TNF-α signaling via NF-kB pathway as a driver of CAR-T cell dysfunction. This functional relationship establishes a technical link among the 4 genes, rather than an arbitrary grouping.

In view of the above results, the present invention refers to immune cells having reduced or eliminated the function and/or the expression of one or more of the 14 candidate genes, preferably ZC3H12C, UCP3, TG, ITGB8, and LILRB1.

### The immune cell of the invention

The **first aspect** of the invention relates to an immune cell, also called herein **"the immune cell of the invention",** or a population thereof, expressing an antigen receptor, wherein the antigen receptor comprises an antigen-binding domain, and wherein the immune cell has been modified to have reduced or eliminated the function and/or the expression of one or more target protein(s) encoded by one or more target gene(s).

The terms **"a population of an immune cell"** and **"immune cells"** are to be understood as synonyms throughout the present specification. Said terms refer to a plurality of immune cells.

An **"immune cell"** or a **"cell of the immune system"** as used herein can refer cells of the immune system or cells derived thereof, such as lymphoid stem cells that originate lymphocytes (B cell progenitor, T cell progenitor, natural killer cells) and myeloid progenitors which encompass granulocytes (neutrophils, eosinophils, basophils, mast cells, monocytes (e.g. dendritic cells and macrophages). Preferably, the immune cell is a mammal immune cell, more preferably a human immune cell. The immune cell of the invention encompasses any precursor or progenitor cells of the immune system.

In preferred embodiments, the immune cell is an effector immune cell, also called herein the **"immune effector cell of the invention". "Immune effector cell"** as used herein, refers to a cell that is involved in an immune response, e.g., in the promotion of an immune effector response. Examples of immune effector cell of the invention include T cells, e.g., alpha/beta T cells and gamma/delta T cells, B cells, natural killer (NK) cells, natural killer T (NKT) cells, mast cells, and myeloid-derived phagocytes. **"Immune effector function or immune effector response"** as used herein, refers to function or response, e.g., of an immune effector cell, that enhances or promotes an immune attack of a target cell (such as a tumour cell). For example, an immune effector function or response refers a property of a T or NK effector cell that promotes killing or the inhibition of growth or proliferation, of a target cell. Preferably, the effector immune cell is a mammal effector immune cell, more preferably a human effector immune cell. The terms **"a population of an immune effector cell"** and **"immune effector cells"** are to be understood as synonyms throughout the present specification. Said terms refer to a plurality of immune effector cells.

In some embodiments, the immune cell of the invention or a population thereof can be a T cell, a B cell, a natural killer (NK) cell, a natural killer T (NKT) cell, a mast cell and/or a myeloid-derived phagocyte. Preferably, the immune effector cell of the invention is a T cell, preferably human T cell, also called herein the **"T cell of the invention".**

In some embodiments, the immune cell of the invention or population thereof include one or more subsets of T cells or other cell types, such as whole T cell populations, CD4+ cells, CD8+ cells, and subpopulations thereof, such as those defined by function, activation state, maturity, potential for differentiation, expansion, recirculation, localization, and/or persistence capacities, antigen specificity, type of antigen receptor, presence in a particular organ or compartment, marker or cytokine secretion profile, and/or degree of differentiation.

In some embodiments, T cells of the invention can be derived from one or more suitable mammals, for example, one or more human donors. T cells of the invention can be obtained from a number of sources, including, but not limited to, peripheral blood mononuclear cells, bone marrow, lymph nodes tissue, cord blood, thymus issue, tissue from a site of infection, ascites, pleural effusion, spleen tissue, and tumors. In certain embodiments, T cells of the invention can be obtained from a unit of blood collected from a subject using any number of techniques known to the skilled person, such as sedimentation, e.g., FICOLL^{™} separation.

In some embodiments, T cells of the invention can be isolated from a mixture of immune cells (e.g., those described herein) to produce an isolated T cell population. For example, after isolation of peripheral blood mononuclear cells (PBMC), both cytotoxic and helper T lymphocytes can be sorted into naive, memory, and effector T cell subpopulations either before or after activation, expansion, and/or genetic modification.

A specific subpopulation of T cells of the invention may express one or more of the following cell surface markers: TCRab, CD3, CD4, CD8, CD27 CD28, CD38, CD45RA, CD45RO, CD62L, CD127, CD122, CD95, CD197, CCR7, KLRG1, MCH-I proteins and/or MCH-II proteins. In some embodiments, the specific subpopulation of T cells of the invention expresses one or more of the markers selected from the group consisting of TCRab, CD4 and CD8. They can be isolated by positive or negative selection techniques. In some embodiments, subpopulations of T cells may be isolated by positive or negative selection prior to genetic engineering and/or post genetic engineering. The T cells of the invention may be derived from stem cells (e.g., HSCs or iPSCs).

An isolated population of T cells of the invention may express one or more of the T cell markers, including, but not limited to a CD3+, CD4+, CD8+, or a combination thereof. In some embodiments, the T cells of the invention are isolated from a donor, or subject, and first activated and stimulated to proliferate *in vitro* prior to undergoing gene editing.

In a most preferred embodiment, the immune cell of the invention, preferably the immune effector cell, or a population thereof, is a T cell, optionally wherein the T cell is a CD4⁺ T cell, a CD8⁺ T cell or a CD4⁺/CD8⁺ T cell.

Among the sub-types and subpopulations of T cells and/or of CD4+ and/or of CD8+ T cells of the invention are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta (αβ) T cells, and/or gamma/delta (γδ) T cells.

In a preferred embodiment, the immune cell of the invention, preferably the immune effector cell, or the population thereof is selected from the group consisting of cytotoxic T-cell, helper T-cell, memory T-cell, regulatory T-cell, tissue infiltrating lymphocyte, γδ T-cell and any combination thereof. In some embodiments, the T cell population of the invention comprises primary T cells isolated from one or more human donors.

T cells from a suitable source can be subjected to one or more rounds of stimulation, activation and/or expansion. T cells can be activated and expanded using methods known in the art, and this can be done before, after or simultaneously to gene editing.

In some embodiments, T cells are activated at the same time that genome editing compositions are introduced into the T cells. In some embodiments, the T cell population can be expanded and/or activated after the genetic editing. T cell populations or isolated T cells generated by any of the gene editing methods described herein are also within the scope of the present invention.

In an embodiment, the immune cell of the invention may be autologous ("self") to the subject, i.e., the cells are from the same subject. In a preferred embodiment, the immune cell of the invention or the population thereof is autologous. As used herein, the term **"autologous"** is meant to refer to any material derived from the same individual to which it is later to be reintroduced into the individual. Alternatively, the immune cell of the invention can be non-autologous or allogenic ("non-self," e.g., allogeneic, syngeneic or xenogeneic) to the subject. **"Allogeneic"** means that the immune cells are not derived from the subject who receives the treatment but from different individuals (donors) of the same species as the subject. "Allogeneic" thus refers to a graft derived from a different animal of the same species.

Since the immune cell of the invention has been modified to have reduced or eliminated the function and/or the expression of one or more of the target proteins, it is understood that the cell of the invention is a cell that has been altered from its original, natural state. Hence, the immune cell of the invention is a non-naturally occurring cell. In an embodiment, the immune cell of the invention or a population thereof is an engineered immune cell. As used herein, an **"engineered immune cell"** includes an immune cell that has been genetically or chemically modified as compared to a naturally occurring immune cell. For example, an engineered T cell has been modified to lack a nucleic acid, such as a target gene, wherein said nucleic acid is naturally found in a cell from which it was derived (a wild type cell). For example, an engineered T cell has been modified to comprise molecules that inhibit the function and/or expression of the target protein, such as blockers, inhibitors, as defined below. The engineered immune cell may be an engineered effector immune cell T cell, such as an engineered natural killer (NK) cell, an engineered T cell, an engineered monocyte, an engineered macrophage, or an engineered dendritic cell. An engineered immune cell is also considered a modified immune cell.

As explained above, the immune cell of the invention or the population thereof has been modified to have reduced or eliminated the function and/or the expression of a target gene or target protein. The function of a target gene refers to the specific biological role that the gene plays within a cell or organism. This function is typically determined by the gene's ability to produce a particular protein or RNA that contributes to various cellular processes. In essence, the function of a target gene describes how the gene contributes to the structure, function, or regulation of cells and biological systems. This can include producing proteins, regulating other genes, or maintaining cellular homeostasis. The function of a target protein refers to the specific biological role that the protein plays within a cell or organism. This function is typically linked to the protein's biochemical properties, structure, and interactions with other molecules.

The expression of a target gene or protein refers to the process by which a specific gene is transcribed into messenger RNA (mRNA) and then translated into a functional target protein. This expression determines how much of that gene's protein product is produced in a cell, tissue, or organism, which influences various biological functions. Gene expression refers to multi-step process where a target gene's DNA sequence is transcribed into RNA, and then the RNA is translated into a protein. Target protein expression refers to the amount of the target protein present in the cell.

A **"reduction of the function of a target gene or protein"** refers to a decrease of the gene or protein function or activity of at least 10%, 20%, 30%, 40%, 50%, 60%, 70%, 80%, 90%, 95% or 99% or more as compared to the function or activity of the same target protein or gene within a reference value. An **"elimination of the function of a gene or protein"** refers to a decrease of the target gene or protein function or activity of 100% as compared to a reference value. Preferably, the elimination of a target gene leads to the absence in the cell of substantial detectable level of a target protein, preferably as measured by Western Blot.

A **"reduction of the expression of a target gene or protein"** refers to a decrease of the target gene or protein expression or amount or level of at least 10%, 20%, 30%, 40%, 50%, 60%, 70%, 80%, 90%, 95% or 99% or more as compared to a reference value. An **"elimination of the expression of a target gene or protein"** refers to a decrease of the target gene or protein expression, amount or level of 100% as compared to a reference value. Preferably, the reduction or elimination of target gene or protein expression, amount or level, leads to the absence in the cell of substantial detectable expression, amount or level, of the target protein, as measured preferably by Western blot. Preferably, the reduction or elimination of target gene expression, amount or level is measured by PCR or RT-PCR.

The terms **"decrease," "reduced,"** and **"reduction"** are all used herein preferably to mean a decrease by a statistically significant amount. Thus, preferably, the reduction or elimination of the function and/or the expression of a target gene or protein is a statistically significant reduction or elimination as compared to a reference value. By **"statistically significant"** or **"significant"** is referred herein as the determination by an analyst that the results in the data are not explainable by chance alone. Statistical hypothesis testing is the method by which the skilled person makes this determination. This test provides a p-value, which is the probability of observing results as extreme as those in the data, assuming the results are truly due to chance alone. A p-value of 0.1 or lower (preferably 0.05, 0.01, 0.001 or lower) is considered herein to be statistically significant.

As explained above, the reduction or elimination of the function and/or expression of the target gene or protein is calculated with respect to a reference value. **"Reference value",** as used herein, refers to a predetermined quantitative or qualitative measurement, threshold, or parameter used as a standard for comparison in the evaluation of the function and/or expression of a target gene or protein. The reference value may be derived from empirical data, computational models, or established scientific literature and serves as a benchmark for determining the presence, absence, reduction, or activity of a target protein or gene in a test sample. Thus, the reference value or reference level can be an absolute value; a relative value; a value that has an upper and/or lower limit; a range of values; an average value; a median value, a mean value, or a value as compared to a particular control or baseline value. The reference value can be based on a large number of samples, or based on a pool of samples including or excluding the sample to be tested.

In an embodiment, the reference value is a reference cell or population thereof. A **"reference cell",** as used herein, refers to a cell or a population of cells that can be used as reference value for the analysis and comparison of the reduction or elimination of the function and/or expression of a target gene or protein. Preferably, the reference cell is the same cell type of the immune cell of the invention but does not have the modification that results in the reduced or eliminated the function and/or the expression of a target gene or protein. Preferably, the comparison between the reference cell and the immune cell of the invention is performed under identical culture conditions. Preferably, the reference cell is a wild-type cell of the same type as the immune cell of the invention.

In a preferred embodiment, the immune cell of the invention has been modified to have reduced or eliminated the function and/or expression of one or more target gene(s) or protein(s), and this reduction or elimination is assessed by comparing the modified immune cell of the invention to a reference cell of the same type that has not been modified. **"A cell of the same type that has not been modified"** is preferably an immune cell identical to the immune cell of the invention but without having the modification that caused the reduction or elimination of the function and/or the expression of the one or more target gene(s) or protein(s). A reference cell is a cell that has been treated with the same conditions and reagents used to modify the immune cell in the invention, but in a mock manner, and is then compared to the modified immune cell. A mock condition or mock reagent refers to a control setup in an experiment where cells or samples are treated with an inactive substance or vehicle to mimic experimental conditions without introducing the actual test treatment. An example of a reference cell is a cell that has been modified at a safe harbor site. A **"safe harbor site"** refers to a specific location in the genome that is known to be genetically stable and where the insertion or deletion of genetic material does not disrupt important genes or cause harmful effects. These sites are used as control locations for experiments that involve **genetic modification,** ensuring that the inserted or deleted genetic material does not interfere with the normal function of the cell.

The reference immune cell without having the modification that reduces or eliminates the function and/or expression a target gene or protein, such as the genes of Table 4, Table 6, Table 7 or Table 8, may also be referred to as a **"control immune cell", "control immune effector cell"** or simply **"control cell".**

Thus, to measure the reduction or elimination of a target protein's or gene's function or expression in the modified immune cell of the invention, it is compared to a reference cell that has not been modified. The function or activity of a target protein and/or the expression of a target gene, amount or level of a target protein can be determined by any method known in the art, such as those suitable for the determination and quantification of a protein, e. g. in a sample or in a cell. By way of a non-limiting illustration, the level of a target protein can be determined by means of a technique which comprises the use of antibodies with the capacity for binding specifically to the target protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. There is a wide variety of known tests that can be used according to the present invention to measure the reduction or elimination of the expression of a target protein, such as combined application of non-labeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for embodiment, affinity chromatography techniques or ligand-binding assays, sequence similarity, co-immunoprecipitation, fluorescence resonance energy transfer (FRET), two-hybrid system, phage display, surface plasmon resonance (SPR) or DNA footprinting (for proteins that bind to DNA). Most preferably, the expression levels of a target protein is assessed by Western Blot.

Alternatively or additionally, the level of expression of a target gene may be assessed by any method known to a person of skill in the art. For example, expression may be assessed by measuring the levels of gene expression in the transduced tissue on the level of the mRNA or the protein by standard assays known to a person of skill in the art, such as qPCR, RNA sequencing, Northern blot analysis, in situ hybridization, microarrays, reporter gene assays, single-cell RNA-Seq, ChIP-Seq, Western blot analysis, mass spectrometry analysis of protein-derived peptides or ELISA. Most preferably, the function or expression of a target gene or protein is assessed by Real time PCR (RT-PCR).

As explained above, the immune cell of the invention has been modified to have reduced or eliminated the function and/or the expression of one or more target gene(s) or protein(s). As used herein, a **"target gene"** or "candidate gene" is one or more of genes selected from the group consisting of ZC3H12C, UCP3, TG, ITGB8, LILRB1, AHNAK2, STON1, MYH6, PTK7, INSRR, ACTG2, ADAM12, FN1 and COL4A1 genes, or any combination thereof. Said target genes are listed in Table 4. As shown in the examples of the 300 initial candidate genes, the KO for 14 genes was significantly enriched **(****Figure 6A** and **Table 3).**

As used herein, a **"target protein"** or "candidate protein" is a protein encoded by a target gene. That is, a target protein is a protein selected from the group of ZC3H12C, UCP3, TG, ITGB8, LILRB1, AHNAK2, STON1, MYH6, PTK7, INSRR, ACTG2, ADAM12, FN1 or COL4A1 proteins.

**Table 4: List of target genes (abbreviatures and full gene name)**

| | |
|---|---|
| ZC3H12C | Zinc finger CCCH-type containing 12C |
| UCP3 | Uncoupling protein 3 |
| TG | Thyroglobulin |
| ITGB8 | Integrin subunit beta-8 |
| LILRB1 | Leukocyte immunoglobulin like receptor B1 |
| AHNAK2 | AHNAK nucleoprotein 2 |
| STON1 | Stonin 1 |
| MYH6 | myosin heavy chain 6 |
| PTK7 | protein tyrosine kinase 7 |
| INSRR | Insulin receptor related receptor |
| ACTG2 | Actin gamma 2, smooth muscle |
| ADAM12 | ADAM metallopeptidase domain 12 |
| FN1 | Fibronectin 1 |
| COL4A1 | Type IV collagen alpha 1 chain |

As depicted in Fig. 7, the target genes of Table 4 can be further categorized into three subgroups (Cluster 6, Cluster 1 and Cluster 7) based on their kinetic pattern of expression in CAR-TILs as shown in Table 6, 7 and 8, respectively:

**Table 6: List of progressive exhaustion target genes (cluster 6, genes steadily upregulated throughout the development of exhaustion) (abbreviatures and full gene name)**

| | |
|---|---|
| ZC3H12C | Zinc finger CCCH-type containing 12C |
| UCP3 | Uncoupling protein 3 |
| TG | Thyroglobulin |
| INSRR | Insulin receptor related receptor |
| STON1 | Stonin 1 |
| ACTG2 | Actin gamma 2, smooth muscle |
| FN1 | Fibronectin 1 |

**Table 7: List of early exhaustion target genes (cluster 1, genes upregulated early in the exhaustion trajectory) (abbreviatures and full gene name)**

| | |
|---|---|
| ITGB8 | Integrin subunit beta-8 |
| LILRB1 | Leukocyte immunoglobulin like receptor B1 |
| ADAM12 | ADAM metallopeptidase domain 12 |

**Table 8: List of late exhaustion target genes (cluster 7, genes upregulated at later stages of exhaustion) (abbreviatures and full gene name)**

| | |
|---|---|
| PTK7 | protein tyrosine kinase 7 |
| COL4A1 | Type IV collagen alpha 1 chain |
| MYH6 | myosin heavy chain 6 |
| AHNAK2 | AHNAK nucleoprotein 2 |

Based on this categorization, in an embodiment, the target gene or protein is selected from one or more of the target genes depicted in Table 6, 7, or 8.

In an embodiment, the target gene or protein is selected from the list consisting of ZC3H12C, UCP3, TG, ITGB8, and LILRB1. As shown in Fig. 8, four of the target genes (ZC3H12C, UCP3, TG and ITGB8) can be grouped based on their role in TNF-α signaling via NF-kB pathway. Hence, in the most preferably embodiment, the target gene or protein is selected from the list consisting of ZC3H12C, UCP3, TG and ITGB8.

It is noted that the same name or abbreviation can be used for both a gene and the protein it encodes, even though they are technically different entities (a gene is the DNA sequence, and the protein is the functional product of that gene). For example, "ZC3H12C" can be used to refer to the target gene and to the target protein encoded by said target gene. Hence, in many embodiments of the present invention, the terms "target gene" and "target protein" are used interchangeably. Similarly, the expressions "the immune cell has been modified to have reduced or eliminated the function and/or the expression of the one or more of the target genes or proteins" and "the immune cell has been modified to have reduced or eliminated the function and/or the expression of the one or more of the target proteins encoded by one or more target genes" are considered equivalent in the context of the present invention.

Further, in the context of the present invention, a target gene is to be understood to encompass all variants, transcripts, homologues and functional equivalents. In the context of the present invention, the target protein is to be understood to encompass all post-translational modifications, variants, isoforms, homologues and functional equivalents. "Functional equivalents" refer to target genes or proteins with different sequences but having the same function, utility, or technical effect as a given target gene.

Hence, the immune cell of the invention is modified to have reduced or eliminated the function and/or the expression of one or more target proteins encoded by one or more target genes of Table 4, Table 6, Table 7 or Table 8. This reduction or elimination is assessed by comparing the function or expression of these target gene or protein in the immune cell of the invention to those in a reference cell. Preferably, the immune cell of the invention is modified to have reduced or eliminated the function and/or the expression of at least 1, 2, 3, 4, 5, 6, 7, 8, 9, 10, 11, 12, 13 or 14 of the target gene(s) listed in Table 4, or at least 1, 2, 3, 4, 5, 6, 7, 8, 9, 10, 11, 12, 13 or 14 of the target protein(s) they encode.

Preferably, the target gene or target protein is selected from the group consisting of ZC3H12C, UCP3, TG, ITGB8 and LILRB1, more preferably ZC3H12C. Thus, in a preferred embodiment, the immune cell of the invention has been modified to have reduced or eliminated the function and/or the expression of ZC3H12C, UCP3, TG, ITGB8 or LILRB1, more preferably ZC3H12C, target genes or target proteins. Most preferably, the immune T cell of the invention is modified to have eliminated the expression of one or more target proteins encoded by at least the target genes ZC3H12C, UCP3, TG, ITGB8 or LILRB1, more preferably ZC3H12C, or any combination thereof.

**"ZC3H12C", "zinc finger CCCH-type containing 12C"** or **"MCPIP3"** target gene refers to the gene that encodes "probable ribonuclease ZC3H12C" or "regnase-3" target protein, which belongs to the same subfamily of zinc finger proteins (ZFPs) as ZC3H12A (regnase-1), which is a master RNA regulator in macrophages and T cells. ZC3H12C has been identified as a regulator of macrophage activation. Preferably, the ZC3H12C target gene comprises or consists of SEQ ID NO: 1, or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 1. Preferably, the ZC3H12C target protein comprises or consists of SEQ ID NO: 2, or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 2.

Therefore, in a preferred embodiment of the first aspect, the immune cell, preferably the immune effector cell, of the invention or a population thereof expresses an antigen receptor, wherein the antigen receptor comprises an antigen-binding domain, and wherein the immune cell of the invention is or has been modified to have reduced or eliminated the function and/or the expression of ZC3H12C target gene or ZC3H12C target protein, wherein the reduction or elimination is preferably calculated with respect to the same immune cell without having said modification.

The **"UCP3"** or **"uncoupling protein 3"** target gene is a member of the uncoupling protein (UCP) subfamily, which codes for **"putative mitochondrial transporter UCP3"** target protein or **"uncoupling protein 3".** The physiological function of UCP is to catalyze electrophoretic efflux of fatty acid (FA) anions from the matrix, leading to futile cycling of FA, which also applies to UCP3. In humans, UCP3 mRNA was reported in skeletal muscle and in trace quantities in the heart. In rodents, it has been associated to the brown adipose tissue. Mice deficient in UCP3 exhibited higher ROS levels in skeletal muscle. UCP3 antioxidant activity has been demonstrated in isolated skeletal muscle mitochondria. Preferably, the UCP3 target gene comprises or consists of SEQ ID NO: 3, or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 3. Preferably, the UCP3 target protein comprises or consists of SEQ ID NO: 4, or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 4.

Therefore, in a preferred embodiment of the first aspect, the immune cell, preferably the immune effector cell, of the invention or a population thereof expresses an antigen receptor, wherein the antigen receptor comprises an antigen-binding domain, and wherein the immune cell of the invention is or has been modified to have reduced or eliminated the function and/or the expression of UCP3 target gene or UCP3 target protein, wherein the reduction or elimination is preferably calculated with respect to the same immune cell without having said modification.

The **"TG"** target gene is the gene that encodes **"thyroglobulin"** or **"Tg"** target protein, a vertebrate secretory protein synthesized in the thyrocyte endoplasmic reticulum (ER), where it acquires N-linked glycosylation and conformational maturation (including formation of many disulfide bonds), leading to homodimerization. Its primary functions include iodide storage and thyroid hormonogenesis. Preferably, the TG target gene comprises or consists of SEQ ID NO: 5, or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 5. Preferably, the TG target protein comprises or consists of SEQ ID NO: 6 or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 6.

Therefore, in a preferred embodiment of the first aspect, the immune cell, preferably the immune effector cell, of the invention or a population thereof expresses an antigen receptor, wherein the antigen receptor comprises an antigen-binding domain, and wherein the immune cell of the invention is or has been modified to have reduced or eliminated the function and/or the expression of TG target gene or TG target protein, wherein the reduction or elimination is preferably calculated with respect to the same immune cell without having said modification.

**"ITBG8"** target gene refers to the gene that encodes **"Integrin subunit beta-8"** or **"integrin β₈"** target protein. It forms an heterodimer with subunit α_{V}, α_{V}β₈, which is an atypical integrin as it does not mediate adhesion through its ligands. Integrin α_{V}β₈ recognizes (Arg-Gly-Asp)RGD-containing ligands, such as transforming growth factor (TGF)-β. Studies have shown that integrin αᵥβ₈ is specialized for binding to latent TGF-β, and its binding to cell- or matrix-attached latent TGF-β results in the release of active TGF-β, which is essential for TGF-β functions. αᵥβ₈ in this process does not involve cell rolling or adhesion. In addition, integrin αᵥβ₈ is an important regulator in the immune system that is involved in monocyte inflammatory responses and intestinal macrophage homeostasis through interaction with its ligand TGF-β. It is expressed in normal epithelial cells, neuronal and vascular cells of the brain, and mature tissue macrophages. Integrin β₈ plays essential roles in regulating neuroepithelium organization, endothelial cell proliferation, vasculogenesis, and capillary vessel growth in the early development of embryonic brain, and is required for vascular morphogenesis in the placenta, yolk sac, and embryonic brain. Preferably, the ITGB8 target gene comprises or consists of SEQ ID NO: 7, or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 7. Preferably, the ITGB8 target protein comprises or consists of SEQ ID NO: 8 or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 8.

Therefore, in a preferred embodiment of the first aspect, the immune cell, preferably the immune effector cell, of the invention or a population thereof expresses an antigen receptor, wherein the antigen receptor comprises an antigen-binding domain, and wherein the immune cell of the invention is or has been modified to have reduced or eliminated the function and/or the expression of ITGB8 target gene or ITGB8 target protein, wherein the reduction or elimination is preferably calculated with respect to the same immune cell without having said modification.

**"LILRB1"** target gene refers to the gene that encodes **"Leukocyte immunoglobulin like receptor B1"** target protein. LILRB1 gene is also known as CD85J, ILT2, LIR1, and MIR7. It contains four extracellular immunoglobulin domains and four intracellular ITIMs. LILRB1 is expressed on monocytes, macrophages, DCs, eosinophils and basophils, B cells, T cells, and natural killer (NK) cells, as well as on in vitro cultured cord blood-derived progenitor mast cells and osteoclasts. Preferably, the LILRB1 target gene comprises or consists of SEQ ID NO: 9, or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 9. Preferably, the LILRB1 target protein comprises or consists of SEQ ID NO: 10 or a sequence with at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% identity to SEQ ID NO: 10.

Therefore, in a preferred embodiment of the first aspect, the immune cell, preferably the immune effector cell, of the invention or a population thereof expresses an antigen receptor, wherein the antigen receptor comprises an antigen-binding domain, and wherein the immune cell of the invention is or has been modified to have reduced or eliminated the function and/or the expression of LILRB1 target gene or LILRB1 target protein, wherein the reduction or elimination is preferably calculated with respect to the same immune cell without having said modification.

In an embodiment, the immune cell of the invention expresses an antigen receptor comprising an antigen-binding domain, and it has been modified to have reduced or eliminated the function and/or the expression of two or more of the target genes depicted in Table 4. Preferably, the immune cell of the invention expresses an antigen receptor comprising an antigen-binding domain, and it has been modified to have reduced or eliminated the function and/or the expression of two or more of the target genes or proteins selected from the group consisting of ZC3H12C, UCP3, TG, ITGB8 and LILRB1. In an embodiment the immune cell of the invention expresses an antigen receptor comprising an antigen-binding domain, and it has been modified to have reduced or eliminated the function and/or the expression of three or more of the target genes or proteins selected from the group consisting of ZC3H12C, UCP3, TG, ITGB8 or LILRB1. In an embodiment, the immune cell of the invention expresses an antigen receptor comprising an antigen-binding domain, and it has been modified to have reduced or eliminated the function and/or the expression of four or five of the target genes or proteins selected from the group consisting of ZC3H12C, UCP3, TG, ITGB8 and LILRB1.

In an embodiment, the immune cell of the invention or the population thereof expresses an antigen receptor comprising an antigen-binding domain, and it has been modified to have reduced or eliminated the function and/or the expression of:
- one or more target genes or proteins selected from the group consisting of ZC3H12C UCP3, TG, ITGB8, LILRB1, and
- one or more target gene(s) or protein(s) selected from the group consisting of AHNAK2, STON1, MYH6, PTK7, INSRR, ACTG2, ADAM12, FN1, and COL4A1,
wherein the reduction or elimination of the function and/or the expression of said target gene(s) or protein(s) is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- one or more target genes or proteins selected from the group consisting of ZC3H12C, UCP3, TG, and ITGB8, and
- one or more target gene(s) or protein(s) selected from the group consisting of LILRB1, AHNAK2, STON1, MYH6, PTK7, INSRR, ACTG2, ADAM12, FN1, and COL4A1,
wherein the reduction or elimination of the function and/or the expression of said target gene(s) or protein(s) is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- ZC3H12C target gene or protein, and
- one or more target gene(s) or protein(s) selected from the group consisting of UCP3, TG, ITGB8, LILRB1, AHNAK2, STON1, MYH6, PTK7, INSRR, ACTG2, ADAM12, FN1, and COL4A1,
wherein the reduction or elimination of the function and/or the expression of said target gene(s) or protein(s) is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- ZC3H12C target gene or protein, and
- one or more target gene(s) or protein(s) selected from the group consisting of UCP3, TG, ITGB8, and LILRB1,
wherein the reduction or elimination of the function and/or the expression of said target gene(s) or protein(s) is preferably calculated with respect to the same immune cell without having said modification.

In some embodiments of the first aspect, the immune cell, preferably the immune effector cell, of the invention or the population thereof, expresses an antigen receptor comprising an antigen-binding domain, wherein the immune cell of the invention is or has been modified to have a reduction or elimination, preferably a statistically significant reduction or elimination, of the proteins encoded by ZC3H12C and UCP3 target genes, wherein said reduction or elimination is preferably calculated with respect to the same immune cell without having said modification.

In some embodiments of the first aspect, the immune cell, preferably the immune effector cell, of the invention or the population thereof expresses an antigen receptor comprising an antigen-binding domain, wherein the immune cell of the invention is or has been modified to have a reduction or elimination, preferably a statistically significant reduction or elimination, of the proteins encoded by ZC3H12C and ITGB8 target genes, wherein said reduction or elimination is preferably calculated with respect to the same immune cell without having said modification.

In some embodiments of the first aspect, the immune cell, preferably the immune effector cell, of the invention or the population thereof expresses an antigen receptor comprising an antigen-binding domain, wherein the immune cell of the invention is or has been modified to have a reduction or elimination, preferably a statistically significant reduction or elimination, of the proteins encoded by ZC3H12C and TG target genes, wherein said reduction or elimination is preferably calculated with respect to the same immune cell without having said modification.

In some embodiments of the first aspect, the immune cell, preferably the immune effector cell, of the invention or the population thereof expresses an antigen receptor comprising an antigen-binding domain, wherein the immune cell of the invention is or has been modified to have a reduction or elimination, preferably a statistically significant reduction or elimination, of the proteins encoded by ZC3H12C and LILRB1 target genes, wherein said reduction or elimination is preferably calculated with respect to the same immune cell without having said modification.

In some embodiments of the first aspect, the immune cell, preferably the immune effector cell, of the invention or the population thereof expresses an antigen receptor comprising an antigen-binding domain, wherein the immune cell of the invention is or has been modified to have a reduction or elimination, preferably a statistically significant reduction or elimination, of the proteins encoded by UCP3 and TG target genes, wherein said reduction or elimination is preferably calculated with respect to the same immune cell without having said modification.

In some embodiments of the first aspect, the immune cell, preferably the immune effector cell, of the invention or the population thereof expresses an antigen receptor comprising an antigen-binding domain, wherein the immune cell of the invention is or has been modified to have a reduction or elimination, preferably a statistically significant reduction or elimination, of the proteins encoded by UCP3 and ITGB8 target genes, wherein said reduction or elimination is preferably calculated with respect to the same immune cell without having said modification.

In some embodiments of the first aspect, the immune cell, preferably the immune effector cell, of the invention or the population thereof expresses an antigen receptor comprising an antigen-binding domain, wherein the immune cell of the invention is or has been modified to have a reduction or elimination, preferably a statistically significant reduction or elimination, of the proteins encoded by UCP3 and LILRB1 target genes, wherein said reduction or elimination is preferably calculated with respect to the same immune cell without having said modification.

In some embodiments of the first aspect, the immune cell, preferably the immune effector cell, of the invention or the population thereof expresses an antigen receptor comprising an antigen-binding domain, wherein the immune cell of the invention is or has been modified to have a reduction or elimination, preferably a statistically significant reduction or elimination, of the proteins encoded by TG and ITGB8 target genes, wherein said reduction or elimination is preferably calculated with respect to the same immune cell without having said modification.

In some embodiments of the first aspect, the immune cell, preferably the immune effector cell, of the invention or the population thereof expresses an antigen receptor comprising an antigen-binding domain, wherein the immune cell of the invention is or has been modified to have a reduction or elimination, preferably a statistically significant reduction or elimination, of the proteins encoded by TG and LILRB1 target genes, wherein said reduction or elimination is preferably calculated with respect to the same immune cell without having said modification.

In some embodiments of the first aspect, the immune cell, preferably the immune effector cell, of the invention or the population thereof expresses an antigen receptor comprising an antigen-binding domain, wherein the immune cell of the invention is or has been modified to have a reduction or elimination, preferably a statistically significant reduction or elimination, of the proteins encoded by ITGB8 and LILRB1 target genes, wherein said reduction or elimination is preferably calculated with respect to the same immune cell without having said modification.

Other modifications can be performed in the immune cell of the invention to improve its functionality. For instance, the immune cell of the invention of population thereof can be further modified to have reduced or eliminated the function and/or the expression of PD-1 or LAG-3 proteins, wherein the reduction or elimination of the function and/or the expression of PD-1 or LAG-3 is preferably calculated with respect to the same immune cell without having said modification.

**"LAG-3",** also named FDC, gene refers to the gene that codes for **"lymphocyte activation gene 3 protein",** "LAG-3" or "CD223" protein. LAG-3 is express on immune cells such as activated T cells, T regulatory cells (Tregs) and natural killer cells (NK cells). It is an inhibitory immune checkpoint that suppresses s excessive immune responses in the immune system by inhibiting the activation and proliferation of T cells, thereby reducing the risk of immune system attack on autologous tissue. This function depends on the interaction of LAG-3 expressed on Tregs and ligands on antigen-presenting cells (APCs). However, in the tumor microenvironment (TME), tumor cells can escape immune surveillance by hijacking this mechanism.

**"PD-1"** gene is the **"programmed Cell Death 1 gene"** that encodes the PD-1 protein (Programmed Cell Death Protein 1 protein receptor). It is crucial immune checkpoint protein expressed on T cells. PD-1 plays a significant role in regulating immune responses by preventing excessive immune activation and maintaining self-tolerance, primarily through interactions with its ligands PD-L1 and PD-L2. This pathway is exploited by tumors to evade immune detection, making PD-1 a key target in cancer immunotherapy.

In an embodiment, the immune cell of the invention or the population thereof expresses an antigen receptor comprising an antigen-binding domain, and it has been modified to have reduced or eliminated the function and/or the expression of:
- one or more target gene(s) selected from the group of Table 4, and
- PD-1 gene,
wherein the reduction or elimination of the function and/or the expression of said genes is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- one or more target gene(s) selected Table 6, 7, or 8, and
- PD-1 gene,
wherein the reduction or elimination of the function and/or the expression of said genes is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- one or more target gene(s) selected from the group of Table 4, and
- LAG-3 gene,
wherein the reduction or elimination of the function and/or the expression of said genes is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- one or more target gene(s) selected from Table 6, 7, or 8, and
- LAG-3 gene,
wherein the reduction or elimination of the function and/or the expression of said genes is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- one or more target gene(s) or protein(s) selected from the group consisting of ZC3H12C UCP3, TG, ITGB8, and LILRB1, and
- PD-1 gene,
wherein the reduction or elimination of the function and/or the expression of said genes is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- one or more target gene(s) or protein(s) selected from the group consisting of ZC3H12C, UCP3, TG, and ITGB8, and
- PD-1 gene,
wherein the reduction or elimination of the function and/or the expression of said genes or protein(s) is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- one or more target gene(s) or protein(s) selected from the group consisting of ZC3H12C UCP3, TG, ITGB8, and LILRB1, and
- LAG-3 gene,
wherein the reduction or elimination of the function and/or the expression of said genes or protein(s) is preferably calculated with respect to the same immune cell without having said modification.

In an embodiment, the immune cell of the invention or the population thereof expresses an antigen receptor comprising an antigen-binding domain, and it has been modified to have reduced or eliminated the function and/or the expression of:
- one or more target gene(s) or protein(s) selected from the group consisting of ZC3H12C, UCP3, TG, and ITGB8, and
- LAG-3 gene,
wherein the reduction or elimination of the function and/or the expression of said genes or protein(s) is preferably calculated with respect to the same immune cell without having said modification.

In an embodiment, the immune cell of the invention expresses an antigen receptor comprising an antigen-binding domain, and it has been modified to have reduced or eliminated the function and/or the expression of:
- ZC3H12C target gene or protein, and
- PD-1 and/or LAG-3 gene,
wherein the reduction or elimination of the function and/or the expression of ZC3H12C, PD-1 and/or LAG-3 genes is preferably calculated with respect to the same immune cell without having said modification.

In an embodiment, the immune cell, preferably the immune effector cell of the invention, or a population thereof, expressing an antigen receptor comprising an antigen-binding domain has been modified to have a reduction or elimination, preferably a statistically significant reduction or elimination, of the function and/or expression of one or more of the target proteins encoded by the target genes of Table 4. In an embodiment, said reduction or elimination of the function and/or expression is achieved by one or more of:
1) reducing or eliminating of the activity and/or function of the target protein (target protein inhibition),
2) reducing or eliminating the translation of the target protein by interfering with the target gene transcript (target gene transcript inhibition), and/or
3) reducing or eliminating the transcription of the target gene by interfering with the target gene (target gene disruption).

Each of 1), 2), and 3) are explained below:

### 1) Target protein inhibition

Target protein inhibition refers to the process of reducing or blocking the activity or function of a specific target protein, resulting in a non-functional target protein. By **"non-functional"** target protein it is herein intended a protein with a reduced activity or a lack of detectable activity as described above.

In an embodiment, the immune cell, preferably the immune effector cell, of the invention or a population thereof expressing an antigen receptor comprising an antigen-binding domain has been modified to have reduced or eliminated the activity and/or function of a target protein. Inhibition of the target protein in the immune cell of the invention can be permanent and irreversible or transient or reversible. Preferably however, target protein inhibition is permanent and irreversible. Inhibition of target protein in the immune cell of the invention may be achieved prior or after injection of the cell in the targeted patient as described below. Inhibitors of target protein activity in a cell according to the invention can be selected among any compound or agent natural or not having the ability of inhibiting the function or activity of said protein or inhibiting the protein gene expression.

In some embodiments, the immune cell of the invention or a population thereof has a reduction or elimination, preferably a statistically significant reduction or elimination, of the function and/or the expression of the target proteins because the immune cell has been modified to comprise an inhibitor, antagonist or blocker that binds the target protein, thereby inhibiting its function.

The inhibition, blockade or antagonism of a target protein by an agent or compound can be determined by any method known in the art suitable for the determination and measurement of the inhibitory capacity of a compound or agent of said target protein. By way of a non-limiting illustration, the inhibition of a target protein can be determined by radiometric methods, colorimetric methods (e.g., ELISA), luminescence (e.g., UV-Vis and fluorescence, including quantitative polymerase chain reaction), microscale thermophoresis, NMR, separation techniques - mass spectrometry (MS) (e.g., capillary electrophoresis, liquid chromatography - MS, capillary electrophoresis - MS, KiNativ), sensors, including gravimetric/electrochemical (surface acoustic waves, quartz crystal microbalance, electrochemical impedance spectroscopy, chrono-coulometry, voltammetry) and optical (reflectometric interference spectroscopy, surface plasmon resonance, biolayer interferometry and photonic crystals), and others (isothermal titration calorimetry and thermal stability shift assay), or any combination thereof.

Said agent can be selected from small molecule inhibitors; antibodies, antibodies derivatives such as intrabodies, nanobodies, aptamers or affibodies that can block or inhibit target protein activity.

In an embodiment, the agent is an inhibitor of UCP3, preferably MitoPBN.

In an embodiment, the agent is an inhibitor of ITGB8 target proteins, preferably selected from the list consisting of CRB-601 and PLN-101095.

In an embodiment, the agent is an inhibitor of LILRB1 target protein, preferably OR641.

In an embodiment, the agent is an inhibitor of TG target protein, preferably Carbimazole.

These agents are also represented in the following Table 5:

| **Gene** | **Name of the inhibitor** | **Brief description** | **Company** |
|---|---|---|---|
| ITGB8 | CRB-601 | Monoclonal antibody targeting αvβ8 to inhibit the production of TGFβ | Columbus Pharmaceutical |
| | PLN-101095 | Small molecule dual inhibitor of the integrins alpha V beta 1 (aVb1) and 8 (aVb8) | Pliant Therapeutics |
| LILRB1 | OR641 | Dual antagonist antibody targeting LILRB1 and LILRB2 | OncoResponse |
| UCP3 | MitoPBN | Inhibits superoxide activation of mitochondrial uncoupling protein 1 (UCP1), UCP2, and UCP3 | N/A |
| TG | Carbimazole | Inhibits the thyroid peroxidase enzyme, which acts on thyroglobulin (TG) to produce thyroid hormones | Generic drug |

### 2) Target gene transcript inhibition

Target gene transcript inhibition refers to the process of reducing or blocking the function of messenger RNA (mRNA) from a specific target gene, thereby decreasing or preventing the expression of the corresponding target protein.

In an embodiment, the immune cell, preferably the immune effector cell, of the invention or a population thereof expressing an antigen receptor comprising an antigen-binding domain has been modified to have reduced or eliminated the transcription or translation of the messenger RNA (mRNA) of a target gene. Nucleic acid molecules that block transcription or translation, include antisense molecules complementary to mRNA; RNA interfering agents such as a small interfering RNA (siRNA), small hairpin RNA (shRNA), microRNA (miRNA), or a piwiRNA (piRNA); antisense oligonucleotides, ribozymes and/or any combination thereof.

siRNA technology includes that based on RNAi utilizing a double- stranded RNA molecule having a sequence homologous with the nucleotide sequence of mRNA which is transcribed from the gene, and a sequence complementary with the nucleotide sequence. siRNA generally is homologous/complementary with one region of mRNA which is transcribed from the target gene, or may be siRNA including a plurality of RNA molecules which are homologous/complementary with different regions.

Anti-sense oligonucleotides include anti-sense RNA molecules and anti-sense DNA molecules that act to directly block the translation of a target gene and thus preventing target protein translation or increasing mRNA degradation, thus decreasing the level of the target protein encoded by said target gene and thus its activity in a cell.

An RNA interfering agent is an agent that interferes with or inhibits expression of a target biomarker gene by RNA interference (RNAi). Such RNA interfering agents include, but are not limited to, nucleic acid molecules including RNA molecules, which are homologous to a target gene of the invention (e.g., ZC3H12C, UCP3, TG, ITGB8 and LILRB1, more preferably ZC3H12C, UCP3, TG and ITGB8), or a fragment thereof, short interfering RNA (siRNA), and small molecules which interfere with or inhibit expression of the target nucleic acid by RNA interference (RNAi).

"Small inhibitory RNAs", "short interfering RNAs", small interfering RNA" or "small silencing RNA" (siRNAs) can also function as inhibitors of expression for use in the present invention. Target gene expression can be reduced by contacting a subject or cell with a small double stranded RNA (dsRNA), or a vector or construct causing the production of a small double stranded RNA, such that the gene expression is specifically inhibited (i.e. RNA interference or RNAi).

shRNAs (short hairpin RNA) can also function as inhibitors of expression for use in the present invention. shRNAs are typically composed of a short (e.g., 19-25 nucleotide) antisense strand, followed by a 5-9 nucleotide loop, and the analogous sense strand. Alternatively, the sense strand may precede the nucleotide loop structure and the antisense strand may follow.

Ribozymes can also function as inhibitors of target gene expression for use in the present invention. Ribozymes are enzymatic RNA molecules capable of catalyzing the specific cleavage of RNA.

Both antisense oligonucleotides and ribozymes useful as inhibitors of transcription and expression of a target gene can be prepared and designed by known methods.

In an embodiment, the immune cell of the invention is modified to comprise an agent that binds a nucleotide region in the transcriptome of the immune cell that is a regulator of the target gene(s), so that the binding results in the reduction or elimination of the function and/or expression of the target protein encoded by at least one of said target genes.

### 3) Target gene disruption

In other embodiments of the invention, the reduction or elimination of the function or expression of the target protein can be achieved by target gene repression/suppression via target gene disruption. In this case, the immune cell of the invention or a population thereof is a modified cell or population thereof that has been genetically modified to have one or more of the target genes disrupted.

Gene disruption comprises, without being limited to, knock-out, exon, or region, and/or replacement with an exogenous sequence, and/or by mutation, e.g., frameshift or missense mutation, within the target gene, typically within an exon of the target gene. In some embodiments, the disruption results in a premature stop codon being incorporated into the gene, such that the protein is not expressed or is non-functional. The disruption is preferably carried out at the DNA level. The disruption preferably is permanent, irreversible, or not transient. Thus, preferably, the immune cell of the invention or population thereof comprises a disruption in one or more of the target genes.

In a preferred embodiment, the immune cell of the invention, preferably the immune effector cell of the invention, or a population thereof, expressing an antigen receptor comprising an antigen-binding domain has been modified to have a reduction or elimination, preferably a statistically significant reduction or elimination, of the expression and/or function of at least 1, 2, 3, 4, 5, 6, 7, 8, 9, 10, 11, 12, 13 or 14 of the target genes of Table 4, wherein the reduction or elimination is preferably measured with respect to the same immune effector cell without having said modification, and wherein the modification is selected from an insertion, a frameshift mutation, a deletion, a knock-in, a knock-down, a knock-out of the target gene, or part of the target gene, or the deletion of the entire target gene. The term "knock-out", "knockout" or "knock out" as used herein refers to the ablation of gene expression of one or more genes.

A disrupted target gene may contain one or more mutations *(e.g.,* insertion, deletion, or nucleotide substitution, *etc.)* relative to the wild-type counterpart to substantially reduce or completely eliminate the activity of the target protein. The one or more mutations may be located in a non-coding region, for example, a promoter region, a regulatory region that regulates transcription or translation; or an intron region. Alternatively, the one or more mutations may be located in a coding region *(e.g.,* in an exon). In some embodiments, the disrupted gene does not express or expresses a substantially reduced level of the encoded target protein. In other embodiments, the disrupted target gene expresses the encoded target protein in a mutated form, which is either not functional or has substantially reduced activity. In some embodiments, a disrupted target gene is a gene that does not encode a functional target protein or encodes a non-functional protein. In some embodiments, an immune cell of the invention that comprises a disrupted target gene does not express a detectable level (measured by *e.g.* by antibody, *e.g.,* by flow cytometry) of the target protein encoded by the gene. An immune cell of the invention that does not express a detectable level of the target protein may be referred to as a knockout cell of the invention. For example, a cell having a ZC3H12C gene edit may be considered a ZC3H12C knockout cell if ZC3H12C protein cannot be detected in the cell using an antibody that specifically binds ZC3H12C protein, or the mRNA level of the ZC3H12C gene cannot be detected or is detected in a very low level with specific primers with respect to a control gene. Preferably, the reduction or elimination of the expression of a target protein is achieved through repression of the corresponding target gene expression or through target gene disruption. Preferably, the immune cell of the invention has disrupted one or more of the genes of Table 4, preferably ZC3H12C UCP3, TG, ITGB8 or LILRB1 genes. Preferably, the immune cell of the invention has disrupted one or more of the target genes selected from the group consisting of ZC3H12C, UCP3, TG, and ITGB8.

In some embodiments, target gene disruption is achieved using gene editing agents such as a DNA-targeting molecule, such as a DNA-binding protein or DNA-binding nucleic acid, or complex, compound, or composition, containing the same, which specifically binds to or hybridizes to the gene. In some embodiments, the DNA-targeting molecule comprises a DNA-binding domain, *e.g.,* a zinc finger protein (ZFP) DNA-binding domain, a transcription activator-like protein (TAL) or TAL effector (TALE) DNA-binding domain, a clustered regularly interspaced short palindromic repeats (CRISPR) DNA-binding domain, or a DNA-binding domain from a meganuclease. Zinc finger, TALE, and CRISPR system binding domains can be engineered to bind to a predetermined nucleotide sequence. Preferably, the target gene disruption is achieved using CRISPR/Cas-mediated gene editing system.

In some embodiments, the DNA-targeting molecule, complex, or combination contains a DNA-binding molecule and one or more additional domain, such as an effector domain to facilitate the repression or disruption of the gene. For example, in some embodiments, the gene disruption is carried out by fusion proteins that comprise DNA-binding proteins and a heterologous regulatory domain or functional fragment thereof.

Typically, the additional domain is a nuclease domain. Thus, in some embodiments, gene disruption is facilitated by gene or genome editing, using engineered proteins, such as nucleases and nuclease-containing complexes or fusion proteins, composed of sequence-specific DNA-binding domains fused to, or complexed with, non-specific DNA-cleavage molecules such as nucleases.

These targeted chimeric nucleases or nuclease-containing complexes carry out precise genetic modifications by inducing targeted double- stranded breaks or single-stranded breaks, stimulating the cellular DNA -repair mechanisms, including error-prone nonhomologous end joining (NHEJ) and homology-directed repair (HDR). In some embodiments the nuclease is an endonuclease, such as a zinc finger nuclease (ZFN), TALE nuclease (TALEN), an RNA-guided endonuclease (RGEN), such as a CRISPR-associated (Cas) protein, or a meganuclease. Such genetic strategies can use constitutive expression systems or inducible expression systems according to well-known methods in the art.

The modification performed in the immune effector cell of the invention is preferably a gene disruption, wherein the gene disrupted is selected from Table 4, preferably at least one of the target genes ZC3H12C, UCP3, TG, ITGB8 or LILRB1, most preferably at least one of ZC3H12C, UCP3, TG, and ITGB8. Gene disruptions can be carried out using one or more DNA -binding nucleic acids, such as disruption via an RNA-guided endonuclease (RGEN), or other form of repression by another RNA-guided effector molecule. For example, in a preferred embodiment, the gene disruption is carried out using clustered regularly interspaced short palindromic repeats (CRISPR) system and CRISPR-associated proteins. In an embodiment, the immune cell of the invention or a population thereof has been modified, namely genetically modified, to have a reduction or elimination of one or more target gene expression. Preferably, this is achieved via RGEN methods, such as CRISPR methods.

In general, **"CRISPR system"** or **"CRISPR methods"** refers collectively to transcripts and other elements involved in the expression of, or directing the activity of, CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), and/or other sequences and transcripts from a CRISPR locus.

The CRISPR-Cas system relies on the DNA nuclease, such as Cas9, and two noncoding RNAs, crisprRNA (crRNA) and transactivating RNA (tracrRNA), to target the cleavage of DNA. Numerous types and classes of CRISPR/Cas systems have been described.

In some embodiments, the Cas9 (CRISPR associated protein 9) endonuclease is used in a CRISPR method for making the immune cells of the invention, i.e., for disruption one or more of the target genes. In some embodiments, the CRISPR/Cas system comprises components derived from a Type-I, Type-II, or Type-III system.

In some embodiments, the Cas nuclease is from a Type-II CRISPR/Cas system (e.g., a Cas9 protein from a CRISPR/Cas9 system). In some embodiments, the Cas nuclease is from a Type-I CRISPR/Cas system. In some embodiments, the Cas nuclease is a component of the Cascade complex of a Type-I CRISPR/Cas system. For example, the Cas nuclease is a Cas3 nuclease.

The CRISPR system involves the use of a genome-targeting nucleic acid that can direct the endonuclease to a specific target sequence within a target gene for gene editing at the specific target sequence. The genome-targeting nucleic acid can be a RNA. A genome-targeting RNA is referred to as a "guide RNA" or "gRNA" herein. A guide RNA comprises at least a spacer sequence that hybridizes to a target nucleic acid sequence within a target gene for editing, and a CRISPR repeat sequence. As is understood by the person of ordinary skill in the art, each guide RNA is designed to include a spacer sequence complementary to its genomic target sequence.

In some embodiments, the genome-targeting nucleic acid is a double-molecule guide RNA. In some embodiments, the genome-targeting nucleic acid is a single-molecule guide RNA or sgRNA. **"sgRNA"** or **"single guide RNA"** refers to the gRNA used in the CRISPR system. Both "sgRNA" and "gRNA" are interchangeable terms.

The **"target sequence"** is a sequence located in a target gene that is adjacent to a PAM sequence and is the sequence to be modified by an RNA-guided nuclease (e.g., Cas9). The "target sequence" is on the so-called PAM-strand in a "target nucleic acid," which is a doublestranded molecule containing the PAM-strand and a complementary non-PAM strand. One of skill in the art recognizes that the gRNA spacer sequence hybridizes to the complementary sequence located in the non-PAM strand of the target nucleic acid of interest. Thus, the gRNA spacer sequence is the RNA equivalent of the target sequence. The spacer of a gRNA interacts with a target nucleic acid of interest in a sequence-specific manner via hybridization (i.e., base pairing). The nucleotide sequence of the spacer thus varies depending on the target sequence of the target nucleic acid of interest.

Preferably, the immune cell of the invention expressing an antigen receptor comprising an antigen-binding domain is knock-out for one or more of the target genes of Table 4. Preferably, the immune T cell of the invention expressing an antigen receptor comprising an antigen-binding domain is knock-out for one or more of the target genes or proteins selected from the group consisting of ZC3H12C, UCP3, TG, ITGB8 and LILRB1, most preferably ZC3H12C, UCP3, TG and ITGB8. Preferably, the immune T cell of the invention expressing an antigen receptor comprising an antigen-binding domain is knock-out for ZC3H12C target gene. Preferably, the immune T cell of the invention expressing an antigen receptor comprising an antigen-binding domain is a double knock-out for ZC3H12C target gene and one of UCP3, TG, ITGB8, PD-1 and LILRB1 target genes.

In some embodiments, the sgRNA targets a gene of Table 4. Preferably, the sgRNA targets the ZC3H12C gene, for example, targets a site within exon 1, exon 2, exon 3, exon 4, exon 5, exon 6 or exon 7 of the ZC3H12C gene. An exemplary sgRNA sequence specific to the ZC3H12C gene is SEQ ID NO: 12.

In some embodiments, the sgRNA targets a UCP3 gene, for example, targets a site within exon 1, exon 2, exon 3, exon 4, exon 5, exon 6 or exon 7 of the UCP3 gene. An exemplary sgRNA sequence specific to the UCP3 gene is SEQ ID NO: 13.

In some embodiments, the sgRNA targets a TG gene, for example, targets a site within exon 1, exon 2, exon 3, exon 4, exon 5, exon 6, exon 7, exon 8, exon 9, exon 10, exon 11, exon 12, exon 13, exon 14, exon 15, exon 16, exon 17, exon 18, exon 19, exon 20, exon 20, exon 21, exon 22, exon 23, exon 24, exon 25, exon 26, exon 27, exon 28, exon 29, exon 30, exon 31, exon 32, exon 33, exon 34, exon 35, exon 36, exon 37, exon 38, exon 39, exon 40, exon 41, exon 42, exon 43, exon 44, exon 45, exon 46, exon 47, exon 48, exon 49, exon 50, exon 51 or exon 52 of a TG gene. An exemplary sgRNA sequence specific to the TG gene is SEQ ID NO: 14.

In some embodiments, the sgRNA targets a ITGB8 gene, for example, targets a site within exon 1, exon 2, exon 3, exon 4, exon 5, exon 6, exon 7, exon 8, exon 9, exon 10, exon 11, exon 12, exon 13, exon 14, exon 15, exon 16, exon 17, exon 18, or exon 19 of a ITGB8 gene. An exemplary sgRNA specific to the ITGB8 gene is SEQ ID NO: 15.

In some embodiments, the sgRNA targets a LILRB1 gene, for example, targets a site within exon 1, exon 2, exon 3, exon 4, exon 5, exon 6, exon 7, exon 8, exon 9, exon 10, exon 11, exon 12, exon 13, exon 14, exon 15, exon 16 or exon 17 of a LILRB1 gene. An exemplary sgRNA specific to the LlLRB1 gene is SEQ ID NO: 16.

In an embodiment, the immune cell of the invention or a population thereof has been modified to have reduced or eliminated the function and/or expression of a target gene using CRISPR/Cas nuclease system, as exemplified below. The CRISPR/Cas nuclease system disclosed herein, comprising one or more sgRNAs and at least one RNA-guided nuclease, optionally a donor template as disclosed below, can be delivered to a target cell (e.g., the immune cell of the invention) for genetic editing of a target gene, via a conventional method. In some embodiments, components of a CRISPR/Cas nuclease system as disclosed herein may be delivered to a target cell separately, either simultaneously or sequentially. In other embodiments, the components of the CRISPR/Cas nuclease system may be delivered into a target together, for example, as a complex. In some embodiments, sgRNA and a RNA-guided nuclease can be pre-complexed together to form a ribonucleoprotein (RNP), which can be delivered into a target cell.

RNPs are useful for target gene editing, at least because they minimize the risk of promiscuous interactions in a nucleic acid-rich cellular environment and protect the RNA from degradation. Methods for forming RNPs are known in the art. In some embodiments, an RNP containing an RNA-guided nuclease (e.g., a Cas nuclease, such as a Cas9 nuclease) and one or more gRNAs targeting one or more genes of interest can be delivered into a cell (e.g., the immune cell of the invention). In some embodiments, an RNP can be delivered to the immune cell of the invention by electroporation.

In a most preferred embodiment, the immune cell of the invention is an immune T cell or a population thereof, expressing an antigen receptor, wherein the antigen receptor comprises an antigen-binding domain, and wherein the immune T cell has been modified to have reduced or eliminated the function and/or the expression of the one or more of the target genes of Table 4, wherein the reduction or elimination of the function and/or the expression of the one or more of the target genes of Table 4 is calculated with respect to the same immune T cell without having said modification, and wherein the modification comprises disrupting, preferably performing a knock-out of the one or more target genes of Table 4, preferably by CRISPR/Cas nuclease system. The knock-out efficiency of a target gene may be assessed by any method known to a person of skill in the art. Examples of methods to assess knock-out efficiency may be, without being limited to, genomic PCR, Western Blotting, RT-qPCR, Sanger sequencing, Next-Generation Sequencing (NGS), flow cytometry, immunohistochemistry, T7 endonuclease I (T7E1) assay and functional assays (phenotypic analysis).

### Antigen receptors comprising an antigen-binding domain

As explained above, the immune cell of the invention or the population thereof expresses an antigen receptor comprising an antigen-binding domain. In some embodiments, the antigen receptor can be a T cell receptor or a chimeric antigen receptor or CAR, each of them are further defined below. Preferably, the antigen receptor comprises an antigen binding domain that is capable of binding to an antigen.

By **"capable of binding to an antigen"** is meant that the antigen-binding domain has binding affinity for an antigen. Preferably, the binding affinity is specific, i.e., the antigen-binding domain is capable of specifically binding to said antigen (which is its target molecule) in a manner which distinguishes from the binding to non-target molecules, i.e., from other molecules that are not said antigen. Thus, the antigen-binding domain either does not bind to non-target molecules or exhibits negligible or substantially-reduced (as compared to the target) e. g. background, binding to nontarget molecules. The antigen-binding domain specifically recognizes the antigen. Specific binding between the targeting moiety and the antigen can be assessed by numerous techniques, such as surface plasmon resonance, flow cytometry, or ELISA assays.

Preferably, the antigen is HER2, CD19 or mesothelin.

In an embodiment, the antigen-binding domain is capable of binding to HER2 antigen and thus is a HER2-binding domain. The term **"HER2"** or "human epidermal growth factor receptor 2" (also known as "receptor tyrosine-protein kinase erbB2") refers to the protein encoded by ERBB2 gene (also named as MLN19, NEU, NGL or HER2 gene). It is a transmembrane glycoprotein receptor of the epidermal growth factor receptor (EGFR) family. HER2 plays a crucial role in cell proliferation, survival, and differentiation. Its overexpression has been observed in cancers like breast, ovary, endometrium, bladder, lung, colon, and head and neck.

In an embodiment, antigen-binding domain is capable of binding to CD19 antigen and thus is a CD19-binding domain. The term **"CD19"** refers to the CD19 antigen, which is a transmembrane glycoprotein belonging to the immunoglobulin superfamily. It is encoded by the CD9 gene. CD19 is classified as a type I transmembrane protein, with a single transmembrane domain, a cytoplasmic C-terminus, and extracellular N-terminus. CD19 is a biomarker for normal and neoplastic B cells, as well as follicular dendritic cells. CD19 is critically involved in establishing intrinsic B cell signaling thresholds through modulating both B cell receptor-dependent and independent signaling. CD19 functions as the dominant signaling component of a multimolecular complex on the surface of mature B cells, alongside complement receptor CD21, and the tetraspanin membrane protein CD81 (TAPA-1), as well as CD225. CD19 plays a critical role in maintaining the balance between humoral, antigen-induced response and tolerance induction.

In an embodiment, the antigen-binding domain is capable of binding to mesothelin antigen and thus is a mesothelin-binding domain. The term **"mesothelin"** or "MSLN" (also named CAK1 antigen or pre-pro-megakaryocyte-potentiating factor) refers to the cell surface-bound, glycosylphosphatidylinositol (GPI) anchored protein encoded by the MSLN gene. Its normal expression has been found in the mesothelial cells of the pleura, pericardium, peritoneum, and tunica vaginalis in men, whereas very low MSLN expression (trace amounts) has also been observed in the epithelial lining of the ovaries, fallopian tubes, and rete testis. Overexpression of MSLN has been found in several cancers including malignant mesothelioma, ovarian cancer, breast cancer, pancreatic cancer, lung cancer, gastric cancer, endometrial cancer, cervical cancer, biliary cancer, uterine serous carcinoma, cholangiocarcinoma and pediatric acute myeloid leukemia.

In an embodiment, the immune cell of the invention comprises or expresses an antigen receptor that is a T cell receptor. Preferably, the immune cell is an immune T cell and it expresses or comprises a T cell receptor (TCR).

The TCR can be a wild-type TCR, a high affinity TCR, and/or a chimeric TCR. A high affinity TCR may be the result of modifications to a wild-type TCR that confers a higher affinity for a target antigen compared to the wild-type TCR. A high affinity TCR may be an affinity-matured TCR. Methods for modifying TCRs and/or the affinity-maturation of TCRs are known to those of skill in the art.

The TCR comprises an antigen-binding domain with affinity to a target antigen, wherein preferably the target antigen is on a target cell. The target antigen may include any type of protein, or epitope thereof, associated with the target cell. For example, the TCR may comprise an antigen-binding domain with affinity or capable of binding to a target antigen on a target cell that indicates a particular disease state of the target cell. In some embodiments, the target antigen is processed and presented by MHCs. In some embodiments, the target cell can be, without being limited to, a tumor cell. In some embodiments, the target antigen expressed by the target cell may be a tumor associated antigen. In some embodiments, the antigen may be HER2, CD19 or mesothelin.

In an embodiment, the immune cell of the invention comprises or expresses a Chimeric Antigen Receptor (CAR). Preferably, the immune cell is an immune T cell and it expresses or comprises a CAR, and this cell is also called herein, the **"CAR-T cell of the invention".**

Preferably, the CAR comprises:
a) an antigen-binding domain
b) a transmembrane domain,
c) hinge domain,
d) an intracellular signaling domain, and
e) optionally, a costimulatory domain.

There are various generations of CARs, each of which contains different components, which of them are further explained below:

### (a) Antigen Binding Domain

The **"antigen-binding domain"** or **"antigen-binding extracellular domain"** is the region of a CAR polypeptide that is exposed to the extracellular fluid when the CAR is expressed on cell surface. In an embodiment, the antigen binding domain comprises a targeting moiety. The term **"targeting moiety"** refers to a part of the antigen binding domain that is capable of binding, preferably specifically, to a specific antigen.

Preferably, the targeting moiety comprised in the antigen binding domain is capable of binding to HER2, CD19 or mesothelin antigens, and thus it is a HER2, CD19 or mesothelin targeting moiety. Preferably, the targeting moiety is an antibody, anticalin, repebody, monobody, scFv, Fab, F(ab')2, scFab, affibody, fynomer, DARPin, nanobody, or peptide aptamer. Preferably, the targeting moiety is a scFv.

The antigen-binding extracellular domain may be specific to a target antigen of interest, for example, a pathologic antigen such as a tumor associated antigen. In some embodiments, a **"tumor associated antigen"** refers to an immunogenic molecule, such as a protein, that is generally expressed at a higher level in tumor cells than in non-tumor cells, in which it may not be expressed at all, or only at low levels. In some embodiments, tumor associated structures, which are recognized by the immune system of the tumor-harboring host, are referred to as tumor-associated antigens. In some embodiments, a tumor-associated antigen is a universal tumor antigen, if it is broadly expressed by most types of tumors. In some embodiments, tumor-associated antigens are differentiation antigens, mutational antigens, overexpressed cellular antigens or viral antigens. In some embodiments, a tumor associated antigen is a tumor specific antigen or TSA, referring to an immunogenic molecule, such as a protein, that is unique to a tumor cell. Tumor associated antigens are exclusively expressed in tumor cells, for example, in a specific type of tumor cells. Preferred tumor associated antigens include, but are not limited to, HER2, CD19 and mesothelin.

In a particular embodiment, the targeting moiety can be a single-chain variable fragment (scFv) that binds human HER2. In a preferred embodiment, the antigen-binding domain is a mutated version of the trastuzumab-based 4D5 scFv, as exemplified below. In another particular embodiment, the targeting moiety can be a single-chain variable fragment (scFv) that binds human CD19. In another particular embodiment, the targeting moiety can be a single-chain variable fragment (scFv) that binds human mesothelin.

In some embodiments, a signal peptide may be located at the N-terminus of the antigen-binding domain to facilitate cell surface expression.

### (b) Transmembrane Domain

The CAR polypeptide disclosed herein may contain a transmembrane domain, which can be a hydrophobic alpha helix that spans the membrane. As used herein, a **"transmembrane domain"** refers to any protein structure that is thermodynamically stable in a cell membrane, preferably a eukaryotic cell membrane. The transmembrane domain can provide stability of the CAR containing such. In an embodiment, the transmembrane domain is linked to domain a) by a spacer or linker, wherein the spacer or linker is placed at the N-terminal of the transmembrane domain.

In some embodiments, the transmembrane domain of a CAR as provided herein can be a CD8 transmembrane domain. In other embodiments, the transmembrane domain can be a CD28 transmembrane domain. In yet other embodiments, the transmembrane domain is a chimera of a CD8 and CD28 transmembrane domain. Other transmembrane domains may be used as provided herein.

### (c) Hinge Domain

In some embodiments, a hinge domain may be located between an extracellular domain (comprising the antigen binding domain) and a transmembrane domain of a CAR. A **"hinge domain"** can be any oligopeptide or polypeptide that functions to link the transmembrane domain to the extracellular domain and/or the intracellular domain in the polypeptide chain. A hinge domain may function to provide flexibility to the CAR, or domains thereof, or to prevent steric hindrance of the CAR, or domains thereof.

In some embodiments, a hinge domain may comprise up to 300 amino acids (e.g., 10 to 100 amino acids, or 5 to 20 amino acids). In some embodiments, one or more hinge domain(s) may be included in other regions of a CAR. In some embodiments, the hinge domain may be a CD8α hinge domain or CD28 hinge domain. Other hinge domains may be used.

### (d) Intracellular Signaling Domain

Any of the CAR constructs contain one or more intracellular signaling domains (e.g., CD3ζ, and optionally one or more co-stimulatory domains), which are the functional end of the receptor. Following antigen recognition, receptors cluster and a signal is transmitted to the cell.

**"Intracellular signaling domain"** as used herein refers to all or a portion of one or more domains of a molecule (here the chimeric receptor molecule) that provides for activation of a lymphocyte. Intracellular domains of such molecules mediate a signal by interacting with cellular mediators to result in proliferation, differentiation, activation and other effector functions. Examples of intracellular signaling domains for use in a CAR of the invention include the intracellular sequences of the CD3ζ chain, and/or co-receptors that act in concert to initiate signal transduction following CAR engagement, as well as any derivative or variant of these sequences and any synthetic sequence that has the same functional capability. T cell activation can be said to be mediated by two distinct classes of cytoplasmic signaling sequence: those that initiate antigen-dependent primary activation and provide a T cell receptor like signal (primary cytoplasmic signaling sequences) and those that act in an antigen-independent manner to provide a secondary or co-stimulatory signal (secondary cytoplasmic signaling sequences). Primary cytoplasmic signaling sequences that act in a stimulatory manner may contain signaling motifs which are known as receptor tyrosine-based activation motifs or ITAMs. Examples of ITAM containing primary cytoplasmic signaling sequences include those derived from CD3ζ, FcRγ, CD3γ, CD3δ, CD3ε, CD5, CD22, CD79a, CD79b, and CD66d.

CD3ζ is the cytoplasmic signaling domain of the T cell receptor complex. CD3ζ contains three (3) immunoreceptor tyrosine-based activation motif (ITAM)s, which transmit an activation signal to the T cell after the T cell is engaged with a cognate antigen. In many cases, CD3ζ provides a primary T cell activation signal but not a fully competent activation signal, which requires a co-stimulatory signaling.

### (e) Co-stimulatory domain

Preferably, the CAR comprises a co-stimulatory domain. The term **"costimulatory domain"** or "costimulatory signaling domain" refers to a signaling moiety that provides to T cells a signal which, in addition to the primary signal provided by, for embodiment, the CD3ζ chain of the TCR/CD3 complex, mediates a T cell response, including, but not limited to, activation, proliferation, differentiation, cytokine secretion, and the like. A co-stimulatory domain can include all or a portion of, but is not limited to, CD27, CD28, 4-1BB (CD137), OX40 (CD134), CD30, CD40, ICOS, lymphocyte function-associated antigen-1 (LFA-1), CD2, CD7, LIGHT, NKG2C, B7-H3, and a ligand that specifically binds with CD83. In some embodiments, the co-stimulatory signaling domain is an intracellular signaling domain that interacts with other intracellular mediators to mediate a cell response including activation, proliferation, differentiation and cytokine secretion, and the like.

In some embodiments, the CAR polypeptides disclosed herein may further comprise one or more co-stimulatory signaling domains. The terms "co-stimulatory domain" and "costimulatory domain" are considered synonyms throughout this specification. For example, the co-stimulatory domains of CD28 and/or 4-1BB may be used to transmit a full proliferative/survival signal, together with the primary signaling mediated by CD3ζ. In some examples, the CAR disclosed herein comprises a CD28 co-stimulatory molecule. In other examples, the CAR disclosed herein comprises a 4-1BB co-stimulatory molecule. In some embodiments, a CAR includes a CD28 transmembrane domain, a CD3ζ signaling domain and a CD28 co-stimulatory domain. In some embodiments, a CAR includes a CD8 transmembrane domain, a CD3ζ signaling domain and a 4-1BB co-stimulatory domain. In other embodiments, a CAR includes a CD3ζ signaling domain and 4-1BB co-stimulatory domain. In still other embodiments, a CAR includes a CD3ζ signaling domain, a CD28 co-stimulatory domain, and a 4-1BB co-stimulatory domain.

In a preferred embodiment, the immune cell of the invention or the population thereof comprises an antigen receptor comprising an antigen-binding domain, wherein the antigen receptor is a chimeric antigen receptor (CAR), and wherein said CAR comprises:
- an antigen-binding domain;
- a transmembrane domain, preferably a CD8 or CD28 transmembrane domain;
- an intracellular signaling domain, preferably a CD3ζ endodomain; and
- optionally, a CD28 or a 4-1BB costimulatory domain.

### Preferred CAR-T cells of the invention

The following paragraphs represent preferred embodiments of the CAR-T cell of the invention:
In preferred embodiments, the immune T cell of the invention or the population thereof expresses a CAR, preferably an anti-HER2 CAR, anti-CD19 CAR, or anti-mesothelin CAR, and has been modified to have reduced or eliminated the function and/or the expression of at least one target gene selected from the group of Table 4.

In preferred embodiments, the immune T cell of the invention or the population thereof expresses a CAR, preferably an anti-HER2 CAR, anti-CD19 CAR, or anti-mesothelin CAR, and has been modified to have reduced or eliminated the function and/or the expression of at least one target gene selected from the group of ZC3H12C, UCP3, TG, ITGB8 and LILRB1.

In preferred embodiments, the immune T cell of the invention or the population thereof expresses a CAR, preferably an anti-HER2 CAR, anti-CD19 CAR, or anti-mesothelin CAR, and has been modified to have reduced or eliminated the function and/or the expression of one or more of the target genes selected from the group consisting of ZC3H12C, UCP3, TG, and ITGB8.

In preferred embodiments, the immune T cell of the invention or the population thereof expresses a CAR, preferably an anti-HER2 CAR, anti-CD19 CAR, or anti-mesothelin CAR, and has been modified to have disrupted at least one target gene selected from the group of Table 4.

In preferred embodiments, the immune T cell of the invention or the population thereof expresses a CAR, preferably an anti-HER2 CAR, anti-CD19 CAR, or anti-mesothelin CAR, and has been modified to have disrupted at least one target gene selected from the group of ZC3H12C, UCP3, TG, ITGB8 and LILRB1.

In preferred embodiments, the immune T cell of the invention or the population thereof expresses a CAR, preferably an anti-HER2 CAR, anti-CD19 CAR, or anti-mesothelin CAR, and has been modified to have disrupted at least one of the target genes selected from the group consisting of ZC3H12C, UCP3, TG, and ITGB8.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of ZC3H12C target gene.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of UCP3 target gene.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of TG target gene.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of ITGB8 target gene.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of LILRB1 target gene.

Preferably, the immune T cell of the invention or a population thereof expresses a CAR against HER2 and has been modified to have a reduction or elimination, preferably a statistically significant reduction or elimination, of the function and/or expression of the proteins encoded by at least one of the target genes selected from the group of ZC3H12C, UCP3, TG, ITGB8 and LILRB1, wherein said reduction or elimination is preferably calculated with respect to the same immune T cell without having said modification.

Preferably, the immune T cell of the invention or a population thereof expresses a CAR against HER2 and has been modified to have a reduction or elimination, preferably a statistically significant reduction or elimination, of the function and/or expression of the proteins encoded by at least one of the target genes selected from the group consisting of ZC3H12C, UCP3, TG, and ITGB8, wherein said reduction or elimination is preferably calculated with respect to the same immune T cell without having said modification.

Preferably, the immune T cell of the invention or a population thereof expresses a CAR against CD19 and has been modified to have a reduction or elimination, preferably a statistically significant reduction or elimination, of the function and/or expression of the proteins encoded by at least one of the target genes selected from the group of ZC3H12C, UCP3, TG, ITGB8 and LILRB1, wherein said reduction or elimination is preferably calculated with respect to the same immune T cell without having said modification.

Preferably, the immune T cell of the invention or a population thereof expresses a CAR against CD19 and has been modified to have a reduction or elimination, preferably a statistically significant reduction or elimination, of the function and/or expression of the proteins encoded by at least one of the target genes selected from the group consisting of ZC3H12C, UCP3, TG, and ITGB8, wherein said reduction or elimination is preferably calculated with respect to the same immune T cell without having said modification.

Preferably, the immune T cell of the invention or a population thereof expresses a CAR against mesothelin and has been modified to have a reduction or elimination, preferably a statistically significant reduction or elimination, of the function and/or expression of the proteins encoded by at least one of the target genes selected from the group of ZC3H12C, UCP3, TG, ITGB8 and LILRB1, wherein said reduction or elimination is preferably calculated with respect to the same immune T cell without having said modification.

Preferably, the immune T cell of the invention or a population thereof expresses a CAR against mesothelin and has been modified to have a reduction or elimination, preferably a statistically significant reduction or elimination, of the function and/or expression of the proteins encoded by at least one of the target genes selected from the group consisting of ZC3H12C, UCP3, TG, and ITGB8, wherein said reduction or elimination is preferably calculated with respect to the same immune T cell without having said modification.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of ZC3H12C target gene and UCP3 target gene.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of ZC3H12C target gene and TG target gene.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of ZC3H12C target gene and ITGB8 target gene.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of ZC3H12C target gene and LILRB1 target gene.

In another embodiment, the immune T cell of the invention or the population thereof expresses a CAR, preferably an anti-HER2 CAR, anti-CD19 CAR, or anti-mesothelin CAR, and has been modified to have reduced or eliminated the function and/or the expression of ZC3H12C target gene and PD-1 gene.

Preferably, the immune T cell of the invention is a CAR-T cell and is a double knock-out of the following target genes:
- ZC3H12C and UCP3 genes,
- ZC3H12C and TG genes,
- ZC3H12C and LILRB1 genes,
- ZC3H12C and ITGB8 genes, or
- ZC3H12C and PD-1 genes.

In some embodiments at least 55%, at least 60%, at least 70%, at least 75%, at least 80%, at least 85%, at least 90%, or at least 95% of a population of the immune cells of the invention, preferably CAR-T cells of the invention, does not express a detectable level of at least one of the target genes selected from Table 4, preferably ZC3H12C, UCP3, TG, ITGB8 or LILRB1 target genes, most preferably ZC3H12C, UCP3, TG, or ITGB8 target genes.

### Method for producing the immune cells of the invention

In **a second aspect,** the present invention also relates to a method, preferably an *in vitro* or *ex vivo* method, of producing an immune cell, the method comprising the steps of:
(a) introducing an antigen receptor into an immune cell and
(b) modifying the immune cell to reduce or eliminate the function and/or the expression of one or more of the target proteins encoded by the target genes of Table 4, Table 6, Table 7 or Table 8,
wherein the reduction or elimination the function and/or the expression is calculated with respect the same immune cell without said modification, and wherein steps (a) and (b) may be performed in any order. Preferably, the immune cell is an immune T cell.

Preferably, the method of the second aspect is for producing the immune cell of the invention and comprises the steps of:
(a) introducing into an immune cell an antigen receptor that comprises an antigen-binding domain as defined above in the first aspect or in any of its embodiments; and
(b) modifying the immune cell to reduce or eliminate, preferably to statistically significantly reduce or eliminate, the function and/or the expression of one or more of the target genes selected from the list consisting of ZC3H12C, UCP3, TG, ITGB8 and LILRB1, preferably ZC3H12C, UCP3, TG, and ITGB8,
wherein the reduction or elimination the function and/or the expression is calculated with respect the same immune cell without said modification, and wherein steps (a) and (b) may be performed in any order.

Preferably, the method of the second aspect is for producing the immune cell of the invention and comprises the steps of:
(a) introducing into an immune cell an antigen receptor that comprises an antigen-binding domain as defined above in the first aspect or in any of its embodiments; and
(b) modifying the immune cell to reduce or eliminate, preferably to statistically significantly reduce or eliminate, the function and/or the expression of one or more of the target genes depicted in Table 4, preferably ZC3H12C, UCP3, TG, ITGB8 or LILRB1,
wherein the reduction or elimination the function and/or the expression is calculated with respect the same immune cell without said modification, and wherein steps (a) and (b) may be performed in any order.

Preferably, the method of the second aspect is for producing the immune cell of the invention and comprises the steps of:
(a) introducing into an immune cell an antigen receptor that comprises an antigen-binding domain as defined above in the first aspect or in any of its embodiments; and
(b) modifying the immune cell to reduce or eliminate, preferably to statistically significantly reduce or eliminate, the function and/or the expression of one or more of the target genes selected from the group consisting of ZC3H12C, UCP3, TG, and ITGB8,
wherein the reduction or elimination the function and/or the expression is calculated with respect the same immune cell without said modification, and wherein steps (a) and (b) may be performed in any order.

Step (a) comprises or consists of in introducing into the immune cell an antigen receptor that comprises an antigen-binding domain as defined above in the first aspect or in any of its embodiments. In some embodiments, a nucleic acid encoding an antigen receptor comprising an antigen-binding domain can be introduced into any of the immune cells disclosed herein by methods known to those of skill in the art. For example, a coding sequence of the antigen receptor comprising an antigen-binding domain may be cloned into a vector, which may be introduced into the immune cells for expression of the antigen receptor comprising an antigen-binding domain. A variety of different methods known in the art can be used to introduce any of the nucleic acids or expression vectors disclosed herein into an immune cell. Non-limiting examples of methods for introducing nucleic acid into a cell include: lipofection, transfection (e.g., calcium phosphate transfection, transfection using highly branched organic compounds, transfection using cationic polymers, dendrimer-based transfection, optical transfection, particle-based transfection (e.g., nanoparticle transfection), or transfection using liposomes (e.g., cationic liposomes)), microinjection, electroporation, cell squeezing, sonoporation, protoplast fusion, hydrodynamic delivery, gene gun, magnetofection, viral transduction, and nucleofection.

In some embodiments, a nucleic acid encoding an antigen receptor comprising an antigen-binding domain can be delivered to a cell using a vector, such as an adeno-associated virus (AAV). In a preferred embodiment, the antigen receptor comprising an antigen-binding domain sequences are delivered into the immune cells using a retroviral or lentiviral vector.

A nucleic acid encoding a CAR can be designed to be inserted into a genomic site of interest in the immune cell, to produce the knock-out of the target genes selected from Table 4, preferably ZC3H12C, UCP3, TG, ITGB8 or LILRB1, most preferably target genes selected from the group consisting of ZC3H12C, UCP3, TG, and ITGB8.

A donor template as disclosed herein can contain a coding sequence for a therapeutic transgene. In some examples, the transgene-coding sequence may be flanked by two regions of homology to allow for efficient HDR at a genomic location of interest, for example, at a target gene using a gene editing method known in the art. Alternatively, a donor template may have no regions of homology to the targeted location in the DNA and may be integrated by NHEJ-dependent end joining following cleavage at the target site.

When needed, additional gene editing (e.g., gene knock-in or knock-out) can be introduced into the immune cells as disclosed herein to improve immune cell function and therapeutic efficacy. Other examples include knock-in or knock-out genes to improve target cell lysis, knock-in or knock-out genes to enhance performance of therapeutic T cells such as CAR-T cells.

In some embodiments, a donor template for delivering an anti-HER2 CAR (also referred as a CAR directed to HER2 antigen or targeting HER2 antigen) may be a vector, inserted with a nucleic acid fragment comprising the coding sequence of the anti-HER2 CAR, and regulatory sequences for expression of the anti-HER2 CAR (e.g., a constitutive promoter), which can be flanked by homologous arms for inserting the coding sequence and the regulatory sequences into a genomic locus of interest. In some embodiments, the nucleic acid fragment is inserted in the endogenous gene locus of at least one target gene of Table 4, preferably ZC3H12C, UCP3, TG, ITGB8 or LILRB1, most preferably the target genes selected from the group consisting of ZC3H12C, UCP3, TG, and ITGB8, thereby disrupting expression of the target gene.

In some embodiments, a donor template for delivering an anti-CD19 CAR (also referred as a CAR directed to CD19 antigen or targeting CD19 antigen) may be a vector, inserted with a nucleic acid fragment comprising the coding sequence of the anti-CD19 CAR, and regulatory sequences for expression of the anti-CD19 CAR (e.g., a constitutive promoter), which can be flanked by homologous arms for inserting the coding sequence and the regulatory sequences into a genomic locus of interest. In some embodiments, the nucleic acid fragment is inserted in the endogenous gene locus of at least one target gene of Table 4, preferably ZC3H12C, UCP3, TG, ITGB8 or LILRB1, most preferably the target genes selected from the group consisting of ZC3H12C, UCP3, TG, and ITGB8, thereby disrupting expression of the target gene.

In some embodiments, a donor template for delivering an anti-mesothelin CAR (also referred as a CAR directed to mesothelin antigen or targeting mesothelin antigen) may be a vector inserted with a nucleic acid fragment comprising the coding sequence of the anti- mesothelin CAR, and optionally regulatory sequences for expression of the anti-mesothelin CAR (e.g., constitutive a promoter), which can be flanked by homologous arms for inserting the coding sequence and the regulatory sequences into a genomic locus of interest. In some embodiments, the nucleic acid fragment is inserted in the endogenous gene locus of at least one target gene of Table 4, preferably ZC3H12C, UCP3, TG, ITGB8 or LILRB1, most preferably target genes selected from the group consisting of ZC3H12C, UCP3, TG, and ITGB8, thereby disrupting expression of the target gene.

Step (b) of the method of the second aspect comprises or consists of modifying the immune cell to reduce or eliminate, preferably to statistically significantly reduce or eliminate, the function and/or the expression of the target genes of Table 4. Preferably, step (b) comprises modifying the immune cell to reduce or eliminate, preferably to statistically significantly reduce or eliminate, the function and/or the expression of at least 1, 2, 3, 4, 5, 6, 7, 8, 9, 10, 11, 12, 13 or 14 of the target genes of Table 4, preferably ZC3H12C, UCP3, TG, ITGB8 or LILRB1 target genes, most preferably target genes selected from the group consisting of ZC3H12C, UCP3, TG, and ITGB8, wherein the reduction or elimination is preferably calculated with respect the same immune cell without said modification, as also defined in the first aspect or any of its embodiments.

The immune cell of the invention or a population thereof, the antigen receptor, and the modification to reduce or eliminate the function and/or activity of a target protein encoded by a target gene are to be understood as defined in the first aspect of the invention or any of its embodiments.

The modification of the immune cell to reduce or eliminate the function and/or the expression of the proteins encoded by at least one of the genes and the introduction of an antigen receptor that comprises an antigen-binding domain in the immune cell can be carried out simultaneously or sequentially in any order.

In a preferred embodiment of the method of the second aspect, step (b) comprises introducing into the immune cell CRISPR/Cas-mediated gene editing system that produces the knock-out or gene disruption of one or more of the target genes, preferably ZC3H12C, UCP3, TG, ITGB8 or LILRB1 target genes, most preferably target genes selected from the group consisting of ZC3H12C, UCP3, TG, and ITGB8.

In **a third aspect,** the present invention provides an immune cell, preferably an immune effector cell, obtained or obtainable from the method of the second aspect or any of its embodiments.

### The composition of the invention

In **a fourth aspect,** the invention provides a composition, preferably a pharmaceutical composition, comprising the immune cell, preferably the immune effector cell, or the population thereof according to any of the previous aspects, or any of their embodiments, and a pharmaceutically acceptable excipient.

The pharmaceutical composition and formulation is for administration, such as for immunotherapy, cellular therapy (e.g. immune cell therapy including adoptive cell therapy (ACT)), or gene therapy. The pharmaceutical composition may be formulated using any suitable method. Formulation of cells with standard pharmaceutically acceptable carriers and/or excipients may be carried out using routine methods in the pharmaceutical art. The exact nature of a formulation will depend upon several factors including the cells to be administered and the desired route of administration.

Pharmaceutically acceptable excipients include, but are not limited to a carrier or diluent, such as a gum, a starch (e.g. corn starch, pregeletanized starch), a sugar (e.g. lactose, mannitol, sucrose, dextrose), a cellulosic material (e.g. microcrystaline cellulose), an acrylate (e.g. polymethylacrylate), calcium carbonate, magnesium oxide, talc, or mixtures thereof; a binder (e.g. acacia, cornstarch, gelatin, carbomer, ethyl cellulose, guar gum, hydroxypropyl cellulose, hydroxypropyl methyl cellulose, povidone); a disintegrating agent (e.g. cornstarch, potato starch, alginic acid, silicon dioxide, croscarmelose sodium, crospovidone, guar gum, sodium starch glycolate), a buffer (e.g. Tris-HCl, acetate, phosphate, bicarbonate) of various pH and ionic strength; and additive such as albumin or gelatin to prevent absorption to surfaces; a detergent (e.g. Tween 20, Tween 80, Pluronic F68, bile acid salts); a protease inhibitor; a surfactant (e.g. sodium lauryl sulfate); a permeation enhancer; a solubilizing agent (e.g. glycerol, polyethylene glycerol); an antioxidants (e.g. ascorbic acid, sodium metabisulfite, butylated hydroxyanisole); a stabilizer (e.g. hydroxypropyl cellulose, hydroxypropylmethyl cellulose); a viscosity increasing agent (e.g. carbomer, colloidal silicon dioxide, ethyl cellulose, guar gum); a sweetener (e.g. aspartame, citric acid); a preservative (e.g. thimerosal, benzyl alcohol, parabens); a lubricant (e.g. stearic acid, magnesium stearate, polyethylene glycol, sodium lauryl sulfate); a flow-aid (e.g. colloidal silicon dioxide), a plasticizer (e.g. diethyl phthalate, triethyl citrate); an emulsifier (e.g. carbomer, hydroxypropyl cellulose, sodium lauryl sulfate); a polymer coating (e.g. poloxamers or poloxamines); a coating and film forming agent (e.g. ethyl cellulose, acrylates, polymethacrylates); a pharmaceutically acceptable carrier for liquid formulations, such as an aqueous (water, alcoholic/aqueous solution, emulsion or suspension, including saline and buffered media) or non-aqueous (e.g., propylene glycol, polyethylene glycol, and injectable organic esters, such as ethyl oleate) solution, suspension, emulsion or oil; and a parenteral vehicle (e.g., for subcutaneous, intravenous, intraarterial, or intramuscular injection), including but not limited to, water, oils, saline solution, Ringer's dextrose, aqueous dextrose and other sugar solutions. A pharmaceutically acceptable excipient also includes excipients for nanoencapsulation purposes, such as a cationic polyelectrolyte (e.g. gelatin and an anionic polyelectrolyte (e.g. arabic gum).

In some embodiments, the pharmaceutical composition further comprises other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some embodiments, the immune cell or population thereof is administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, p-toluenesulphonic acid.

### Therapeutic applications

The invention provides in **a fifth aspect** an immune cell, preferably the immune effector cell, of the invention or a population thereof as defined in the first and third aspects, or the pharmaceutical composition as defined in the fourth aspect, or any of their embodiments, for use in therapy. Preferably, the use is in a subject in need thereof. The term "subject" as used herein refers to a mammalian subject. Preferably, it is selected from a human, companion animal, non-domestic livestock or zoo animal. For example, the subject may be selected from a human, mouse, rat, dog, cat, cow, pig, sheep, horse, bear, and so on. In a preferred embodiment, said mammalian subject is a human subject. A subject may be any subject for whom diagnosis, treatment, or therapy is desired. In some embodiments, the subject is a human having cancer.

Thus, the immune cell of the invention can be used as a medicament. The term "medicament" describes a drug or combination of drugs that have the ability to treat diseases, disorders or conditions in subjects or that they can be used in subjects with the aim of restoring, correcting or modifying the physiological functions performing a pharmacological, immunological or metabolic function. The terms "medicament" and "medication" are used herein interchangeably.

The immune cell or population thereof disclosed herein is administered to a subject for therapeutic purposes, for example, treatment of a solid tumor targeted by the CAR construct expressed and/or comprised by the immune cells.

The step of administering may include the placement (e.g., transplantation) of the immune cell or population thereof into a subject by a method or route that results in at least partial localization of the immune cells at a desired site, such as a tumor site, such that a desired effect(s) can be produced. The immune cell or population thereof can be administered by any appropriate route that results in delivery to a desired location in the subject where at least a portion of the implanted cells or components of the cells remain viable. The period of viability of the cells after administration to a subject can be as short as a few hours, e.g., twenty-four hours, to a few days, to as long as several years, or even the lifetime of the subject, i.e., long-term engraftment. For example, in some embodiments described herein, an effective amount of the immune cells can be administered via a systemic route of administration, such as an intraperitoneal or intravenous route.

In some embodiments, the immune cells of the invention are administered systemically, which refers to the administration of a population of cells other than directly into a target site, tissue, or organ, such that it enters, instead, the subject's circulatory system and, thus, is subject to metabolism and other like processes. Suitable modes of administration include injection, infusion, instillation, or ingestion. Injection includes, without limitation, intravenous, intramuscular, intra-arterial, intrathecal, intraventricular, intracapsular, intraorbital, intracardiac, intradermal, intraperitoneal, transtracheal, subcutaneous, subcuticular intraarticular, sub capsular, subarachnoid, intraspinal, intracerebrospinal, and intrasternal injection and infusion. In some embodiments, the route is intravenous.

In an embodiment, the immune cell of the invention, or population thereof, as defined in the first or aspect, the immune cell of the invention obtained or obtainable from the method of the second aspect as defined in the third aspect, or the composition as defined in the fourth aspect or any of their embodiments is administrated to a subject in need thereof in an effective amount. An "**effective amount**" refers to the amount of an immune cell of the invention or a population thereof needed to treat or alleviate at least one or more signs or symptoms of a medical condition (e.g., cancer), and relates to a sufficient amount of a composition to provide the desired effect, e.g., to treat a subject having a medical condition. Preferably, the disease treated is cancer.

The **sixth aspect** of the invention relates to the immune cell, preferably the immune effector cell, of the invention or the population thereof as defined in the first or third aspect, the pharmaceutical composition according to the fourth aspect, or any of their embodiments, for use in the treatment of cancer. The terms "**cancer**" and "**tumor**" are used herein interchangeably.

In a related aspect, the invention also refers to the use of the immune cell, preferably the immune effector cell, of the invention or the population thereof as defined in the first or third aspect, the pharmaceutical composition according to the fourth aspect, or any of their embodiments, for the manufacture of a medicament for the treatment of cancer.

The cancer may be a non-solid tumor (such as a hematological tumor) or a solid tumor. Adult tumors/cancers and pediatric tumors/cancers are also included. In one embodiment, the cancer is a solid tumor or a hematological tumor. In one embodiment, the cancer is a carcinoma. In one embodiment, the cancer is a sarcoma. In one embodiment, the cancer is a leukemia. In a preferred embodiment, the cancer is a solid tumor.

In a preferred embodiment, the immune cell of the invention or the population thereof as defined in the first aspect, the immune cell of the invention obtained or obtainable from the method of the second aspect or the composition of the invention is used in the treatment of a cancer selected from breast cancer, ovarian cancer, lung cancer and/or pancreatic cancer.

In another preferred embodiment, the immune cell of the invention or the population thereof as defined in the first aspect, the immune cell of the invention obtained or obtainable from the method of the second aspect or the composition of the invention are used in the treatment of a cancer that expresses an antigen or that said cancer is positive for an antigen expression, e.g. an HER2-expressing cancer or HER2-positive cancer, a CD19-expressing cancer or CD19-positive cancer, or a mesothelin-expressing cancer or mesothelin-positive cancer.

"**HER2-expressing cancer**" or "HER2-positive cancer" refers to a one comprising cells, which overexpress HER2 present at their cell surface.

"**CD19-expressing cancer**" or "CD19-positive cancer" refers to a one comprising cells, which express CD19 present at their cell surface. The term "CD19-expressing cancer" also refers to a cancer that produces sufficient levels of CD19 at the surface of cells thereof, such that a CAR-comprising cell of the present invention has a therapeutic effect, mediated by the binding of the CAR to CD19. In addition, "CD19-expressing cancer" also relates to a cancer that does not naturally express CD19 antigen but said cancer cell has been genetically modified to express said antigen.

"**Mesothelin-expressing cancer**" or "mesothelin-positive cancer" refers to a one comprising cells, which express mesothelin present at their cell surface. The term "mesothelin-expressing cancer" also refers to a cancer that produces sufficient levels of mesothelin at the surface of cells thereof, such that a CAR-comprising cell of the present invention has a therapeutic effect, mediated by the binding of the CAR to mesothelin. In addition, "mesothelin-expressing cancer" also relates to a cancer that does not naturally express mesothelin antigen but said cancer cell has been genetically modified to express said antigen.

Preferably, the CAR-T cell of the invention or a population thereof expresses a CAR against HER2, and it has been modified to reduce or eliminate, preferably to statistically significantly reduce or eliminate, the function and/or the expression of the proteins encoded by at least one of the target genes selected from Table 4, Table 6, Table 7 or Table 8, wherein the reduction or elimination is preferably calculated with respect to the same T cell without having said modification, and wherein said CAR-T cell of the invention is used in the treatment of a HER2-positive cancer.

Preferably, the CAR-T cell of the invention or a population thereof expresses a CAR against HER2, and it has been modified to reduce or eliminate, preferably to statistically significantly reduce or eliminate, the function and/or the expression of the proteins encoded by at least one of the target genes selected from the group consisting of ZC3H12C, UCP3, TG, ITGB8 and LILRB1, wherein the reduction or elimination is preferably calculated with respect to the same T cell without having said modification, and wherein said CAR-T cell of the invention is used in the treatment of a HER2-positive cancer.

Preferably, the CAR-T cell of the invention or a population thereof expresses a CAR against HER2, and it has been modified to reduce or eliminate, preferably to statistically significantly reduce or eliminate, the function and/or the expression of the proteins encoded by at least one target genes selected from the group consisting of ZC3H12C, UCP3, TG, and ITGB8, wherein the reduction or elimination is preferably calculated with respect to the same T cell without having said modification, and wherein said CAR-T cell of the invention is used in the treatment of a HER2-positive cancer.

Preferably, the CAR-T cell of the invention or a population thereof expresses a CAR against mesothelin, and it has been modified to reduce or eliminate, preferably to statistically significantly reduce or eliminate, the function and/or the expression of the proteins encoded by at least one of the target genes selected from Table 4, Table 6, Table 7, or Table 8, wherein the reduction or elimination is preferably calculated with respect to the same T cell without having said modification, and wherein said CAR-T cell of the invention is used in the treatment of a mesothelin-positive cancer.

Preferably, the CAR-T cell of the invention or a population thereof expresses a CAR against mesothelin, and it has been modified to reduce or eliminate, preferably to statistically significantly reduce or eliminate, the function and/or the expression of the proteins encoded by at least one of the target genes selected from the group consisting of ZC3H12C, UCP3, TG, ITGB8 and LILRB1, wherein the reduction or elimination is preferably calculated with respect to the same T cell without having said modification, and wherein said CAR-T cell of the invention is used in the treatment of a mesothelin-positive cancer.

Preferably, the CAR-T cell of the invention or a population thereof expresses a CAR against mesothelin, and it has been modified to reduce or eliminate, preferably to statistically significantly reduce or eliminate, the function and/or the expression of the proteins encoded by at least one of the target genes selected from the group consisting of ZC3H12C, UCP3, TG, and ITGB8, wherein the reduction or elimination is preferably calculated with respect to the same T cell without having said modification, and wherein said CAR-T cell of the invention is used in the treatment of a mesothelin-positive cancer.

Preferably, the CAR-T cell of the invention or a population thereof expresses a CAR against CD19, and it has been modified to reduce or eliminate, preferably to statistically significantly reduce or eliminate, the function and/or the expression of the proteins encoded by at least one of the target genes selected from Table 4, Table 6, Table 7 or Table 8, wherein the reduction or elimination is preferably calculated with respect to the same T cell without having said modification, and wherein said CAR-T cell of the invention is used in the treatment of a CD19-positive cancer.

Preferably, the CAR-T cell of the invention or a population thereof expresses a CAR against CD19, and it has been modified to reduce or eliminate, preferably to statistically significantly reduce or eliminate, the function and/or the expression of the proteins encoded by at least one of the target genes selected from the group consisting of ZC3H12C, UCP3, TG, ITGB8 and LILRB1, wherein the reduction or elimination is preferably calculated with respect to the same T cell without having said modification, and wherein said CAR-T cell of the invention is used in the treatment of a CD19-positive cancer.

Preferably, the CAR-T cell of the invention or a population thereof expresses a CAR against CD19, and it has been modified to reduce or eliminate, preferably to statistically significantly reduce or eliminate, the function and/or the expression of the proteins encoded by at least one of the target genes selected from the group consisting of ZC3H12C, UCP3, TG, and ITGB8, wherein the reduction or elimination is preferably calculated with respect to the same T cell without having said modification, and wherein said CAR-T cell of the invention is used in the treatment of a CD19-positive cancer.

In some embodiments, the immune cell of the invention or the population thereof, the composition of the invention or the immune cell obtained or obtainable from the method of the second aspect is administered as part of a combination treatment or therapy, such as simultaneously with or sequentially with, in any order, another therapeutic intervention, such as an antibody or engineered cell or receptor or agent, such as a cytotoxic or therapeutic agent. In some embodiments, the methods comprise administration of a chemotherapeutic agent. The term "**combination therapy**" as used throughout the specification, is meant to encompass the administration of the cells of the invention together with other therapeutic agents or drugs, to a subject suffering from cancer, in the same or separate pharmaceutical formulations, and at the same time or at different times.

In certain embodiments, the immune cells of the invention, the composition of the invention or the immune cell obtained or obtainable from the method of the second aspect is administered to a subject in combination with an immune checkpoint antibody (e.g., an anti-PD1, anti-CTLA-4, or anti-PDL1 antibody).

Following administration of the immune cells of the invention, the biological activity of the immune cell or the population thereof in some embodiments is measured by any known methods. Parameters to assess the efficiency of the immune cells of the invention include specific binding of an engineered or natural T cell or other immune cell to antigen, *in vivo,* e.g., by imaging, or *ex vivo,* e.g., by ELISA or flow cytometry. In certain embodiments, the ability of the immune cells of the invention to bind and destroy target cells can be measured using any suitable method known in the art. In certain embodiments, the biological activity of the immune cells of the invention, preferably the immune effector cell or a population thereof, is measured by assaying expression and/or secretion of one or more cytokines, such as IFN-y, IL-2, and TNF-α. In some embodiments the biological activity is measured by assessing clinical outcome, such as reduction in tumor burden, tumor load or tumor growth or probability of survival.

In a **seventh aspect,** the invention refers to a method of treating cancer in a subject in need thereof, comprising:
(a) administering to a subject an effective amount of the immune cell of the invention as defined in the first and third aspects, or the pharmaceutical composition as defined in the fourth aspect, or any of their embodiments, where the immune cell expresses an antigen receptor that specifically binds to an antigen expressed by said cancer; and
(b) administering an effective amount of an agent to the subject, wherein the agent reduces or eliminates the function and/or expression of at least 1, 2, 3, 4, 5, 6, 7, 8, 9, 10, 11, 12, 13 or 14 of the target genes of Table 4, preferably ZC3H12C, UCP3, TG, ITGB8 or LILRB1 target genes;
wherein (a) and (b) are carried out simultaneously, sequentially or concurrently.

Preferably, the seventh aspect refers to a method of treating cancer in a subject in need thereof, comprising:
(a) administering to a subject an effective amount of the immune cell of the invention as defined in the first and third aspects, or the pharmaceutical composition as defined in the fourth aspect, or any of their embodiments, where the immune cell expresses an antigen receptor that specifically binds to an antigen expressed by said cancer; and
(b) administering an effective amount of an agent to the subject, wherein the agent reduces or eliminates the function and/or expression of at least 1, 2, 3, or 4 of the target genes selected from the group consisting of ZC3H12C, UCP3, TG, and ITGB8;
wherein (a) and (b) are carried out simultaneously, sequentially or concurrently.

In a related aspect, the invention also provides an agent for use in treating cancer in a subject, wherein the subject has received or is receiving the immune cell of the invention as defined in the first and third aspects, or the pharmaceutical composition as defined in the fourth aspect, or any of their embodiments, and wherein the agent reduces or eliminates the function and/or expression of at least 1, 2, 3, 4, 5, 6, 7, 8, 9, 10, 11, 12, 13 or 14 of the target genes of Table 4, preferably ZC3H12C, UCP3, TG, ITGB8 or LILRB1 target genes, most preferably target genes selected from the group consisting of ZC3H12C, UCP3, TG, and ITGB8.

In **an eighth aspect,** the present invention refers to a method for:
- increasing the anti-tumor effect or capacity of an immune cell, preferably an immune effector cell, in comparison to a reference cell,
- increasing the survival of an immune cell, preferably an immune effector cell, in comparison to a reference cell,
- increasing the cytokine release and/or production of an immune cell, preferably an immune effector cell, in comparison to a reference cell, and/or
- generating immune cells, preferably an immune effector cell, with reduced cell dysfunction in comparison to a reference cell,
the method comprising modifying the immune cell to reduce or eliminate, preferably to statistically significantly reduce or eliminate, the function and/or the expression of the proteins encoded the one or more of the target genes selected from Table 4, preferably Table 6, 7 or 8, and wherein the reduction or elimination is preferably calculated with respect to a reference cell as defined in the first aspect or any of its embodiments.

Preferably, the method is for:
- increasing the anti-tumor effect or capacity of an immune cell, preferably an immune effector cell, in comparison to a reference cell,
- increasing the survival of an immune cell, preferably an immune effector cell, in comparison to a reference cell,
- increasing the cytokine release and/or production of an immune cell, preferably an immune effector cell, in comparison to a reference cell, and/or
- generating immune cells, preferably an immune effector cell, with reduced cell dysfunction in comparison to a reference cell,
the method comprising modifying the immune cell to reduce or eliminate, preferably to statistically significantly reduce or eliminate, the function and/or the expression of the proteins encoded the one or more of the target genes selected from Table 4, preferably ZC3H12C, UCP3, TG, ITGB8 or LILRB1, and wherein the reduction or elimination is preferably calculated with respect to a reference cell as defined in the first aspect or any of its embodiments.

In an embodiment of the eight aspect, the present invention refers to a method for:
- increasing the anti-tumor effect or capacity of an immune cell, preferably an immune effector cell, in comparison to a reference cell,
- increasing the survival of an immune cell, preferably an immune effector cell, in comparison to a reference cell,
- increasing the cytokine release and/or production of an immune cell, preferably an immune effector cell, in comparison to a reference cell, and/or
- generating immune cells, preferably an immune effector cell, with reduced cell dysfunction in comparison to a reference cell,
the method comprising modifying the immune cell to reduce or eliminate, preferably to statistically significantly reduce or eliminate, the function and/or the expression of the proteins encoded the one or more of the target genes selected from the group consisting of ZC3H12C, UCP3, TG, and ITGB8, and wherein the reduction or elimination is preferably calculated with respect to a reference cell as defined in the first aspect or any of its embodiments.

Preferably, the method comprises modifying the immune cell to reduce or eliminate, preferably to statistically significantly reduce or eliminate, the function and/or the expression of the proteins encoded by at least 1, 2, 3, 4, 5, 6, 7, 8, 9, 10, 11, 12, 13 or 14 of target genes selected from Table 4, preferably ZC3H12C, UCP3, TG, ITGB8 or LlLRB1, most preferably target genes selected from the group consisting of ZC3H12C, UCP3, TG, and ITGB8, and wherein the reduction or elimination is preferably calculated with respect to the same immune cell without having said modification.

Preferably, the method of the eighth aspect is a method for:
- increasing the anti-tumor effect or capacity of an immune cell, preferably an immune effector cell, in comparison to a reference cell,
- increasing the survival of an immune cell, preferably an immune effector cell, in comparison to a reference cell,
- increasing the cytokine release and/or production of an immune cell, preferably an immune effector cell, in comparison to a reference cell and/or
- generating immune cells, preferably an immune effector cell, with a reduced cell dysfunction in comparison to a reference cell,
the method comprising modifying the immune cell to reduce or eliminate, preferably to statistically significantly reduce or eliminate, the function and/or the expression of the proteins encoded by one or more of ZC3H12C, UCP3, TG, ITGB8 or LILRB1 target genes, preferably target genes selected from the group consisting of ZC3H12C, UCP3, TG, and ITGB8, and wherein the reduction or elimination is preferably calculated with respect to the said reference cell without having said modification.

"**The anti-tumor effect or capacity**" of an immune refers to its ability to inhibit tumor growth, reduce tumor size, or eliminate cancerous cells. An increase of the anti-tumour effect or capacity refers to an augment of the anti-tumor effect or capacity of at least 10%, 20%, 30%, 40%, 50%, 60%, 70%, 80%, 90%, 100%, 150%, 200%, 250%, 300%, 350%, 400%, 450%, 500% or more as compared to the anti-tumor effect or capacity of a reference cell. It can be measured as a reduction in tumor volume or in tumor growth or as an increase of the probability of survival in comparison to a reference cell. The anti-tumor effect of an immune cell can be measured by methods known in the art, such as cell viability assays, xenograft tumour models, etc.

"**Survival of an immune cell**" as used herein refers to the ability of an immune cell to continue to grow, proliferate, survive, self-renew, expand, and/or maintain healthy activity in culture or in vivo. An increase of the survival may refer to an augment of the longevity of the immune cell by at least 10%, 20%, 30%, 40%, 50%, 60%, 70%, 80%, 90%, 100%, 150%, 200%, 250%, 300%, 350%, 400%, 450%, 500% or more as compared to the longevity of a reference cell. The longevity of immune cells can be measured by conventional methods and/or assays described in the art, such as flow cytometry, proliferation assays, TUNEL assay, etc. In other embodiments, survival of the immune cell may be represented by the reduction of cell death (e.g., apoptosis) or reduction in cell states characterized by exhaustion or replicative senescence. In yet other embodiments, survival of the immune cell may be presented by the maintenance of immune cell activation capacity in culture.

In some embodiments, an increase of the persistence or proliferation of an immune cell of the invention or a population thereof may be measured as an augment of the immune cells (e.g. cells/volume of blood, population doublings) over a period of time in comparison to a reference cell. Said augment or increase of the persistence or proliferation may be at least 2x, 3x, 4x, 5x, 6x, 7x, 8x, 9x, 10x, 11x, 12x, 13x, 14x, 15x, 16x, 17x, 18x, 19x, 20x, or more. Said period of time may be 0.5 days, 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days or more in comparison to a reference cell.

"**Cytokine release and/or production of an immune cell**" as used herein refers to the process by which immune cells synthesize and secrete cytokines. An increase of the cytokine release and/or production refers to an augment of the amount of cytokines produced and/or secreted by the immune cell by at least 10%, 20%, 30%, 40%, 50%, 60%, 70%, 80%, 90%, 100%, 150%, 200%, 250%, 300%, 350%, 400%, 450%, 500% or more as compared to the cytokine production and/or secretion of a reference cell. Cytokine production and/or secretion can be measured by methods known in the art, such as ELISA, flow cytometry, ELISPOT, in vivo assays, etc.

In an embodiment, an increase of the cytokine release or cytokine production of an immune cell or a population thereof may be measured as an augment of a cytokine level or concentration over a period of time. The augment of a cytokine level or concentration may be at least of 0.1x, 0.2x, 0.3x, 0.4x, 0.5x, 0.6x, 0.7x, 0.8x, 0.9x, 1.0x, 1.1x, 1.2x, 1.3x, 1.4x, 1.5x, 1.6x, 1.7x, 1.8x, 1.9x, 2.0x, 2.1x, 2.2x, 2.3x, 2.4x, 2.5x, 2.6x, 2.7x, 2.8x, 2.9x, 3.0x, 3.1x, 3.2x, 3.3x, 3.4x, 3.5x, 3.6x, 3.7x, 3.8x, 3.9x, 4.0x, 4.1x, 4.2x, 4.3x, 4.4x, 4.5x, 4.6x, 4.7x, 4.8x, 4.9x, 5.0x or more. The cytokine refers to any cytokine that is involved in the immune cell activity upon antigen binding. Exemplary cytokines can be TNF-α, IFN-γ, IL-2 and/or granzyme B.

"**Cell dysfunction**" or "**cell exhaustion**" is a state of immune cells that may arise in chronic infections and cancer. Cell dysfunction leads to impaired biological processes. This can affect various cellular activities such as metabolism, signaling, proliferation, differentiation, immune response, and apoptosis. An immune cell that is dysfunctional refers to immune cells that are not able to expand, secrete cytokines or lyse target cells, survive after removal of antigen stimulation or all do all the above upon secondary antigen challenge in comparison to a reference cell. Cell dysfunction can comprise cell exhaustion, senescence and anergy. An immune cell has reduced cell dysfunction when it maintains better functional capacity in key immune activities, such as when the immune cell is more effective in killing tumor cells, producing cytokines, proliferating, etc, with fewer signs of impairment.

A reference cell in the context of the eighth aspect is the same as defined in the first aspect, that is, it refers to a reference cell or population thereof that has not been modified to have reduced or eliminated the function and/or the expression of the target genes or proteins. Similar as above, the reference cell or its population preferably refers to a cell or group of cells of the same type as the modified immune cell to which it is being compared.

Preferably, the method of the eighth aspect is an *in vitro* method.

Preferably, the method of the eighth aspect comprises modifying the immune cell, preferably an immune T cell, to have reduced or eliminated the function and/or the expression of:
- ZC3H12C target protein, and
- one or more target proteins selected from the groups consisting of UCP3, TG, ITGB8, LILRB1, AHNAK2, STON1, MYH6, PTK7, INSRR, ACTG2, ADAM12, FN1, and COL4A1, more preferably selected from the group consisting of UCP3, TG, ITGB8, and LILRB1 target genes.

Preferably, the method of the eighth aspect comprises modifying the immune cell, preferably an immune T cell, to disrupt:
- ZC3H12C target gene, and
- one or more of UCP3, TG, ITGB8, LILRB1, AHNAK2, STON1, MYH6, PTK7, INSRR, ACTG2, ADAM12, FN1, and COL4A1, more preferably one or more of UCP3, TG, ITGB8, and LILRB1 target genes.

### Kits of the invention

The present invention also provides kits, and kit for use in producing the immune cell, preferably the immune effector cell, or the population thereof as defined in any of the previous aspects and for therapeutic uses as defined in any of the previous aspects.

In an embodiment, the kit comprises:
a) components for reducing or eliminating the function and/or expression of one or more of the target genes of Table 4, preferably Tables 6, 7 or 8, and
b) components for introducing the antigen receptor in an immune cell, preferably an immune T cell.

In an embodiment, the kit comprises:
a) components for reducing or eliminating the function and/or expression of one or more of the target genes of Table 4, preferably Tables 6, 7 or 8, and
b) at least one viral vector encoding the antigen receptor, preferably the CAR as defined above.

In an embodiment, the kit comprises:
a) components for reducing or eliminating the function and/or expression of one or more of the genes of Table 4, preferably of ZC3H12C, UCP3, TG, ITGB8 or LILRB1, most preferably target genes selected from the group consisting of ZC3H12C, UCP3, TG, and ITGB8, and
b) at least one viral vector encoding the antigen receptor, preferably the CAR as defined above.

In an embodiment, the kit comprises:
a) components for performing knock-out of one or more of the genes of Table 4, preferably of ZC3H12C, UCP3, TG, ITGB8 or LILRB1, most preferably target genes selected from the group consisting of ZC3H12C, UCP3, TG, and ITGB8, and
b) at least one viral vector encoding the antigen receptor, preferably the CAR as defined above.

Thus, in some embodiments, a kit provided herein may comprise a population of immune cells, such as immune T cells, and/or components for reducing or eliminating the function and/or expression of at least 1, 2, 3, 4, 5, 6, 7, 8, 9, 10, 11, 12, 13 or 14 of the target genes selected from Table 4, preferably ZC3H12C, UCP3, TG, ITGB8 or LILRB1, most preferably target genes selected from the group consisting of ZC3H12C, UCP3, TG, and ITGB8, in said immune cells.

In some embodiments, a kit provided herein may comprise components for performing genetic edit of at least 1, 2, 3, 4, 5, 6, 7, 8, 9, 10, 11, 12, 13 or 14 of the target genes selected from Table 4, preferably ZC3H12C, UCP3, TG, ITGB8 or LILRB1, most preferably target genes selected from the group consisting of ZC3H12C, UCP3, TG, and ITGB8, and optionally an immune cell or a population thereof to which the genetic editing will be performed. Preferably, said components for genetic edit are CRISPR/Cas-mediated gene editing system. The components for genetically editing one or more of the target genes may comprise a suitable endonuclease such as an RNA-guided endonuclease and one or more nucleic acid guides, which direct cleavage of one or more suitable genomic sites by the endonuclease. For example, the kit may comprise a Cas enzyme such as Cas 9 and one or more gRNAs targeting at least 1, 2, 3, 4, 5, 6, 7, 8, 9, 10, 11, 12, 13 or 14 of the target genes selected from Table 4, preferably ZC3H12C, UCP3, TG, ITGB8 or LILRB1, most preferably target genes selected from the group consisting of ZC3H12C, UCP3, TG, and ITGB8. Any of the gRNAs specific to these target genes can be included in the kit. Such a kit may further comprise components for further gene editing, for example, gRNAs and optionally additional endonucleases for editing other target genes.

In an embodiment, the kit comprises components for performing knock-out of one or more of the target genes of Table 4, preferably of ZC3H12C, UCP3, TG, ITGB8 or LILRB1, most preferably target genes selected from the group consisting of ZC3H12C, UCP3, TG, and ITGB8.

In an embodiment, the kit comprises components for introducing the antigen receptor in an immune cell. Preferably, said components are viral vectors, such as lentivirus vector, encoding for the antigen receptor of interest, preferably the CAR of interest. In an embodiment, the components for introducing the antigen receptor in an immune cell are components for the permanent expression of the antigen receptor in said immune cell, such as components for the gene editing of the genome of the immune cell to include a nucleic acid encoding for the antigen receptor of interest.

In an embodiment, the kit comprises component for performing a double knock-out of one or of the following target genes:
- ZC3H12C and UCP3 genes,
- ZC3H12C and TG genes,
- ZC3H12C and LILRB1 genes,
- ZC3H12C and ITGB8 genes, or
- ZC3H12C and PD-1 genes.

In some embodiments, a kit provided herein may comprise the immune cells, preferably the immune T cells as disclosed herein, and one or more components for producing the immune cells, preferably the immune T cells as also disclosed herein. Such components may comprise an endonuclease suitable for gene editing and a nucleic acid coding for an antigen receptor of interest. In some embodiments, the antigen receptor may be encoded by a viral vector such as a lentiviral vector.

In yet other embodiments, the kit disclosed herein may comprise immune cells as disclosed for the intended therapeutic purposes.

Any of the kit disclosed herein may further comprise instructions for making the immune cells, or therapeutic applications of the therapeutic immune cells. In some embodiments, the included instructions may comprise a description of using the gene editing components to genetically engineer one or more of the target genes of Table 4, or a combination thereof. In other examples, the included instructions may comprise a description of how to introduce a nucleic acid encoding a CAR construct into the immune cell or population thereof for making therapeutic immune cells of the invention.

Alternatively, the kit may further comprise instructions for administration of the immune cells as disclosed herein to achieve the intended activity, e.g., eliminating disease cells targeted by the CAR expressed on the therapeutic immune cells. The kit may further comprise a description of selecting a subject suitable for treatment based on identifying whether the subject is in need of the treatment. The instructions relating to the use of the therapeutic immune cells described herein generally include information as to dosage, dosing schedule, and route of administration for the intended treatment. The containers may be unit doses, bulk packages (e.g., multi-dose packages) or sub-unit doses. Instructions supplied in the kits of the disclosure are typically written instructions on a label or package insert. The label or package insert indicates that the therapeutic T cells are used for treating, delaying the onset, and/or alleviating a disease or disorder in a subject.

The kits provided herein are in suitable packaging. Suitable packaging includes, but is not limited to, vials, bottles, jars, flexible packaging, and the like. Also contemplated are packages for use in combination with a specific device, such as an infusion device for administration of the therapeutic immune cells. A kit may have a sterile access port (for example, the container may be an intravenous solution bag or a vial having a stopper pierceable by a hypodermic injection needle). The container may also have a sterile access port.

Kits optionally may provide additional components such as buffers and interpretive information. Normally, the kit comprises a container and a label or package insert(s) on or associated with the container. In some embodiment, the disclosure provides articles of manufacture comprising contents of the kits described above.

It is contemplated that any embodiment discussed in this specification can be implemented with respect to any medical use, pharmaceutical composition, method of treatment, method of manufacturing a medicament and combination therapies of the invention, and vice versa. It will be understood that particular embodiments described herein are shown by way of illustration and not as limitations of the invention. The principal features of this invention can be employed in various embodiments without departing from the scope of the invention. Those skilled in the art will recognize or be able to ascertain using no more than routine experimentation, numerous equivalents to the specific procedures described herein. Such equivalents are considered to be within the scope of this invention and are covered by the claims.

The present invention also comprises the following items:
1. An immune cell, preferably an immune effector cell, or a population thereof expressing an antigen receptor, wherein:
the antigen receptor comprises an antigen-binding domain, and
the immune cell has been modified to have reduced or eliminated the function and/or the expression of proteins encoded by at least one of the genes ZC3H12C, UCP3, TG or ITGB8, wherein the reduction or elimination is calculated with respect to a reference cell, preferably with respect to the same immune cell without having said modification.
2. The immune cell or the population thereof according to item 1, wherein the modification is selected from an insertion, a frameshift mutation, a missense mutation, a deletion, a knock-in, a knock-down, or a knock-out of the gene or part of the gene, or deletion of the entire gene.
3. The immune cell or the population thereof according to items 1 or 2, wherein the immune cell is a T cell, optionally wherein the T cell is a CD4⁺ T cell, a CD8⁺ T cell or a CD4⁺/CD8⁺ T cell.
4. The immune cell or the population thereof according to any one of items 1 to 3, wherein the immune cell is selected from the group consisting of cytotoxic T-cell, helper T-cell, memory T-cell, regulatory T-cell, tissue infiltrating lymphocyte, γδ T-cell and any combination thereof.
5. The immune cell or the population thereof according to items 1 to 4, wherein the antigen receptor is a chimeric antigen receptor (CAR), and wherein said CAR comprises:
- an antigen-binding domain;
- a transmembrane domain, preferably a CD8 or CD28 transmembrane domain;
- an intracellular signaling domain, preferably a CD3ζ endodomain; and
- optionally, a CD28 or 4-1BB costimulatory domain.
6. The immune cell or the population thereof according to items 1 to 5, wherein the antigen-binding domain of the antigen receptor binds an antigen selected from the group consisting of HER2, CD19 or mesothelin.
7. The immune cell or the population thereof according to items 1 to 6, wherein the immune cell is autologous.
8. The immune cell or the population thereof according to items 1 to 6, wherein the immune cell is allogenic.
9. The immune cell or the population thereof according to items 1 to 8, wherein the immune cell has been modified to have reduced or eliminated the function and/or the expression of the proteins encoded by:
- at least two genes selected from the group of ZC3H12C, UCP3, TG or ITGB8; or
- at least three genes selected from the group of ZC3H12C, UCP3, TG or ITGB8; or
- at least four genes selected from the group of ZC3H12C, UCP3, TG or ITGB8.
10. An *in vitro* method of producing an immune cell or a population thereof, the method comprising the steps of:
(a) introducing into the immune cell an antigen receptor that comprises an antigen-binding domain; and
(b) modifying the immune cell to reduce or eliminate the function and/or the expression of the proteins encoded by at least one of the genes ZC3H12C, UCP3, TG or ITGB8, wherein the reduction or elimination is calculated with respect the same immune cell without said modification.
11. The method of item 10, wherein step (b) comprises introducing into the immune cell a DNA binding protein or DNA-binding nucleic acid that specifically binds to or hybridizes to the gene or optionally introducing a zinc finger nuclease (ZFN), a TAL-effector nuclease (TALEN), or and a CRISPR-Cas9 combination that specifically binds to, recognizes, or hybridizes to at least one of the genes ZC3H12C, UCP3, TG or ITGB8.
12. The method of items 10 or 11, wherein step (b) comprises modifying the immune cell to reduce or eliminate the function and/or the expression of the proteins encoded by:
- at least two genes selected from the group of ZC3H12C, UCP3, TG or ITGB8; or
- at least three genes selected from the group of ZC3H12C, UCP3, TG or ITGB8; or
- at least four genes selected from the group of ZC3H12C, UCP3, TG or ITGB8.
13. A cell of a population thereof obtained or obtainable from the method according to any of items 10 to 12.
14. A pharmaceutical composition comprising the immune cell or the population thereof according to any one of items 1 to 9 or 13, and a pharmaceutically acceptable excipient.
15. The immune cell or the population thereof as defined in items 1 to 9 or 13, or the pharmaceutical composition according to item 14, for use in therapy.
16. The immune cell or the population thereof as defined in items 1 to 9 or 13, or the pharmaceutical composition according to item 14, for use in the treatment of cancer.
17. The immune cell or the population thereof for use according to item 16, wherein the antigen-binding domain of the antigen receptor binds an antigen selected from the group consisting of HER2, CD19 or mesothelin, and wherein the cancer is selected from the group consisting of an HER2-positive cancer, a CD19-positive cancer or a mesothelin-positive cancer.
18. An *in vitro* method for increasing the anti-tumor effect or capacity of an immune cell or a population thereof expressing an antigen receptor that comprises an antigen-binding domain, the method comprising a step of
modifying the immune cell to reduce or eliminate the function and/or the expression of the proteins encoded by at least one of the genes ZC3H12C, UCP3, TG or ITGB8, wherein said reduction or elimination is calculated with respect to the same immune cell without having said modification; and
wherein the anti-tumor effect of the immune cell or the population thereof is increased with respect to the same immune cell without having said modification.
19. An *in vitro* method for increasing the persistence or proliferation of an immune cell, preferably an immune effector cell, or a population thereof expressing an antigen receptor that comprises an antigen-binding domain, the method comprising a step of
modifying the immune cell to reduce or eliminate the function and/or the expression of the proteins encoded by at least one of the genes ZC3H12C, UCP3, TG or ITGB8; wherein said reduction or elimination is calculated with respect to the same immune cell without having said modification; and
wherein the persistence or proliferation of the immune cell or the population thereof is increased with respect to the same immune cell without having said modification.
20. An *in vitro* method for increasing the cytokine release and/or production of an immune cell, preferably an immune effector cell, or a population thereof expressing an antigen receptor that comprises an antigen-binding domain, the method comprising a step of
modifying the immune cell to reduce or eliminate the function and/or the expression of the proteins encoded by at least one of the genes ZC3H12C, UCP3, TG or ITGB8 wherein said reduction or elimination is calculated with respect to the same immune cell without having said modification; and
wherein the cytokine release/production of the immune cell or the population thereof is increased with respect to the same immune cell without having said modification.
21. An *in vitro* method of producing an immune cell or a population thereof expressing an antigen receptor that comprises an antigen-binding domain, wherein the immune cell or the population thereof has reduced cell dysfunction, the method comprising the step of
modifying the immune cell to reduce or eliminate the function and/or the expression of the proteins encoded by at least one of the genes ZC3H12C, UCP3, TG or ITGB8, wherein said reduction or elimination is calculated with respect to the same immune cell without having said modification; and
wherein the reduction in cell dysfunction of the immune cell or the population thereof is reduced with respect to the same immune cell without having said modification.
22. The method according to any of items 18 to 21, wherein the immune cell has been modified to have reduced or eliminated the function and/or the expression of the proteins encoded by:
- at least two genes selected from the group of ZC3H12C, UCP3, TG or ITGB8; or
- at least three genes selected from the group of ZC3H12C, UCP3, TG or ITGB8; or
- at least four genes selected from the group of ZC3H12C, UCP3, TG or ITGB8.
23. An immune cell, preferably T cell, expressing an antigen receptor, wherein the immune T cell has been modified to have reduced or eliminated the function and/or the expression of one or more of the target proteins encoded by the target genes of Table 4:

**Table 4: List of target genes**

| | |
|---|---|
| ZC3H12C | Zinc finger CCCH-type containing 12C |
| UCP3 | Uncoupling protein 3 |
| TG | Thyroglobulin |
| ITGB8 | Integrin subunit beta-8 |
| LILRB1 | Leukocyte immunoglobulin like receptor B1 |
| AHNAK2 | AHNAK nucleoprotein 2 |
| STON1 | Stonin 1 |
| MYH6 | myosin heavy chain 6 |
| PTK7 | protein tyrosine kinase 7 |
| INSRR | Insulin receptor related receptor |
| ACTG2 | Actin gamma 2, smooth muscle |
| ADAM12 | ADAM metallopeptidase domain 12 |
| FN1 | Fibronectin 1 |
| COL4A1 | Type IV collagen alpha 1 chain |

wherein the reduction or elimination of the function and/or the expression of one or more of the target proteins encoded by the target genes of Table 4 is calculated with respect to the same immune T cell without having said modification.
24. The immune T cell according to item 23, wherein the immune T cell has been modified to have reduced or eliminated the function and/or the expression of the ZC3H12C protein.
25. The immune T cell according to items 23 or 24, wherein the immune T cell has been modified to have reduced or eliminated the function and/or the expression of one or more proteins selected from the group consisting of UCP3, TG, ITGB8 and LILRB1.
26. The immune T cell according to items 24 or 25, wherein the immune T cell has been modified to have reduced or eliminated the function and/or the expression of the ZC3H12C protein, and one or more target proteins selected from the group consisting of UCP3, TG, ITGB8 and LILRB1.
27. The immune T cell according to items 23 to 26, wherein the immune T cell has been modified to have reduced or eliminated the function and/or the expression of the ZC3H12C target protein and one more of the proteins selected from the group consisting of PD-1 and LAG-3.
28. The immune T cell according to items 23 to 27, wherein the immune T cell has been modified to have eliminated the expression of the target protein, preferably wherein the modification is a genetic modification; further preferably wherein the genetic modification is selected from the group consisting of: an insertion, a frameshift mutation, a missense mutation, a deletion, a knock-in, a knock-down, or a knock-out of the gene or part of the gene, and a deletion of the entire target gene encoding for the target protein.
29. The immune T cell according to items 23 to 28, wherein the immune T cell is a CD4⁺ T cell, a CD8⁺ T cell or a CD4⁺/CD8⁺ T cell.
30. The immune T cell according to items 23 to 29, wherein the antigen receptor is a chimeric antigen receptor (CAR) comprising:
- an antigen-binding domain;
- a transmembrane domain, preferably a CD8 or CD28 transmembrane domain;
- an intracellular signaling domain, preferably a CD3ζ endodomain; and
- optionally, a CD28 or a 4-1BB costimulatory domain.
31. The immune T cell according to items 23 to 30, wherein antigen receptor specifically binds to an antigen selected from the group consisting of: HER2, CD19 and mesothelin.
32. An *in vitro* or *ex vivo* method of producing an immune T cell, the method comprising the steps of:
(a) introducing an antigen receptor into an immune T cell and (b) modifying the immune T cell to reduce or eliminate the function and/or the expression of one or more of the target proteins encoded by the target genes of Table 4, wherein the reduction or elimination the function and/or the expression is calculated with respect the same immune T cell without said modification,
wherein steps (a) and (b) may be performed in any order.
33. An immune T cell obtained or obtainable from the method according to item 32.
34. A pharmaceutical composition comprising the immune T cell according to any one of items 23 to 31 or 33, and a pharmaceutically acceptable excipient.
35. An immune T cell according to items 23 to 31 or 33, or the pharmaceutical composition according to item 34, for use in therapy.
36. The immune T cell according to items 23 to 31 or 33, or the pharmaceutical composition according to item 34, for use in the treatment of cancer.
37. The immune T cell for use according to item 36, wherein the antigen receptor specifically binds to an antigen selected from the group consisting of: HER2, CD19 and mesothelin, and wherein the cancer is selected from the group consisting of: an HER2-positive cancer, a CD19-positive cancer and a mesothelin-positive cancer, respectively.

### SEQUENCE LISTING

**SEQ ID NO: 1** The nucleotide sequence of the ZC3H12C target gene is represented by SEQ ID NO: 1. All variants of the ZC3H12C target gene are included in the context of the invention. For instance, three variants have been described for ZC3H12C target gene. The underlined nucleotides correspond to the ZC3H12C protein coding sequence of the ZC3H12C target gene. 
GCTTTCTCGCGGGGCTGGCTATGCCGGGTGGCGGCTCCCAGGAATACGGGGTGCTTT
GCATTCAGGAATACAGAAAAACAGCAAAGTGGAGTCAAGTACACGTAACAACTTCATG
GGCTTGAAGGATCACCTAGGGCATGACCTCGGCCACCTTTATGTGGAGAGCACTGACC
CACAGTTAAGTCCAGCTGTACCTTGGTCAACAGTAGAAAACCCAAGTATGGATACCGTT
AATGTGGGGAAGGATGAAAAAGAGGCGTCTGAAGAGAATGCAAGCTCTGGTGACTCTG
AAGAAAACACAAATTCTGATCATGAGTCAGAACAATTGGGTAGCATTTCAGTAGAGCCA
GGCTTGATAACTAAGACTCACAGACAGCTCTGCAGGTCTCCCTGTTTAGAGCCTCACAT
ACTCAAGCGCAATGAAATTTTGCAAGACTTTAAACCTGAAGAGTCCCAGACTACATCCAA
GGAAGCAAAGAAACCACCTGATGTGGTGCGAGAATACCAAACAAAACTGGAGTTTGCAC
TTAAGTTAGGTTATTCTGAAGAACAGGTTCAGCTTGTACTAAACAAACTTGGTACTGATG
CTTTAATCAATGATATTTTGGGAGAACTTGTCAAACTTGGAAATAAAAGTGAGGCTGATC
AAACGGTTAGTACAATTAACACTATAACACGGGAAACTTCTTCCCTGGAATCTCAGAGGT
CTGAATCTCCAATGCAAGAGATTGTAACAGATGATGGTGAAAATCTGAGACCAATAGTTA
TTGATGGCAGCAATGTGGCAATGAGCCATGGAAACAAAGAAGTATTTTCCTGCAGAGGA
ATAAAATTGGCAGTGGATTGGTTTTTGGAAAGAGGCCACAAAGACATTACAGTTTTTGTT
CCTGCTTGGAGGAAAGAGCAATCCCGACCTGATGCTCTCATTACAGATCAGGAAATTTT
 
ACGTAAATTAGAGAAGGAGAAAATCCTGGTGTTCACGCCATCCCGGCGAGTCCAGGGG
AGGAGAGTGGTGTGCTATGACGACAGGTTCATCGTGAAGCTGGCTTTTGAGTCGGACG
GTATCATTGTGTCCAATGATAACTACAGGGACTTGGCTAATGAGAAGCCAGAATGGAAG
AAGTTCATAGATGAACGATTATTAATGTATTCATTTGTCAATGACAAGTTCATGCCCCCTG
ATGACCCTCTTGGCAGACATGGCCCCAGTCTGGATAATTTTCTGAGGAAGAAACCTATT
GTTCCTGAACACAAAAAGCAGCCTTGTCCATATGGAAAGAAGTGTACCTATGGACACAA
GTGCAAATATTACCATCCCGAAAGGGGCAGTCAGCCACAGCGGTCAGTGGCTGATGAA
CTCCGTGCCATGTCTAGAAATACGGCAGCCAAAACTGCAAACGAAGGAGGACTGGTGA
AAAGCAACAGTGTTCCTTGTAGCACCAAGGCTGATAGCACTTCTGATGTCAAACGAGGT
GCTCCAAAGAGGCAATCAGATCCAAGCATAAGGACACAAGTCTACCAAGACCTAGAAGA
AAAGCTTCCCACCAAAAACAAATTGGAAACCAGGTCTGTACCTTCCTTAGTTAGCATCCC
AGCTACTTCTACTGCAAAACCCCAAAGCACTACATCTTTAAGCAATGGCCTTCCATCTGG
AGTTCATTTCCCACCTCAGGATCAAAGACCACAGGGACAATATCCTTCAATGATGATGG
CAACCAAAAATCATGGAACGCCAATGCCTTATGAACAGTATCCAAAATGTGACTCACCTG
TCGACATCGGATATTATTCCATGTTGAATGCATACTCAAATCTGAGTCTCTCAGGCCCAC
GAAGCCCTGAAAGGCGTTTCTCCTTAGACACAGATTATAGAATAAGTTCCGTAGCTTCTG
ACTGCAGCAGTGAAGGGAGCATGAGCTGTGGGAGCAGTGACTCCTACGTGGGTTACAA
TGACCGGTCCTATGTCAGCTCCCCCGACCCACAGCTAGAGGAGAATTTGAAGTGTCAAC
ACATGCACCCTCACAGCCGCCTTAATCCTCAACCGTTCCTGCAGAATTTCCACGACCCC
TTAACCAGAGGGCAAAGTTACAGTCACGAAGAACCAAAGTTCCATCACAAGCCTCCTCT
TCCGCACCTGGCTCTGCACCTGCCGCACTCCGCTGTGGGCGCCCGGTCCAGCTGTCCT
GGCGACTACCCCTCTCCTCCAAGTTCAGCACACTCTAAGGCACCACACCTAGGGAGGT
CCTTGGTGGCCACGAGAATAGACAGCATCTCTGACTCTCGACTTTATGACAGTTCTCCT
TCACGACAAAGAAAGCCTTATTCCCGCCAGGAAGGCCTGGGAAGCTGGGAGAGGCCAG
GCTATGGGATCGACGCCTATGGGTACCGGCAGACTTATTCCTTGCCCGATAACTCCACA
CAGCCGTGTTATGAGCAGTTCACCTTCCAGAGCCTCCCTGAGCAACAGGAGCCAGCCT
GGCGGATCCCATACTGTGGAATGCCGCAAGATCCCCCGAGGTATCAAGACAACCGAGA
AAAGATTTATATCAATTTGTGCAACATCTTCCCCCCTGACCTTGTGAGAATTGTCATGAA
AAGGAATCCTCACATGACAGACGCCCAGCAGCTCGCCGCAGCCATTTTAGTGGAGAAA
TCCCAGCTGGGTTATTGAAAGATGATGCATCTTTGTGGTGTTTAGTAGTTTTTTGTTCAG
CTCAAATGCTGAGGGAGGTTTGCTACAATAGCACATGTGATCTCCTTCTCAGCAAGGAG
GTTATATAGTATCCATTTATGTGAAATACTGTATCATGGAATCTGTATGTATAGCCCCACA
TGGTGGAAGTATCACGGGATTGCTTTACATTTAAACTTTTTTTTTTTAACATTTCCTTTTTA
AAGCTATATCCTTGGCTGGAAATTTTTCCAGTTTGATTTAATAGATGTATCTGTGATCTTT
GATATTAATCTTTGGTGCATCAGGGGTTTATATGCAGCACTTTTTATCCTTGTTTTGTGTT
TTATTAACTTGGTGTTTGTCTATCAATTGCAAGCAATTACAATACCTTCAGAATGTGGGAC
ATTTGACTAGACCTAGCAAACTGTTTTTTCGAGCCAAGCTTAGTTAGACTCTTTTACAGC
 
TTTTTAAGTTATTTTTATTTGGGGAAAGTGGCTTCTTTGTGCTATAATCATTATTTATAGA
AACAAAGTTATACTACAGCACTGACTTTATATTTAAACAGAATGTAAGTTACCAGTTTTA
TGTTGAAATGTGTTACAGTATATATATATTAGAATGATTTACAATATGGCACTTTTCGATG
TGTTATTTTTGTTTGGATTTTTTTTCTGTTAAGAAATTAGTTAATTTAATATGGTCAATTTA
AAAGAAAGCAGATGCAATCAATGGAAAAATGTTTCCATTTTTTAAAAATGAATAAGGCAA
AAGCTGTAACTGTTACAGGTTAGAGCTTTGTTATCCAGCTATGATGTGCTTCTTGACAGT
AGAAGTGGAATTGAATTCCTAGATTTCCATTAACCTGTATTTTTAATATGTCTGTCTTTTT
GTTTTGGGGCACAACAATACTGGATAAAATAACCCTTTCACAGCACTTGCCTGTTTTTAA
TGAATCTAATTATTCACAATGCAACTTTTATATTTAACATACTCTTTAGCTTTCCTGCTATT
TATCAAGGCTGGCCTGAGGTGGGTTTATGTGTTGAGGTTATGCAACATTTCTTGATACTG
CACTATAGAGAAATGGTGATGGAGGAGTTGTAAATGGTAACTTAAAATTTTTGTAAGATA
TTGTATATTTTCCATTTTCCTGAAGGTAGTTTTCTTGGGGGGGCCTGTTATATTATTAAGG
CCAGACTCTTGCCACAAATAGTGTAGTTTTAGATACAGACTAAGGTCTGTTCTAGTATTA
GTAAGGGATATTTCTGGTTTCAAAGTCATGGGTTTTGCTAGTGGTGAATACATTTCTGCG
GAGTAGAAGATAGATTTTGCAGTCAGTGGCAGACAGTTGTGTGATTGACATCACTTGAC
TGTTGCGTCCAGGTTTTGAATTGTACTTCACGTAACAGATGCATTCAGATCTTTTTCTGTA
GTCTGCTTAGATGCCCTGGCTATTCTGATTATCCTACATGCTACAGTTTGAAGTGAAGCC
CTGAAAAACCAGAAAGTACCTTTTACTGTTGATACAAATTGTATCTTTTTAACTATAAGAA
CTATTTTGATTTGTAGATCTAGTTAAAACACAAGTATGTAACTATGATTAGACTTTTGGGC
AACATTTTATCCCTTATTTAAATACAAATTTTTAAAGTAAAATTGAGGTCTAGAATAGATTA
GAAAATAAAAATAACAATTTAGATAAATAGAAATGTCTGTCTTAGTTTATATAATATATTA
AAACACAGTAAATAAATTTATTGGCATTTTCTTTCTCCTAAAACTTACCTAGTGTGAACTT
AAAATAAAGGTAAAATGCTGCCTGAAAATAATGTCCAAGCACCTTTGACTAGGATAACAT
TTTCACTACTTGTGTGACACTGTGTGTTGCACGAAGTAGGATTTGGGTATACAGTAAATG
CTTCTAAAAGGCATTGTGCATATTGACATAACCAATAATCTGAACCGTGTTCAGCAAACT
TAATTCAGGAAAGTGGTATTCTACACAATTATTGCTGTTGTGTTTGAAATGAGTGTGGCA
CTCATCTGTATCCAGAAATAATATGGGTGAGGCCACACAACCCATTCTGAGTGGTGTCT
GTCTGAAAGCAACCCTACTCGCATGTGAAATTGTTCTCCTTGATTTGGTCACTATAAAGC
AAGTTTAAATGTAGAGGCTAACTCAGTGCCAAAAACAGGGTTACAAATGTGTAGTATCTT
TTATTTTAGTCATATTCTAAGACTTCTTTTTAGTATGAAATCACTTTTAAATTATACATGTA
GGTTTTGCTTCCATTTTCTTCATTTTACCATTTTAATTATTTGAACATTCGCCAAATTTTAC
ATTTATTTAAATGAGATCTTAGGTGAGATGTGTGTGACACTTTGAATTTGACCTTCTTGTG
TTATTAGCTGACTATTTGTCATTGCCTCATGGATTTTAAATTATGGGAAAATAGTGTAGCC
AGCTGCCACCTCTACTGAAGTGAGTAGCTCTGAACTACCCACACTAAATCCTTCAGTGT
AATTAATTATGAGTTTAAAAATAGCAGTTTTCTTATGTGAAGAGGACAGTTTGTCCCCTTT
TTTTGAAGCACCGATGTTGCGTTCAGAGCTGTAGAATGAGATAATTGGCAGACTTTAGG
TACAGCAAATTCTTACTTATCTAAAGCAATAAGTCAAAGGAGTCCATCATTAAATTAAATC
 
CATAGCTGATCACAAGCCTTCATTTTAGCCAAAACTTTCTCTCTAACCAAATCTTTTACCA
GACTTGCTAATAAATAACCAGAGATGTGTTAATAAGTGAAGTTGCCAGAAATGGTCAA
CTGATCGGAAAAAAAGGATTCAGACTGGAGTATTTTGCCCCTGAATAATTGACAGTTGA
CTGTGCTTTACACAGTAACTAGCCAGTCTGTTGTCTCTGTGTCTTAGTCCAGAGGGAATA
GTACCCAATTGGCCAAATACTGGCCCTTAGTATCTCCTCCTTTCTTGCATGGGATACAGA
CGTTTTCAGTCTTGTTTTTATGATCTCTCTCTATATCCTGATAAGAGTTTGTCATTGACTT
ACACATTTGGAAGAAATGCACACCAGTGTAATATATTTGATACTGGTGGAAACTTGAACT
TTGTGTTTTTATGAAAATTCATTTATGAGAATATGTAATATAACTGAAGAGTATTTTATGTA
TATCTATATACACAAATATGTATTTGTTATGAGGTATTAAAAACAGGGGTTGGGGGGAGT
GCTTCTGATGGCTAACTTTTCTCTAATTAAACTATGTTTCTTTGAGTTGTGAACGACCGA
GTCTGGGGTCTGGACGGCCAATGATAAGATTTAGAACCACTTGGATGGAAAGCAGTTCT
TCACTGGTTTTATTCTTGGTATTTTCAAAGAATTATTTTGATATTTTTAATAGAATGTGTAA
TTTTAAAATACACAAAAAACTTAAAGTAGTATTGATTATACAAATAATTATTTAACATGTCT
TATGGATGTATCTATATGTATATAGACAGTAATATATTTATAAAACAAATATACTTTGCTTA
TGTTATAGCTCTTAGTTTGTGACAGGTGGGAGGATGGCTCTGGGTGTGTGTGTGTGTGT
GTGTGTGTGTGTGTGTGTGTGTGTGTTTTGAAAACTCTCAAGCTGTTTTCCCTCTGATTT
ACAATGGTATTTACTTTAAAGTATGTTTGGTTTTCATTATTCTTTTTGCTCTCCAACTTCCT
TATTCAAGATAAAATAAGACATACAGTTTTCTGGCCATTCTGTTGGTGTGTGGTGCACCC
ATTTTATTGTCATCTAACTCCTGGAGAATTCCCACGTGACCTGAAATCAAACAGATTCTG
GCTGGACATATGCTTATGTTCCAAATATATTAAAGATGTTAATTCAGCTAACAGTTAAGTT
TCCAAGGTATACACCAACAAATAAAATGAGGTATTAAAAAGAGATGGATTACACACATGC
ATTAGAATAAGAGAAAACAGTAGTGTTAATAAAAGTAAAGAAACATACACTATCTTAGCC
CCCTAGGGCAGGGGTTGACTTTTTCTGTAAAGGATCAGATAGTAAATAATACAGGAATC
ATATGGACTTCAAATGCCATTGTAAAAACTACTCAGCTCTGCCTTTAGAGCATAAGAACA
GCCACAGATAACATGTAAATTAGTGTGGCTGTGTTCCAATAAAACTTTATTTACAGAAAC
GGGAAGCCGGTCAGATTGGGCCTGTGGGCCATAATTTGCTGATCTCAAGCAGTAAATC
CTGGTATATGTTAATAGAAGAAATCATAATTGCATTTCAGTTGACATTAAAAGAAAATCTG
GTAGTTTTTGATGTCCTTCAAAAGAGGATTGTTAGACATTGATGTTAAAGCATCTTAATTC
ATTTGAGTTTTCTTACCTGTTTACACCCATTATTTATTAGAGATATTTCTTGTGTTTAACCA
CAAAAAAAAGCACTCTGTTAAAATGTTTTAATATGTATTGAATTTCTTTCATAAACTTTTCA
TTTCTGTTGATAAATGGGAATCCCTTACCAACCTTTTGTTTTTTAAAAGTCTCATAGACCA
AAAAAAATCTGTTGCAGCATTTAATTAGCCACAGATACATTTTGGCTGCATTTACCAGTTA
CATTTTTCCATTGGTTTTGGCTTCTAATAAATAACATATCTGCCATTCTTTAAAAATGATTT
TAAAGAAGATAAATATATTGTAATTTCACATGCTATAGCTTTATTCTGTAAGATTAAAAATT
GTGACTAGTATAATTGTAGCTATAATGTGAGTGGCATGTTACAATGTAACTCTTTATGAG
AAATAAAATGTATCTCTGCTTTGTCTGTCCAGATCTTTAGGATTTTTAGATGCCTTGGGAC
TGTCCTTGGTGAATGATATTCTTCATATGATCATATGTAATTTTGAATTCGTTGGAAGTAC
 
ACGCTGCTGAGCATGTTTATTCACAGTGCTTTATACCGGGTGTTGCATCAGTAATCATTT
TCATAAGAACATTTAAACAGCATGAACATCATCATCCACTTAAATAGTTAAACTTTCTTTT
AAAATTGGAATGCAACTGTAGGTTTTAACAATGTTTATTGTTTTTTAAGTGGTTACTTTGT
TTTTCCTTAATACTTTCTGTTAACTTAATTATTACTCCTGTTGCAGTGTTACTGTTATGTAT
TAGAAGTGGCTTTTCCCCCTAAGATCCTTAGTCTTTTAAAGACAATTTAAGGTATTGGCC
ATTTGGCAGTAGAAAATGTGCATGTTTTAACTTGGTTTTATAAAATCTGTAATGTTTCACT
TCTTGAACCATGTACCAAATTTGCCAATTTTCTGTCCAAGTGTTTCAGATGAATAACAAAA
CGCTGTTCATTGAAGCTTTCGCCACCTTTCTTAAAGCAGCGTATGTTCCAAGGAAAAA
GGCATTGAAAAGCAATCGTTTGTTTTTATGAAGAATAGGTGTTCAGATTCCTTCAGTTTTT
TTGAAATTAGAAATTTCTTACCTTATGTGAAATATTCACAAACGTGCACACTTCTGCAGAG
ACAAAGCATTTCACTGCACGTGTACCAGGTTATTGATTTTATCTTTTCCTTTCAGGGTTTT
GTCCTCCCAAACCAGAGTCATATGCTGCTAGTAGAATTTTTTATTTGATCCTGCGAACTT
TTCTTATAGGAAAAGTAAGGCAAAGGATGTGTAGTGCAACCATCTGATAAACTAGTGTGA
TTGTATTTATCCTCTGTTCTGTGTATTTCTGTAATGGAATCTTTACAATTCCCAAAACGGT
ATTTTAGACCTACTGGAAATCTGTATCGAAACAGCTATGTGATTCTGCCACTGAGAAAAA
AAAAATTTTTAATTCGTTTTTCTTATGCTGGTTTGTTTTTCTTTAATGAAGAAATTGATCTC
ATATGGCATCATAGATGCTAAATAAATAAAAGCATCATACTTCTCTAGTTTGCCTGCATTC
AGTGGCTAACATTATGAGCATTGTGTAAGATAAACACATGGTCAGTATCAATGTAAATGT
TAGAGCCATGATTAATTCCTATGAAAATTGAAATTAAATGTCAAAGACAACTAGACATAA
**SEQ ID NO: 2** The amino acid sequence of the ZC3H12C target protein is represented by SEQ ID NO: 2. In the context of the invention, all isoforms of the ZC3H12C target protein are included. For instance, three isoforms have been described for the ZC3H12C target protein. 
MPGGGSQEYGVLCIQEYRKNSKVESSTRNNFMGLKDHLGHDLGHLYVESTDPQLSPAVPW
STVENPSMDTVNVGKDEKEASEENASSGDSEENTNSDHESEQLGSISVEPGLITKTHRQLC
RSPCLEPHILKRNEILQDFKPEESQTTSKEAKKPPDVVREYQTKLEFALKLGYSEEQVQLVL
NKLGTDALINDILGELVKLGNKSEADQTVSTINTITRETSSLESQRSESPMQEIVTDDGENLR
PIVIDGSNVAMSHGNKEVFSCRGIKLAVDWFLERGHKDITVFVPAWRKEQSRPDALITDQEIL
RKLEKEKILVFTPSRRVQGRRVVCYDDRFIVKLAFESDGIIVSNDNYRDLANEKPEWKKFIDE
RLLMYSFVNDKFMPPDDPLGRHGPSLDNFLRKKPIVPEHKKQPCPYGKKCTYGHKCKYYH
PERGSQPQRSVADELRAMSRNTAAKTANEGGLVKSNSVPCSTKADSTSDVKRGAPKRQS
DPSIRTQVYQDLEEKLPTKNKLETRSVPSLVSIPATSTAKPQSTTSLSNGLPSGVHFPPQDQ
RPQGQYPSMMMATKNHGTPMPYEQYPKCDSPVDIGYYSMLNAYSNLSLSGPRSPERRFS
LDTDYRISSVASDCSSEGSMSCGSSDSYVGYNDRSYVSSPDPQLEENLKCQHMHPHSRLN
PQPFLQNFHDPLTRGQSYSHEEPKFHHKPPLPHLALHLPHSAVGARSSCPGDYPSPPSSA
HSKAPHLGRSLVATRIDSISDSRLYDSSPSRQRKPYSRQEGLGSWERPGYGIDAYGYRQTY
 
SLPDNSTQPCYEQFTFQSLPEQQEPAWRIPYCGMPQDPPRYQDNREKIYINLCNIFPPDLV
RIVMKRNPHMTDAQQLAAAILVEKSQLGY
**SEQ ID NO: 3:** The nucleotide sequence of the UCP3 target gene is represented by SEQ ID NO: 3. All the variants of UCP3 target gene are included in the context of the invention. The underlined nucleotides correspond to the UCP3 target protein coding sequence of UCP3 target gene. 
AGAGACAACAGTGAATGACAAGGAGGGGCCATCCAATCCCTGCTGCCACCTCCTGGGA
TGGAGCCCTAGGGAGCCCCTGTGCTGCCCCTGCCGTGGCAGGACTCACAGCCCCACC
GCTGCACTGAAGCCCAGGGCTGTGGAGCAGCCTCTCTCCTTGGACCTCCTCTCGGCCC
TAAAGGGACTGGGCAGAGCCTTCCAGGACTATGGTTGGACTGAAGCCTTCAGACGTGC
CTCCCACCATGGCTGTGAAGTTCCTGGGGGCAGGCACAGCAGCCTGTTTTGCTGACCT
CGTTACCTTTCCACTGGACACAGCCAAGGTCCGCCTGCAGATCCAGGGGGAGAACCAG
GCGGTCCAGACGGCCCGGCTCGTGCAGTACCGTGGCGTGCTGGGCACCATCCTGACC
ATGGTGCGGACTGAGGGTCCCTGCAGCCCCTACAATGGGCTGGTGGCCGGCCTGCAG
CGCCAGATGAGCTTCGCCTCCATCCGCATCGGCCTCTATGACTCCGTCAAGCAGGTGT
ACACCCCCAAAGGCGCGGACAACTCCAGCCTCACTACCCGGATTTTGGCCGGCTGCAC
CACAGGAGCCATGGCGGTGACCTGTGCCCAGCCCACAGATGTGGTGAAGGTCCGATTT
CAGGCCAGCATACACCTCGGGCCATCCAGGAGCGACAGAAAATACAGCGGGACTATGG
ACGCCTACAGAACCATCGCCAGGGAGGAAGGAGTCAGGGGCCTGTGGAAAGGAACTTT
GCCCAACATCATGAGGAATGCTATCGTCAACTGTGCTGAGGTGGTGACCTACGACATCC
TCAAGGAGAAGCTGCTGGACTACCACCTGCTCACTGACAACTTCCCCTGCCACTTTGTC
TCTGCCTTTGGAGCCGGCTTCTGTGCCACAGTGGTGGCCTCCCCGGTGGACGTGGTGA
AGACCCGGTATATGAACTCACCTCCAGGCCAGTACTTCAGCCCCCTCGACTGTATGATA
AAGATGGTGGCCCAGGAGGGCCCCACAGCCTTCTACAAGGGATTTACACCCTCCTTTTT
GCGTTTGGGATCCTGGAACGTGGTGATGTTCGTAACCTATGAGCAGCTGAAACGGGCC
CTGATGAAAGTCCAGATGTTACGGGAATCACCGTTTTGAACAAGACAAGAAGGCCACTG
GTAGCTAACGTGTCCGAAACCAGTTAAGAATGGAAGAAAACGGTGCATCCACGCACACA
TGGACACAGACCCACACATGTTTACAGAACTGTTGTTTACTTGTTGCTGATTCAAGAAAC
AGAAGTAGAAGAGGAGGAGGGATTCTGGTCTTCACTGCCATGCCTCAAGAACACCTTTG
TTTTGCACTGACAAGATGGAAAATAAATTATATTAATTTTTGAAACCCATTAGGCATGCCT
AATATTTAGGCAAGAGAAAATAAACCAAGATAGATCCATTTGGACAAAATGGAAGGTTGG
AGACGTGTATCCCCGTGAAATCTGGTTAGATAATGAATGATAAGCAGGAAGGATGGCAA
GCACGGGACAGGAGGGGCCCACAATGGAGTGGGAGATCAGCCACGGAGCCTGGAGG
GATGCCACCCAGCAACACAGAGCTGGCGACTGCAGCTGCACCATCACACATGCATCAT
CAGCCTATTTGTAATATGTCTGCCACAGAGAGTCCTTTGGGATTCTAGGAAACCCAAGG
AACAAGAGAAAAAACTAGAGCCTGTGCTAAAGAAGCCCTGCTGGGCCCATGTGAGGCT
 
GGGGCTCGTAAATATTCCCTGACGACACTGAAGAATCAAGAGGGCAGCCCCCACTTTC
CTACAAAATGGAGGGAGCCATCCCTTCCCTGTCCACCTCACCAGGGGTGCTATGACAT
GCAAGTGAGAAGCTGGGCATGAACGCACTTTATAAAAGCAAAAGCTCTGTGTAAATCTA
ACTACAAGGACAATGCCTTGGGAGAGATTTTGTCGGGACAGAGAGGAGTTGCCAGGGA
AGAAGGTTTGAAAGATACGGTTGTCTAGAGGTGAGACCAAAGGATCCAGAGACTTGGG
GACCAGAGGTGACAGTGGATGACGTGAAGCCACAGGAGCCCCACCCCCATGCAGCCT
CTTCCCCACCCCCCCCACCACGTGCTCAATCATGAGTACCTCAAAGGATTGTTGGGCTT
GGGGGAAAAGAGGTGGATTCCTGGGCAAGAACCTAAAGTAGCAGGACTCGGAATTCTC
GGGAAATTATTATGACTCAATAAAAGAATTCACACCTTAGGTGTGGGAGTAAGAACAGC
CA
**SEQ ID NO: 4:** The amino acid sequence of the UCP3 target protein is represented by SEQ ID NO: 4. All the variants of UCP3 target protein are included in the context of the invention. 
MVGLKPSDVPPTMAVKFLGAGTAACFADLVTFPLDTAKVRLQIQGENQAVQTARLVQYRGV
LGTILTMVRTEGPCSPYNGLVAGLQRQMSFASIRIGLYDSVKQVYTPKGADNSSLTTRILAG
CTTGAMAVTCAQPTDVVKVRFQASIHLGPSRSDRKYSGTMDAYRTIAREEGVRGLWKGTL
PNIMRNAIVNCAEVVTYDILKEKLLDYHLLTDNFPCHFVSAFGAGFCATVVASPVDVVKTRY
MNSPPGQYFSPLDCMIKMVAQEGPTAFYKGFTPSFLRLGSWNVVMFVTYEQLKRALMKVQ
MLRESPF
**SEQ ID NO: 5:** The nucleotide sequence of the TG target gene is represented by SEQ ID NO: 5. All the variants of TG target gene are included in the context of the invention. The underlined nucleotides correspond to the TG target protein coding sequence of the TG target gene. 
AAGCAGTGGTTTCTCCTCCTTCCTCCCAGGAAGGGCCAGGAAAATGGCCCTGGTCCTG
GAGATCTTCACCCTGCTGGCCTCCATCTGCTGGGTGTCGGCCAATATCTTCGAGTACCA
GGTGGATGCCCAGCCCCTTCGTCCCTGTGAGCTGCAGAGGGAAACGGCCTTTCTGAAG
CAAGCAGACTACGTGCCCCAGTGTGCAGAGGATGGCAGCTTCCAGACTGTCCAGTGCC
AGAACGACGGCCGCTCCTGCTGGTGTGTGGGTGCCAACGGCAGTGAAGTGCTGGGCA
GCAGGCAGCCAGGACGGCCTGTGGCTTGTCTGTCATTTGTCAGCTACAGAAACAGCA
GATCTTACTGAGTGGCTACATTAACAGCACAGACACCTCCTACCTCCCTCAGTGTCAGG
ATTCAGGGGACTACGCGCCTGTTCAGTGTGATGTGCAGCAGGTCCAGTGCTGGTGTGT
GGACGCAGAGGGGATGGAGGTGTATGGGACCCGCCAGCTGGGGAGGCCAAAGCGAT
GTCCAAGGAGCTGTGAAATAAGAAATCGTCGTCTTCTCCACGGGGTGGGAGATAAGTCA
CCACCCCAGTGTTCTGCGGAGGGAGAGTTTATGCCTGTCCAGTGCAAATTTGTCAACAC
CACAGACATGATGATTTTTGATCTGGTCCACAGCTACAACAGGTTTCCAGATGCATTTGT
GACCTTCAGTTCCTTCCAGAGGAGGTTCCCTGAGGTATCTGGGTATTGCCACTGTGCTG
ACAGCCAAGGGCGGGAACTGGCTGAGACAGGTTTGGAGTTGTTACTGGATGAAATTTAT
 
GACACCATTTTTGCTGGCCTGGACCTTCCTTCCACCTTCACTGAAACCACCCTGTACCG
GATACTGCAGAGACGGTTCCTCGCAGTTCAATCAGTCATCTCTGGCAGATTCCGATGCC
CCACAAAATGTGAAGTGGAGCGGTTTACAGCAACCAGCTTTGGTCACCCCTATGTTCCA
AGCTGCCGCCGAAATGGCGACTATCAGGCGGTGCAGTGCCAGACGGAAGGGCCCTGC
TGGTGTGTGGACGCCCAGGGGAAGGAAATGCATGGAACCCGGCAGCAAGGGGAGCCG
CCATCTTGTGCTGAAGGCCAATCTTGTGCCTCCGAAAGGCAGCAGGCCTTGTCCAGACT
CTACTTTGGGACCTCAGGCTACTTCAGCCAGCACGACCTGTTCTCTTCCCCAGAGAAAA
GATGGGCCTCTCCAAGAGTAGCCAGATTTGCCACATCCTGCCCACCCACGATCAAGGA
GCTCTTTGTGGACTCTGGGCTTCTCCGCCCAATGGTGGAGGGACAGAGCCAACAGTTT
TCTGTCTCAGAAAATCTTCTCAAAGAAGCCATCCGAGCAATTTTTCCCTCCCGAGGGCT
GGCTCGTCTTGCCCTTCAGTTTACCACCAACCCAAAGAGACTCCAGCAAAACCTTTTTG
GAGGGAAATTTTTGGTGAATGTTGGCCAGTTTAACTTGTCTGGAGCCCTTGGCACAAGA
GGCACATTTAACTTCAGTCAATTTTTCCAGCAACTTGGTCTTGCAAGCTTCTTGAATGGA
GGGAGACAAGAAGATTTGGCCAAGCCACTCTCTGTGGGATTAGATTCAAATTCTTCCAC
AGGAACCCCTGAAGCTGCTAAGAAGGATGGTACTATGAATAAGCCAACTGTGGGCAGC
TTTGGCTTTGAAATTAACCTACAAGAGAACCAAAATGCCCTCAAATTCCTTGCTTCTCTC
CTGGAGCTTCCAGAATTCCTTCTCTTCTTGCAACATGCTATCTCTGTGCCAGAAGATGTG
GCAAGAGATTTAGGTGATGTGATGGAAACGGTACTCAGCTCCCAGACCTGTGAGCAGA
CACCTGAAAGGCTATTTGTCCCATCATGCACGACAGAAGGAAGCTATGAGGATGTCCAA
TGCTTTTCCGGAGAGTGCTGGTGTGTGAATTCCTGGGGCAAAGAGCTTCCAGGCTCAA
GAGTCAGAGGTGGACAGCCAAGGTGCCCCACAGACTGTGAAAAGCAAAGGGCTCGCAT
GCAAAGCCTCATGGGCAGCCAGCCTGCTGGCTCCACCTTGTTTGTCCCTGCTTGTACTA
GTGAGGGACATTTCCTGCCTGTCCAGTGCTTCAACTCAGAGTGCTACTGTGTTGATGCT
GAGGGTCAGGCCATTCCTGGAACTCGAAGTGCAATAGGGAAGCCCAAGAAATGCCCCA
CGCCCTGTCAATTACAGTCTGAGCAAGCTTTCCTCAGGACGGTGCAGGCCCTGCTCTCT
AACTCCAGCATGCTACCCACCCTTTCCGACACCTACATCCCACAGTGCAGCACCGATGG
GCAGTGGAGACAAGTGCAATGCAATGGGCCTCCTGAGCAGGTCTTCGAGTTGTACCAA
CGATGGGAGGCTCAGAACAAGGGCCAGGATCTGACGCCTGCCAAGCTGCTAGTGAAGA
TCATGAGCTACAGAGAAGCAGCTTCCGGAAACTTCAGTCTCTTTATTCAAAGTCTGTATG
AGGCTGGCCAGCAAGATGTCTTCCCGGTGCTGTCACAATACCCTTCTCTGCAAGATGTC
CCACTAGCAGCACTGGAAGGGAAACGGCCCCAGCCCAGGGAGAATATCCTCCTGGAG
CCCTACCTCTTCTGGCAGATCTTAAATGGCCAACTCAGCCAATACCCGGGGTCCTACTC
AGACTTCAGCACTCCTTTGGCACATTTTGATCTTCGGAACTGCTGGTGTGTGGATGAGG
CTGGCCAAGAACTGGAAGGAATGCGGTCTGAGCCAAGCAAGCTCCCAACATGTCCTGG
CTCCTGTGAGGAAGCAAAGCTCCGTGTACTGCAGTTCATTAGGGAAACGGAAGAGATTG
TTTCAGCTTCCAACAGTTCTCGGTTCCCTCTGGGGGAGAGTTTCCTGGTGGCCAAGGGA
ATCCGGCTGAGGAATGAGGACCTCGGCCTTCCTCCGCTCTTCCCGCCCCGGGAGGCTT
 
TCGCGGAGCAGTTTCTGCGTGGAGTGATTACGCCATTCGCCTGGCGGCTCAGTCTAC
CTTAAGCTTCTATCAGAGACGCCGCTTTTCCCCGGACGACTCGGCTGGAGCATCCGCC
CTTCTGCGGTCGGGCCCCTACATGCCACAGTGTGATGCGTTTGGAAGTTGGGAGCCTG
TGCAGTGCCACGCTGGGACTGGGCACTGCTGGTGTGTAGATGAGAAAGGAGGGTTCAT
CCCTGGCTCACTGACTGCCCGCTCTCTGCAGATTCCACAGTGCCCGACAACCTGCGAG
AAATCTCGAACCAGTGGGCTGCTTTCCAGTTGGAAACAGGCTAGATCCCAAGAAAACCC
ATCTCCAAAAGACCTGTTCGTCCCAGCCTGCCTAGAAACAGGAGAGTATGCCAGGCTG
CAGGCATCGGGGGCTGGCACCTGGTGTGTGGACCCTGCATCAGGAGAAGAGTTGCGG
CCTGGCTCGAGCAGCAGTGCCCAGTGCCCAAGCCTCTGCAATGTGCTCAAGAGTGGAG
TCCTCTCCAGGAGAGTCAGCCCAGGCTATGTCCCAGCCTGCAGGGCAGAGGATGGGG
GCTTTTCCCCAGTGCAATGTGACCAGGCCCAGGGCAGCTGCTGGTGTGTCATGGACAG
CGGAGAAGAGGTGCCTGGGACGCGCGTGACCGGGGGCCAGCCCGCCTGTGAGAGCC
CGCGGTGTCCGCTGCCATTCAACGCGTCGGAGGTGGTTGGTGGAACAATCCTGTGTGA
GACAATCTCGGGCCCCACAGGCTCTGCCATGCAGCAGTGCCAATTGCTGTGCCGCCAG
GGCTCCTGGAGCGTGTTTCCACCAGGGCCATTGATATGTAGCCTGGAGAGCGGACGCT
GGGAGTCACAGCTGCCTCAGCCCCGGGCCTGCCAACGGCCCCAGCTGTGGCAGACCA
TCCAGACCCAAGGGCACTTTCAGCTCCAGCTCCCGCCGGGCAAGATGTGCAGTGCTGA
CTACGCGGATTTGCTGCAGACTTTCCAGGTTTTCATATTGGATGAGCTGACAGCCCGCG
GCTTCTGCCAGATCCAGGTGAAGACTTTTGGCACCCTGGTTTCCATTCCTGTCTGCAAC
AACTCCTCTGTGCAGGTGGGTTGTCTGACCAGGGAGCGTTTAGGAGTGAATGTTACATG
GAAATCACGGCTTGAGGACATCCCAGTGGCTTCTCTTCCTGACTTACATGACATTGAGA
GAGCCTTGGTGGGCAAGGATCTCCTTGGGCGCTTCACAGATCTGATCCAGAGTGGCTC
ATTCCAGCTTCATCTGGACTCCAAGACGTTCCCAGCGGAAACCATCCGCTTCCTCCAAG
GGGACCACTTTGGCACCTCTCCCAGGACATGGTTTGGGTGCTCGGAAGGATTCTACCA
AGTCTTGACAAGTGAGGCCAGTCAGGACGGACTGGGATGCGTTAAGTGTCCTGAAGGA
AGCTATTCCCAAGATGAGGAATGCATTCCTTGTCCTGTTGGATTCTACCAAGAACAGGC
AGGGAGCTTGGCCTGTGTCCCATGTCCTGTGGGCAGAACGACCATTTCTGCTGGAGCT
TTCAGCCAGACTCACTGTGTCACTGACTGTCAGAGGAACGAAGCAGGCCTGCAATGTG
ACCAGAATGGCCAGTATCGAGCCAGCCAGAAGGACAGGGGCAGTGGAAGGCCTTCT
GTGTGGACGGCGAGGGGCGGAGGCTGCCATGGTGGGAAACAGAGGCCCCTCTTGAGG
ACTCACAGTGTTTGATGATGCAGAAGTTTGAGAAGGTTCCAGAATCAAAGGTGATCTTC
GACGCCAATGCTCCTGTGGCTGTCAGATCCAAAGTTCCTGATTCTGAGTTCCCCGTGAT
GCAGTGCTTGACAGATTGCACAGAGGACGAGGCCTGCAGCTTCTTCACCGTGTCCACG
ACGGAGCCAGAGATTTCCTGTGATTTCTATGCTTGGACAAGTGACAATGTTGCCTGCAT
GACTTCTGACCAGAAACGAGATGCACTGGGGAACTCAAAGGCCACCAGCTTTGGAAGT
CTTCGCTGCCAGGTGAAAGTGAGGAGCCATGGTCAAGATTCTCCAGCTGTGTATTTGAA
AAAGGGCCAAGGATCCACCACAACACTTCAGAAACGCTTTGAACCCACTGGTTTCCAAA
 
ACATGCTTTCTGGATTGTACAACCCCATTGTGTTCTCAGCCTCAGGAGCCAATCTAACC
GATGCTCACCTCTTCTGTCTTCTTGCATGCGACCGTGATCTGTGTTGCGATGGCTTCGT
CCTCACACAGGTTCAAGGAGGTGCCATCATCTGTGGGTTGCTGAGCTCACCCAGTGTC
CTGCTTTGTAATGTCAAAGACTGGATGGATCCCTCTGAAGCCTGGGCTAATGCTACATG
TCCTGGTGTGACATATGACCAGGAGAGCCACCAGGTGATATTGCGTCTTGGAGACCAG
GAGTTCATCAAGAGTCTGACACCCTTAGAAGGAACTCAAGACACCTTTACCAATTTTCAG
CAGGTTTATCTCTGGAAAGATTCTGACATGGGGTCTCGGCCTGAGTCTATGGGATGTAG
AAAAGACACAGTGCCAAGGCCAGCATCTCCAACAGAAGCAGGTTTGACAACAGAACTTT
TCTCCCCTGTGGACCTCAACCAGGTCATTGTCAATGGAAATCAATCACTATCCAGCCAG
AAGCACTGGCTTTTCAAGCACCTGTTTTCAGCCCAGCAGGCAAACCTATGGTGCCTTTC
TCGTTGTGTGCAGGAGCACTCTTTCTGTCAGCTCGCAGAGATAACAGAGAGTGCATCCT
TGTACTTCACCTGCACCCTCTACCCAGAGGCACAGGTGTGTGATGACATCATGGAGTCC
AATGCCCAGGGCTGCAGACTGATCCTGCCTCAGATGCCAAAGGCCCTGTTCCGGAAGA
AAGTTATACTGGAAGATAAAGTGAAGAACTTTTACACTCGCCTGCCGTTCCAAAAACTGA
TGGGGATATCCATTAGAAATAAAGTGCCCATGTCTGAAAAATCTATTTCTAATGGGTTCT
TTGAATGTGAACGACGGTGCGATGCGGACCCATGCTGCACTGGCTTTGGATTTCTAAAT
GTTTCCCAGTTAAAAGGAGGAGAGGTGACATGTCTCACTCTGAACAGCTTGGGAATTCA
GATGTGCAGTGAGGAGAATGGAGGAGCCTGGCGCATTTTGGACTGTGGCTCTCCTGAC
ATTGAAGTCCACACCTATCCCTTCGGATGGTACCAGAAGCCCATTGCTCAAAATAATGCT
CCCAGTTTTTGCCCTTTGGTTGTTCTGCCTTCCCTCACAGAGAAAGTGTCTCTGGACTC
GTGGCAGTCCCTGGCCCTCTCTTCAGTGGTTGTTGATCCATCCATTAGGCACTTTGATG
TTGCCCATGTCAGCACTGCTGCCACCAGCAATTTCTCTGCTGTCCGAGACCTCTGTTTG
TCGGAATGTTCCCAACATGAGGCCTGTCTCATCACCACTCTGCAAACCCAACCTGGGGC
TGTGAGATGTATGTTCTATGCTGATACTCAAAGCTGCACACATAGTCTGCAGGGTCAGA
ACTGCCGACTTCTGCTTCGTGAAGAGGCCACCCACATCTACCGGAAGCCAGGAATCTCT
CTGCTCAGCTATGAGGCATCTGTACCTTCTGTGCCCATTTCCACCCATGGCCGGCTGCT
GGGCAGGTCCCAGGCCATCCAGGTGGGTACCTCATGGAAGCAAGTGGACCAGTTCCTT
GGAGTTCCATATGCTGCCCCGCCCCTGGCAGAGAGGCGCTTCCAGGCACCAGAGCCC
TTGAACTGGACAGGCTCCTGGGATGCCAGCAAGCCAAGGGCCAGCTGCTGGCAGCCA
GGCACCAGAACATCCACGTCTCCTGGAGTCAGTGAAGATTGTTTGTATCTCAATGTGTT
CATCCCTCAGAATGTGGCCCCTAACGCGTCTGTGCTGGTGTTCTTCCACAACACCATGG
ACAGGGAGGAGAGTGAAGGATGGCCGGCTATCGACGGCTCCTTCTTGGCTGCTGTTGG
CAACCTCATCGTGGTCACTGCCAGCTACCGAGTGGGTGTCTTCGGCTTCCTGAGTTCTG
GGTCCGGAGAGGTGAGTGGCAACTGGGGGCTGCTGGACCAGGTGGCGGCTCTGACCT
GGGTGCAGACCCACATCCGAGGATTTGGCGGGGACCCTCGGCGCGTGTCCCTGGCAG
CAGACCGTGGCGGGGCTGATGTGGCCAGCATCCACCTTCTCACGGCCAGGGCCACCA
ACTCCCAACTTTTCCGGAGAGCTGTGCTGATGGGAGGCTCCGCACTCTCCCCGGCCGC
 
CGTCATCAGCCATGAGAGGGCTCAGCAGCAGGCAATTGCTTTGGCAAAGGAGGTCAGT
TGCCCCATGTCATCCAGCCAAGAAGTGGTGTCCTGCCTCCGCCAGAAGCCTGCCAATG
TCCTCAATGATGCCCAGACCAAGCTCCTGGCCGTGAGTGGCCCTTTCCACTACTGGGG
TCCTGTGATCGATGGCCACTTCCTCCGTGAGCCTCCAGCCAGAGCACTGAAGAGGTCT
TTATGGGTAGAGGTCGATCTGCTCATTGGGAGTTCTCAGGACGACGGGCTCATCAACA
GAGCAAAGGCTGTGAAGCAATTTGAGGAAAGTCGAGGCCGGACCAGTAGCAAAACAGC
CTTTTACCAGGCACTGCAGAATTCTCTGGGTGGCGAGGACTCAGATGCCCGCGTCGAG
GCTGCTGCTACATGGTATTACTCTCTGGAGCACTCCACGGATGACTATGCCTCCTTCTC
CCGGGCTCTGGAGAATGCCACCCGGGACTACTTTATCATCTGCCCTATAATCGACATGG
CCAGTGCCTGGGCAAAGAGGGCCCGAGGAAACGTCTTCATGTACCATGCTCCTGAAAA
CTACGGCCATGGCAGCCTGGAGCTGCTGGCGGATGTTCAGTTTGCCTTGGGGCTTCCC
TTCTACCCAGCCTACGAGGGGCAGTTTTCTCTGGAGGAGAAGAGCCTGTCGCTGAAAAT
CATGCAGTACTTTTCCCACTTCATCAGATCAGGAAATCCCAACTACCCTTATGAGTTCTC
ACGGAAAGTACCCACATTTGCAACCCCCTGGCCTGACTTTGTACCCCGTGCTGGTGGA
GAGAACTACAAGGAGTTCAGTGAGCTGCTCCCCAATCGACAGGGCCTGAAGAAAGCCG
ACTGCTCCTTCTGGTCCAAGTACATCTCGTCTCTGAAGACATCTGCAGATGGAGCCAAG
GGCGGGCAGTCAGCAGAGAGTGAAGAGGAGGAGTTGACGGCTGGATCTGGGCTAAGA
GAAGATCTCCTAAGCCTCCAGGAACCAGGCTCTAAGACCTACAGCAAGTGACCAGCCC
TTGAGCTCCCCAAAAACCTCACCCGAGGCTGCCCACTATGGTCATCTTTTTCTCTAAAAT
AGCCACTTACCTTCAATAAAGTATCTACATGCGGTGAA
**SEQ ID NO: 6:** The amino acid sequence of TG target protein is provided as SEQ ID NO: 6. All the variants of TG target protein are included in the context of the invention. The sequence in italics refers to the signal peptide of the TG target protein. 
MALVLEIFTLLASICWVSANIFEYQVDAQPLRPCELQRETAFLKQADYVPQCAEDGSFQTVQ
CQNDGRSCWCVGANGSEVLGSRQPGRPVACLSFCQLQKQQILLSGYINSTDTSYLPQCQD
SGDYAPVQCDVQQVQCWCVDAEGMEVYGTRQLGRPKRCPRSCEIRNRRLLHGVGDKSP
PQCSAEGEFMPVQCKFVNTTDMMIFDLVHSYNRFPDAFVTFSSFQRRFPEVSGYCHCADS
QGRELAETGLELLLDEIYDTIFAGLDLPSTFTETTLYRILQRRFLAVQSVISGRFRCPTKCEVE
RFTATSFGHPYVPSCRRNGDYQAVQCQTEGPCWCVDAQGKEMHGTRQQGEPPSCAEGQ
SCASERQQALSRLYFGTSGYFSQHDLFSSPEKRWASPRVARFATSCPPTIKELFVDSGLLR
PMVEGQSQQFSVSENLLKEAIRAIFPSRGLARLALQFTTNPKRLQQNLFGGKFLVNVGQFNL
SGALGTRGTFNFSQFFQQLGLASFLNGGRQEDLAKPLSVGLDSNSSTGTPEAAKKDGTMN
KPTVGSFGFEINLQENQNALKFLASLLELPEFLLFLQHAISVPEDVARDLGDVMETVLSSQTC
EQTPERLFVPSCTTEGSYEDVQCFSGECWCVNSWGKELPGSRVRGGQPRCPTDCEKQR
ARMQSLMGSQPAGSTLFVPACTSEGHFLPVQCFNSECYCVDAEGQAIPGTRSAIGKPKKC
PTPCQLQSEQAFLRTVQALLSNSSMLPTLSDTYIPQCSTDGQWRQVQCNGPPEQVFELYQ
 
RWEAQNKGQDLTPAKLLVKIMSYREAASGNFSLFIQSLYEAGQQDVFPVLSQYPSLQDVPL
AALEGKRPQPRENILLEPYLFWQILNGQLSQYPGSYSDFSTPLAHFDLRNCWCVDEAGQEL
EGMRSEPSKLPTCPGSCEEAKLRVLQFIRETEEIVSASNSSRFPLGESFLVAKGIRLRNEDL
GLPPLFPPREAFAEQFLRGSDYAIRLAAQSTLSFYQRRRFSPDDSAGASALLRSGPYMPQC
DAFGSWEPVQCHAGTGHCWCVDEKGGFIPGSLTARSLQIPQCPTTCEKSRTSGLLSSWKQ
ARSQENPSPKDLFVPACLETGEYARLQASGAGTWCVDPASGEELRPGSSSSAQCPSLCNV
LKSGVLSRRVSPGYVPACRAEDGGFSPVQCDQAQGSCWCVMDSGEEVPGTRVTGGQPA
CESPRCPLPFNASEVVGGTILCETISGPTGSAMQQCQLLCRQGSWSVFPPGPLICSLESGR
WESQLPQPRACQRPQLWQTIQTQGHFQLQLPPGKMCSADYADLLQTFQVFILDELTARGF
CQIQVKTFGTLVSIPVCNNSSVQVGCLTRERLGVNVTWKSRLEDIPVASLPDLHDIERALVG
KDLLGRFTDLIQSGSFQLHLDSKTFPAETIRFLQGDHFGTSPRTWFGCSEGFYQVLTSEASQ
DGLGCVKCPEGSYSQDEECIPCPVGFYQEQAGSLACVPCPVGRTTISAGAFSQTHCVTDC
QRNEAGLQCDQNGQYRASQKDRGSGKAFCVDGEGRRLPWWETEAPLEDSQCLMMQKFE
KVPESKVIFDANAPVAVRSKVPDSEFPVMQCLTDCTEDEACSFFTVSTTEPEISCDFYAWTS
DNVACMTSDQKRDALGNSKATSFGSLRCQVKVRSHGQDSPAVYLKKGQGSTTTLQKRFEP
TGFQNMLSGLYNPIVFSASGANLTDAHLFCLLACDRDLCCDGFVLTQVQGGAIICGLLSSPS
VLLCNVKDWMDPSEAWANATCPGVTYDQESHQVILRLGDQEFIKSLTPLEGTQDTFTNFQQ
VYLWKDSDMGSRPESMGCRKDTVPRPASPTEAGLTTELFSPVDLNQVIVNGNQSLSSQKH
WLFKHLFSAQQANLWCLSRCVQEHSFCQLAEITESASLYFTCTLYPEAQVCDDIMESNAQG
CRLILPQMPKALFRKKVILEDKVKNFYTRLPFQKLMGISIRNKVPMSEKSISNGFFECERRCD
ADPCCTGFGFLNVSQLKGGEVTCLTLNSLGIQMCSEENGGAWRILDCGSPDIEVHTYPFGW
YQKPIAQNNAPSFCPLVVLPSLTEKVSLDSWQSLALSSVVVDPSIRHFDVAHVSTAATSNFS
AVRDLCLSECSQHEACLITTLQTQPGAVRCMFYADTQSCTHSLQGQNCRLLLREEATHIYR
KPGISLLSYEASVPSVPISTHGRLLGRSQAIQVGTSWKQVDQFLGVPYAAPPLAERRFQAPE
PLNWTGSWDASKPRASCWQPGTRTSTSPGVSEDCLYLNVFIPQNVAPNASVLVFFHNTMD
REESEGWPAIDGSFLAAVGNLIVVTASYRVGVFGFLSSGSGEVSGNWGLLDQVAALTWVQ
THIRGFGGDPRRVSLAADRGGADVASIHLLTARATNSQLFRRAVLMGGSALSPAAVISHERA
QQQAIALAKEVSCPMSSSQEVVSCLRQKPANVLNDAQTKLLAVSGPFHYWGPVIDGHFLRE
PPARALKRSLWVEVDLLIGSSQDDGLINRAKAVKQFEESRGRTSSKTAFYQALQNSLGGED
SDARVEAAATWYYSLEHSTDDYASFSRALENATRDYFIICPIIDMASAWAKRARGNVFMYHA
PENYGHGSLELLADVQFALGLPFYPAYEGQFSLEEKSLSLKIMQYFSHFIRSGNPNYPYEFS
RKVPTFATPWPDFVPRAGGENYKEFSELLPNRQGLKKADCSFWSKYISSLKTSADGAKGG
QSAESEEEELTAGSGLREDLLSLQEPGSKTYSK
**SEQ ID NO: 7:** The nucleotide sequence of ITGB8 target gene is represented by SEQ ID NO: 7. All the variants of ITGB8 target gene are included in the context of the invention. The underlined nucleotides correspond to the ITGB8 target protein coding sequence of ITGB8 target gene. 
AGAGCGCGCCAGCAACTCGGGCTCTGGACTGCGGACGCCTGAGCCGCGCACTGAGG
CGAAAAGGACAAGGGCACGCAGCCCCCGCCCCGCGAAGCCGGGCTCCGGCACCTCG
CGGAACCTCCCCTCCTGCGCTCGCAGCCGCAGGCCCCACTCCCCGCCAGGGAGGGAG
CGGCGATGCCTCGCTCTGTGCCTGCGGGTGTCGGCGGTGCTTCTAGGGCGCTCCCA
GAGCCGCCTCCCCCTGTTGCTGGCATCCCGAGCTTCCTCCCTTGCCAGCCAGGACGCT
GCCGACTTGTCTTTGCCCGCTGCTCCGCAGACGGGGCTGCAAAGCTGCAACTAATGGT
GTTGGCCTCCCTGCCCACCTGTGGAAGCAACTGCGCTGATTGATGCGCCACAGACTTTT
TTCCCCTCGACCTCGCCGGCGTCCCCTCCCACAGATCCAGCATCACCCAGTGAATGTA
CATTAGGGTGGTTTCCCCCCCAGCTTCGGGCTTTGTTTGGGTTTGATTGTGTTTGGCTC
TTCGCTAAGCTGATTTATGCAGCAGAAGCCCCACCGGCTGGAGAGAAACAAAAGCTCTT
TTCTTTGTCCCGGAGCAGGCTGCGGAGCCCTTGCAGAGCCCTCTCTCCAGTCGCCGCC
GGGGCCCTTGGCCGTCGAAGGAGGTGCTTCTCGCGGAGACCGCGGACCCGCCGTG
CCGAGCCGGGAGGGCCGCAGGGGCCCTGAGATGCCGAGCGGTGCCCGGGCCCGCTT
ACCTGCACCGCTTGCTCCGAGCCGCGGGGTCCGCCTGCTAGGCCTGCGGAAAACGTC
CTAGCGACACTCGGCCCGCGGGCCCCGAGGTGCGCCCGGGAGGCGCGAGCCCGCGT
CCGGAAGGCAGTCAGGCGGCGGGCGCGGGGCGGGCTGTTTTGCATTATGTGCGGCTC
GGCCCTGGCTTTTTTACCGCTGCATTTGTCTGCCTGCAAAACGACCGGCGAGGTCCC
GCCTCGTTCCTCTGGGCAGCCTGGGTGTTTTCACTTGTTCTTGGACTGGGCCAAGGTGA
AGACAATAGATGTGCATCTTCAAATGCAGCATCCTGTGCCAGGTGCCTTGCGCTGGGTC
CAGAATGTGGATGGTGTGTTCAAGAGGATTTCATTTCAGGTGGATCAAGAAGTGAACGT
TGTGATATTGTTTCCAATTTAATAAGCAAAGGCTGCTCAGTTGATTCAATAGAATACCCAT
CTGTGCATGTTATAATACCCACTGAAAATGAAATTAATACCCAGGTGACACCAGGAGAA
GTGTCTATCCAGCTGCGTCCAGGAGCCGAAGCTAATTTTATGCTGAAAGTTCATCCTCT
GAAGAAATATCCTGTGGATCTTTATTATCTTGTTGATGTCTCAGCATCAATGCACAATAAT
ATAGAAAAATTAAATTCCGTTGGAAACGATTTATCTAGAAAAATGGCATTTTTCTCCCGTG
ACTTTCGTCTTGGATTTGGCTCATACGTTGATAAAACAGTTTCACCATACATTAGCATCC
ACCCCGAAAGGATTCATAATCAATGCAGTGACTACAATTTAGACTGCATGCCTCCCCAT
GGATACATCCATGTGCTGTCTTTGACAGAGAACATCACTGAGTTTGAGAAAGCAGTTCAT
AGACAGAAGATCTCTGGAAACATAGATACACCAGAAGGAGGTTTTGACGCCATGCTTCA
GGCAGCTGTCTGTGAAAGTCATATCGGATGGCGAAAAGAGGCTAAAAGATTGCTGCTG
GTGATGACAGATCAGACGTCTCATCTCGCTCTTGATAGCAAATTGGCAGGCATAGTGGT
GCCCAATGACGGAAACTGTCATCTGAAAAACAACGTCTATGTCAAATCGACAACCATGG
AACACCCCTCACTAGGCCAACTTTCAGAGAAATTAATAGACAACAACATTAATGTCATCT
TTGCAGTTCAAGGAAAACAATTTCATTGGTATAAGGATCTTCTACCCCTCTTGCCAGGCA
CCATTGCTGGTGAAATAGAATCAAAGGCTGCAAACCTCAATAATTTGGTAGTGGAAGCC
 
TATCAGAAGCTCATTTCAGAAGTGAAAGTTCAGGTGGAAAACCAGGTACAAGGCATCTA
TTTTAACATTACCGCCATCTGTCCAGATGGGTCCAGAAAGCCAGGCATGGAAGGATGCA
GAAACGTGACGAGCAATGATGAAGTTCTTTTCAATGTAACAGTTACAATGAAAAAATGTG
ATGTCACAGGAGGAAAAAACTATGCAATAATCAAACCTATTGGTTTTAATGAAACCGCTA
AAATTCATATACACAGAAACTGCAGCTGTCAGTGTGAGGACAACAGAGGACCTAAAGGA
AAGTGTGTAGATGAAACTTTTCTAGATTCCAAGTGTTTCCAGTGTGATGAGAATAAATGT
CATTTTGATGAAGATCAGTTTTCTTCTGAGAGTTGCAAGTCACACAAGGATCAGCCTGTT
TGCAGTGGTCGAGGAGTTTGTGTTTGTGGGAAATGTTCATGTCACAAAATTAAGCTTGG
AAAAGTGTATGGAAAATACTGTGAAAAGGATGACTTTTCTTGTCCATATCACCATGGAAA
TCTGTGTGCTGGGCATGGAGAGTGTGAAGCAGGCAGATGCCAATGCTTCAGTGGCTGG
GAAGGTGATCGATGCCAGTGCCCTTCAGCAGCAGCCCAGCACTGTGTCAATTCAAAGG
GCCAAGTGTGCAGTGGAAGAGGCACGTGTGTGTGTGGAAGGTGTGAGTGCACCGATCC
CAGGAGCATCGGCCGCTTCTGTGAACACTGCCCCACCTGTTATACAGCCTGCAAGGAA
AACTGGAATTGTATGCAATGCCTTCACCCTCACAATTTGTCTCAGGCTATACTTGATCAG
TGCAAAACCTCATGTGCTCTCATGGAACAACAGCATTATGTCGACCAAACTTCAGAATGT
TTCTCCAGCCCAAGCTACTTGAGAATATTTTTCATCATTTTCATAGTTACATTCTTGATTG
GGTTGCTTAAAGTCCTGATCATTAGACAGGTGATACTACAATGGAATAGTAATAAAATTA
AGTCCTCATCAGATTACAGAGTGTCAGCCTCAAAAAAGGATAAGTTGATTCTGCAAAGTG
TTTGCACAAGAGCAGTCACCTACCGACGTGAGAAGCCTGAAGAAATAAAAATGGATATC
AGCAAATTAAATGCTCATGAAACTTTCAGGTGCAACTTCTAAAAAAAGATTTTTAAACACT
TAATGGGAAACTGGAATTGTTAATAATTGCTCCTAAAGATTATAATTTTAAAAGTCACAGG
AGGAGACAAATTGCTCACGGTCATGCCAGTTGCTGGTTGTACACTCGAACGAAGACTGA
CAAGTATCCTCATCATGATGTGACTCACATAGCTGCTGACTTTTTCAGAGAAAAATGTGT
CTTACTACTGTTTGAGACTAGTGTCGTTGTAGCACTTTACTGTAATATATAACTTATTTAG
ATCAGCATAGAATGTAGATCCTCTGAAGAGCACTGATTACACTTTACAGGTACCTGTTAT
CCCTACGCTTCCCAGAGAGAACAATGCTGTGAGAGAGTTTAGCATTGTGTCACTACAAG
GGTACAGTAATCCCTGCACTGGACATGTGAGGAAAAAAATAATCTGGCAAGTATATTCTA
AGGTTGCCAAACACTTCAACAGTTGGTGGTTGAATAGACAAGAACAGCTAGATGAATAA
ATGATTCGTGTTTCACTCTTTCAAGAGGTGAACAGATACAACCTTAATCTTAAAAGATTAT
TGCTTTTTAAAGTGTGTAGTTTTATGCATGTGTGTTTATGGTTTGCTTATTTTTGCAAGAT
GGATACTAATTCCAGCATTCTCTCCTCTTTGCCTTTATGTTTTGTTTTCTTTTTTACAGGAT
AAGTTTATGTATGTCACAGATGACTGGATTAATTAAGTGCTAAGTTACTACTGCCATAAAA
AACTAATAATACAATGTCACTTTATCAGAATACTAGTTTTAAAAGCTGAATGTTAATAGGG
GACACTGTAAAGTATCATCAAAACCTGAATAGCTTCATTGTGCACAAGTGTGGAGTTTTG
TATCCTCTTACCTGGTAAACTGAAGGGATTGTTTGGCCATTTCATTTATCTTATCATTAAT
TCACAAGATAGTTAGAAATTCTGCCTCAAGCAAAGTACCACATTTTGAATGTTTTCTTAGA
TTTTGATTGCAAGTAGATATCAGCATTTTTTAAATGAAAAGCTATATTATCTTCTCCCTTCA
 
AGGCAGCCTAAGGATGTTCTTTCCCAGAATCACTCCAACCCTTCTTGCCAGAATTCATAA
AAGTACAAAATTGGAGAATAGATGATATCTTAGAAATAAGCTTTTTTTTTTTTTTTTTTT
TTTTGAGACGGAGTTTCACTCTTGTCACCCAGGCTGAAGTGCAATGGCGCAATTAGGGT
TCACTGCAACCTCTGCCTCCCGGGTTCAAGCAGTTCTCCTGCCTCAGCCTCCTGAGTAG
CTGGGATTACAGGCATCCACCACCGTGCCCAGCTAATTTTTGTATTTTTAGTAGAGACG
GGGTTTTGCCATGTTGGACAGGTTGATCTCAAACTCCTGACCTCAGGTGATCTACCCTC
CTCGGCCTCCCAGAGTGTTGGGATTACAGGCATGAGCCACCATGCCAGGCTGCTAATT
CTCCTTTTTAGTGAGTTAGGGAACTGAGCCTCAGAAAACTTAAACGATTTCTCAGAAAAC
ACTCAAGTGATAAAGTGGCCACATTGGAAAGGAGTTTTTATCTTCTCATTGTCAGGCCAG
TGTTCATTGCACAATATCATGCTACCTCTTGAATCTTTAAAATATTCAATTGGCAAATGTT
TTTCAATGTGATTTACTCATGTCTTAAGTGTATGAGGAAAGTTCAAAGCAAAATAGAAAG
GAATAATTCAAACTGAATTGTCCATAATCAGCTTCCAGTCTTTCATGCTAATCAGCTTCTT
AAGAGACTGAAGTATGGCATACCTACAGGGGAATTCCTTCGCACCATAGCCTGTATGAA
CAGTGTTCCCTGGAGTTCTCCAGTGCTCAGCTTGAGACCTTGATACACGGGCCATGAGC
CCTGTCTTCCCCAATGGAAATTTATTTACACTTACCTTATCCCTATGGACTTAGTCTGATT
TTATTGGCTAGGAGTCTAACAGTCCTGTGTGGATATACAGTTTTGCCCATGACAACAAAG
GAATCTATCCGAAATATCTTTTTTTTTATAATAAACTTCCAAGATTTGCTGTCTTCCAGCA
CTTGAGTTAAAGTACTAGATACTGCATTTTGATGAAGACTAACCCCATCTCATATTCTACC
CTAAAGAGAACTGAAAAACCTATAATAAGTTGTTCTGGAGCCAATAAACACAGCAGCTCT
GTTAGATGTCCTCTACAGCCAAGCACTTTCAATGCTAACTTGAACTGCATTTCCTTCCTC
AAATGAGAGATTGACATAATTCAGTACTGTGAGTCACTTGTATAAGAAACCTTTGATCAC
TAAAAATAATGTAAAAATTGGGTTTAGTAGCCTAATACACATAACGTTCTTCTTAAAAAGG
AAAATGGATGGATGCCTGACAACCCTCCAAAAGAAAAAAGTGTAAGATAGCCATTAAGA
TGATGACAATTTTTGAAATGAACATTATGATATTTATGAACAATAAACAAATTTCCGTATG
GAATGAATTATCCAAAAAGAGTATAACAAAATGAAATCCTTAAAAATCCAGAGTTTATATT
TTTTTTATACCCTCACTTGTTTGCACTAACTTTATAGTGGACCAAGGCTGTTACCATAGGA
AGGGACAAACTTCCTTGTAGGCAACTCAGTGTTAGACGATGATTGTGGTTATGCTTGCA
AAGTCTTGTGCTTATCTTTTTTGTTTTTACTTAAAAAGCTAATTTTTAAAGATTGTAGGGCT
TGTATTTTACTTGAATAATTGATATCTTCCTGTGTAATGATTTGTGAGATGAGAATTAATAT
TTGACTAGTTAGAATTAATTAAATGGTAAGGGAACACAGGGTACTCTTAGGTTAAATAAT
GTATGCAAATAGAGTCTATTTTCAACTAATATGGCCACAGGAGCCTTTTGAGATTCATTG
ATATTAAACACAATTAATGAAATTTTAAATTGTTAACAGAATTGAGAACTTGAACAACACT
TTTAGTACTGCAGCATTTTTGTGCCCTAAAGTATGTAATGATTTATAAATGTGCCATACAT
ACACTACAACATAACATTTGCTTTGTTATGCATTTTATTTCTCTGGGGACACCATTGCACT
GCAGTGCACACGTATTTATAAACATTTGTTATATTTTTGGAAACTTGCTAATATTTATTAA
GTCATAGACTTTTCTGGAGGACTTAAAAATTCACTAAAAATCTGATTATGTCTTAAATGTT
CAGTTTATCTTTGGTTTATTAAAATAAAAAAAAAATCTAAGATTAAACACAGTAGATATCTC
 
TGGAGGCAATTTTCCAAAACTCAACATTAAAATTTGTGGATGCATGAGATGCAATCCTTC
AAAGAATGAATCTGAAATATATTTTTAATATTTACTTAATATCCACTGAAGATATCTTTATG
CAAGACAAGAGTCAGCCATCAGACACTGAAATATATTATGATAGATTATGAAGAATTTTC
TCTGTAGAATTATATTCTTCCTGGAACCTGGTAGAGTAGATTAGACTCAAAGGCTTTTTC
TTCCTTTTCTTACTCCTGTTTTTTCCACTCACTCTTCCCAAGAGATTTCCTAAAGCTTCAA
GCTTAATAAGCCTAATAGTGAAAAATAACTGAATTTAATGGTATAATGAAGTTCTTCATTT
CCAGACATCTTTAATTGATCTTAAAGCTCATTTGAGTCTTTGCCCCTGAACAAAGACAGA
CCCATTAAAATCTAAGAATTCTAAATTTTCACAACTGTTTGAGCTTCTTTTCATTTTGAAG
GATTTGGAATATATATGTTTTCATAAAAGTATCAAGTGAAATATAGTTACATGGGAGCTCA
ATCATGTGCAGATTGCATTCTGTTATGTTGACTCAATATTTAATTTACAACTATCCTTATTT
ATATTGACCTCAAGAACTCCATTTTATGCAATGCAGACCACTGAGATATAGCTAACATTC
TTTCAAATAATTTTCCTTTTCTTTTATAATTCCTCTATAGCAAATTTTTATGTATAACTGATT
ATACATATCCATATTTATATTTCATTGATTCCAAGACATCACTTTTTCAATTTAACATCTCT
GAAATTGTGACATTTCTTGCAACTGTTGGCACTTCAGATGCAGTGTTTAAAATTATGCTT
GAATAAATATTACACTAATCCAACTTTACCTAAATGTTTATGCATCTAGGCAAATTTTGTTT
TCTTATAAAGATTTGAGAGCCCATTTATGACAAAATATGAAGGCGAAATTTAAGGACAAC
TGAGTCACGCACAACTCAACATGGAGCCTAACTGATTATCAGCTCAGATCCCGCATATC
TTGAGTTTACAAAAGCTCTTTCAGGTCCCCATTTATACTTTACGTGAGTGCGAATGATTT
CAGCAAACCCTAACTTAACTAACAAGAATGGGTAGGTATGTCTACGTTTCATTAACAAAT
TTTTATTATTTTTATTCTATTATATGAGATCCTTTTATATTATCATCTCACTTTTAAACAAAA
TTAACTGGAAAAATATTACATGGAACTGTCATAGTTAGGTTTTGCAGCATCTTACATGTCT
TGTATCAATGGCAGGAGAAAAATATGATAAAAACAATCAGTGCTGTGAAAAACAACTTTC
TTCTAGAGTCCTCTTACTTTTTATTCTTCTTTATCATTTGTGGGTTTTTCCCCCTTGGCTCT
GATCACTTTAACTTCAAGCTTATGTAACGACTGTTATAAAACTGCATATTTAAATTATTTG
AATTATATGAAATAATTGTTCAGCTATCTGGGCAGCTGTTAATGTAAACCTGAGAGTAAT
AACACTACTCTTTTATCTACCTGGAATACTTTTCTGCATAAAATTTATCTTTGTAAGCTAAC
TCTATTAATCAGGTTTCTTCTAGCCTCTGCAACCTACTTCAGTTAGAATTGTCTAATACTG
CTCTATTAATCAGGTTTCTAGCCTCTACAACCTACTTCAGTTAAAATTGTCTAATACAGCA
ATATTTAAAAAAAAACACTGCAATTGTCAAGGATGGAAAATGTGTGATTTGTGTAAACAA
TTTTTACCAACTTTACATTTTCCTACAGATAAATGTGAAATTTTGATAAGAAGTCTACGCA
ATGACAAGTATGGTACATAAATTTTATTAAGAATATTGAGTATAAAGTACTTTAATTCTAAA
TTATAAGAAAATATACATTTGCACATATTAATATAGAAATTCATTTTGTGTATATTTAACAT
AGCTTTTAAACTATTTTACATTAGCTACTTCATTATGGTTTCTTGAACTTCTGAAAAAAATT
AGAAATGTATTAAACTTATCAGTAACATAAAAACTTATTTTGTTTCACCTAACGAATACTG
CGTTTGTAAAAATAAATTTAATATAGAATATATTTTTAAATTAAATATTTGAATATAAAATAG
CTCTAAGAAAGAAGCAAATTATCACTGAACATATTTCTTATTATTTCTGGCTTTGAATTAT
ACGTAACTTAAATTGTCTTAAATGATACAGAATATTGGAGAATATGATACTTTCACATAAT
 
ATACTATGAACCTGTTCATATAACTCTGATTGACTACTAACTTCTGTTTTATGTATTTATTA
AAGAGCTGACACTGTAGTTTGTGGTGAGATGTTTATTTTTCTAACAGAGCTTATAACAGT
TAGGACAAGGCATTTAATTAATGCATCATTCTGTTTAGTAGTAGGTGTTAATCAATATGAA
ATTCTCTGTTTTAAAATAAAAATGTAAAAATCTAA
**SEQ ID NO: 8:** The amino acid sequence of ITGB8 target protein is represented by SEQ ID NO: 8. All the variants of ITGB8 target protein are included in the context of the invention. The amino acids in italics correspond to the signal peptide of the ITGB8 target protein. 
MCGSALAFFTAAFVCLQNDRRGPASFLWAAWVFSLVLGLGQGEDNRCASSNAASCARCLA
LGPECGWCVQEDFISGGSRSERCDIVSNLISKGCSVDSIEYPSVHVIIPTENEINTQVTPGEV
SIQLRPGAEANFMLKVHPLKKYPVDLYYLVDVSASMHNNIEKLNSVGNDLSRKMAFFSRDF
RLGFGSYVDKTVSPYISIHPERIHNQCSDYNLDCMPPHGYIHVLSLTENITEFEKAVHRQKIS
GNIDTPEGGFDAMLQAAVCESHIGWRKEAKRLLLVMTDQTSHLALDSKLAGIVVPNDGNCH
LKNNVYVKSTTMEHPSLGQLSEKLIDNNINVIFAVQGKQFHWYKDLLPLLPGTIAGEIESKAA
NLNNLVVEAYQKLISEVKVQVENQVQGIYFNITAICPDGSRKPGMEGCRNVTSNDEVLFNVT
VTMKKCDVTGGKNYAIIKPIGFNETAKIHIHRNCSCQCEDNRGPKGKCVDETFLDSKCFQCD
ENKCHFDEDQFSSESCKSHKDQPVCSGRGVCVCGKCSCHKIKLGKVYGKYCEKDDFSCPY
HHGNLCAGHGECEAGRCQCFSGWEGDRCQCPSAAAQHCVNSKGQVCSGRGTCVCGRC
ECTDPRSIGRFCEHCPTCYTACKENWNCMQCLHPHNLSQAILDQCKTSCALMEQQHYVDQ
TSECFSSPSYLRIFFIIFIVTFLIGLLKVLIIRQVILQWNSNKIKSSSDYRVSASKKDKLILQSVCT
RAVTYRREKPEEIKMDISKLNAHETFRCNF
**SEQ ID NO: 9:** The nucleotide sequence of LILRB1 target gene is represented by SEQ ID NO: 9. All the variants of LILRB1 target gene are included in the context of the invention. The underlined nucleotides correspond to the LILRB1 target protein coding sequence of LILRB1 target gene. 
GCATTTCTCTTCTGTGCTCACTGCCACACGCAGCTCAGCCTGGGCGGCACAGCCAGAT
GCGAGATGCGTCTCTGCTGATCTGAGTCTGCCTGCAGCATGGACCTGGGTCTTCCCTG
AAGCATCTCCAGGGCTGGAGGGACGACTGCCATGCACCGAGGGCTCATCCATCCACAG
AGCAGGGCAGTGGGAGGAGACGCCATGACCCCCATCCTCACGGTCCTGATCTGTCTCG
GGCTGAGTCTGGGCCCCCGGACCCACGTGCAGGCAGGGCACCTCCCCAAGCCCACCC
TCTGGGCTGAACCAGGCTCTGTGATCACCCAGGGGAGTCCTGTGACCCTCAGGTGTCA
GGGGGGCCAGGAGACCCAGGAGTACCGTCTATATAGAGAAAAGAAAACAGCACCCTGG
ATTACACGGATCCCACAGGAGCTTGTGAAGAAGGGCCAGTTCCCCATCCCATCCATCAC
CTGGGAACACACAGGGCGGTATCGCTGTTACTATGGTAGCGACACTGCAGGCCGCTCA
GAGAGCAGTGACCCCCTGGAGCTGGTGGTGACAGGAGCCTACATCAAACCCACCCTCT
CAGCCCAGCCCAGCCCCGTGGTGAACTCAGGAGGGAATGTAACCCTCCAGTGTGACTC
 
ACAGGTGGCATTTGATGGCTTCATTCTGTGTAAGGAAGGAGAAGATGAACACCCACAAT
GCCTGAACTCCCAGCCCCATGCCCGTGGGTCGTCCCGCGCCATCTTCTCCGTGGGCCC
CGTGAGCCCGAGTCGCAGGTGGTGGTACAGGTGCTATGCTTATGACTCGAACTCTCCC
TATGAGTGGTCTCTACCCAGTGATCTCCTGGAGCTCCTGGTCCTAGGTGTTTCTAAGAA
GCCATCACTCTCAGTGCAGCCAGGTCCTATCGTGGCCCCTGAGGAGACCCTGACTCTG
CAGTGTGGCTCTGATGCTGGCTACAACAGATTTGTTCTGTATAAGGACGGGGAACGTGA
CTTCCTTCAGCTCGCTGGCGCACAGCCCCAGGCTGGGCTCTCCCAGGCCAACTTCACC
CTGGGCCCTGTGAGCCGCTCCTACGGGGGCCAGTACAGATGCTACGGTGCACACAAC
CTCTCCTCCGAGTGGTCGGCCCCCAGCGACCCCCTGGACATCCTGATCGCAGGACAGT
TCTATGACAGAGTCTCCCTCTCGGTGCAGCCGGGCCCCACGGTGGCCTCAGGAGAGAA
CGTGACCCTGCTGTGTCAGTCACAGGGATGGATGCAAACTTTCCTTCTGACCAAGGAG
GGGGCAGCTGATGACCCATGGCGTCTAAGATCAACGTACCAATCTCAAAAATACCAGGC
TGAATTCCCCATGGGTCCTGTGACCTCAGCCCATGCGGGGACCTACAGGTGCTACGGC
TCACAGAGCTCCAAACCCTACCTGCTGACTCACCCCAGTGACCCCCTGGAGCTCGTGG
TCTCAGGACCGTCTGGGGGCCCCAGCTCCCCGACAACAGGCCCCACCTCCACATCTGC
AGGCCCTGAGGACCAGCCCCTCACCCCCACCGGGTCGGATCCCCAGAGTGGTCTGGG
AAGGCACCTGGGGGTTGTGATCGGCATCTTGGTGGCCGTCATCCTACTGCTCCTCCTC
CTCCTCCTCCTCTTCCTCATCCTCCGACATCGACGTCAGGGCAAACACTGGACATCGAC
CCAGAGAAAGGCTGATTTCCAACATCCTGCAGGGGCTGTGGGGCCAGAGCCCACAGAC
AGAGGCCTGCAGTGGAGGTCCAGCCCAGCTGCCGATGCCCAGGAAGAAAACCTCTATG
CTGCCGTGAAGCACACACAGCCTGAGGATGGGGTGGAGATGGACACTCGGCAGAGCC
CACACGATGAAGACCCCCAGGCAGTGACGTATGCCGAGGTGAAACACTCCAGACCTAG
GAGAGAAATGGCCTCTCCTCCTTCCCCACTGTCTGGGGAATTCCTGGACACAAAGGACA
GACAGGCGGAAGAGGACAGGCAGATGGACACTGAGGCTGCTGCATCTGAAGCCCCCC
AGGATGTGACCTACGCCCAGCTGCACAGCTTGACCCTCAGACGGGAGGCAACTGAGCC
TCCTCCATCCCAGGAAGGGCCCTCTCCAGCTGTGCCCAGCATCTACGCCACTCTGGCC
ATCCACTAGCCCAGGGGGGGACGCAGACCCCACACTCCATGGAGTCTGGAATGCATGG
GAGCTGCCCCCCCAGTGGACACCATTGGACCCCACCCAGCCTGGATCTACCCCAGGAG
ACTCTGGGAACTTTTAGGGGTCACTCAATTCTGCAGTATAAATAACTAATGTCTCTACAA
TTTTGAAATAAAGCAACAGACTTCTCAATAATCAATGAAGTAGCTGAGAAAACTAAGTCA
GAAAGTGCATTAAACTGAATCACAATGTAAATATTACACATCAAGCGATGAAACTGGAAA
ACTACAAGCCACGAATGAATGAATTAGGAAAGAAAAAAAGTAGGAAATGAATGATCTTG
GCTTTCCTATAAGAAATTTAGGGCAGGGCACGGTGGCTCACGCCTGTAATTCCAGCACT
TTGGGAGGCCGAGGCGGGCAGATCACGAGTTCAGGAGATCGAGACCATCTTGGCCAA
CATGGTGAAACCCTGTCTCTCCTAAAAATACAAAAATTAGCTGGATGTGGTGGCAGTGC
CTGTAATCCCAGCTATTTGGGAGGCTGAGGCAGGAGAATCGCTTGAACCAGGGAGTCA
GAGGTTTCAGTGAGCCAAGATCGCACCACTGCTCTCCAGCCTGGCGACAGAGGGAGAC
 
TCCATCTCAAATTAAAAAAAAAAAAAAAAAAAGAAAGAAAAAGAGAAAAAAGAAATTTAGAA
GAATAACAAGTTATTCCAAATGAAGGCGTAAGAAAGGGAATAATAACAATAATAAGAGGA
GTTGTTCATGAGGAAAAACCAAAGCTTGAAAATTCAACAAAGCCAGTGAAGCTCATTCTT
GAAAACATGAATCACACTCATGAATTCTAACTACAATGAAAAAGAGAAAGAAAGAGCAG
GCATGCATTTCCATATGGGAGTGAGCCAGCAGACAGCCCTACAGATCGTACACACGTTT
TCCAAAACTAACAATGGAACAGGCGGCAAACCTATGCCAATATACTAGAAATTGCAGATT
AAATAGATGAAATATTCTAAACTGGAGTTTACATAATGAACATAAGAGTAATCAGAGAAT
CTGACTCATTTTAGATGTGTGTGTGTGTGTATATATATGTGTGTGTGTGTGAAAAACATT
GACTATAATAAAAATAATCTCGAGTTCA
**SEQ ID NO: 10:** The amino acid sequence of LILRB1 target protein is represented by SEQ ID NO: 10. All the variants of LILRB1 target protein are included in the context of the invention. The amino acids in italics correspond to the signal peptide of the LILRB1 target protein. 
MTPILTVLICLGLSLGPRTHVQAGHLPKPTLWAEPGSVITQGSPVTLRCQGGQETQEYRL
YREKKTAPWITRIPQELVKKGQFPIPSITWEHTGRYRCYYGSDTAGRSESSDPLELVVTGAYI
KPTLSAQPSPVVNSGGNVTLQCDSQVAFDGFILCKEGEDEHPQCLNSQPHARGSSRA
IFSVGPVSPSRRWWYRCYAYDSNSPYEWSLPSDLLELLVLGVSKKPSLSVQPGPIVAPEE
TLTLQCGSDAGYNRFVLYKDGERDFLQLAGAQPQAGLSQANFTLGPVSRSYGGQYRCYGA
HNLSSEWSAPSDPLDILIAGQFYDRVSLSVQPGPTVASGENVTLLCQSQGWMQTFLLTKE
GAADDPWRLRSTYQSQKYQAEFPMGPVTSAHAGTYRCYGSQSSKPYLLTHPSDPLELVVS
GPSGGPSSPTTGPTSTSAGPEDQPLTPTGSDPQSGLGRHLGVVIGILVAVILLLLLLLLL
FLILRHRRQGKHWTSTQRKADFQHPAGAVGPEPTDRGLQWRSSPAADAQEENLYAAVKHT
QPEDGVEMDTRQSPHDEDPQAVTYAEVKHSRPRREMASPPSPLSGEFLDTKDRQAEEDR
QMDTEAAASEAPQDVTYAQLHSLTLRREATEPPPSQEGPSPAVPSIYATLAIH
**SEQ ID NO: 11:** sgRNA AAVS1: GGGGCCACUAGGGACAGGAU
**SEQ ID NO: 12:** sgRNA ZC3H12C: UGUGUCCAAUGAUAACUACA
**SEQ ID NO: 13:** sgRNA UCP3: UGUCCAGUGGAAAGGUAACG
**SEQ ID NO: 14** sgRNA TG: GGCCUGGUCACAUUGCACUG
**SEQ ID NO: 15:** sgRNA ITGB8: AGCACAUGGAUGUAUCCAUG
**SEQ ID NO: 16:** sgRNA LILRB1: CUGGCCCCCCUGACACCUGA

The following examples serve to illustrate the present invention and should not be construed as limiting the scope thereof.

### EXAMPLES

### Example 1. - Material and Methods

### CAR construct and lentiviral production

A second-generation CAR was designed to contain the mutated version of the trastuzumab-based 4D5 scFv (4D5.5) along with the CD28 and CD3ζ endodomains. The scFv sequence M11 (targeting Mesothelin) was extracted from the patent WO2015090230A1260. The scFv sequence targeting CD19 (FMC63) was retrieved from GenBank identifier HM852952.1. These scFv were used in a second-generation CAR with the CD8 hinge, the CD28 transmembrane and intracellular domains and the CD3ζ signaling domains. ARI0001 CAR contains the CD19-binding scFv A3B1, a CD8α extracellular hinge region and transmembrane, along with the 4-1BB costimulatory and CD3ζ endodomains.

All DNA sequences (including CARs or HER2 and CD19 antigens) were synthesized by BaseClear B.V. (Leiden, Netherlands) or GenScript Biotech (New Jersey, United States of America) and cloned into a third-generation lentiviral vector pCCL (kindly provided by Dr. Luigi Naldini (San Raffaele Hospital, Milan)). The expression of the CAR is controlled under the EF1α promoter. All CAR lentiviral vectors were generated by transfecting HEK293FT cells using PEI (Polysciences, # 23966-100). The lentivirus-containing supernatant were collected after 48 hours and 72 hours and concentrated using Lenti-X Concentrator (Takara Bio, # 631232). Lentiviral vectors were tittered based on CAR expression in the T cell membrane using wild-type Jurkat cells by using the limiting dilution method.

**Table 9. CAR constructs**

| **Targeted antigen** | **scFv** | **Hinge region** | **Transmembrane domain** | **Costimulatory domain** | **Intracellular domain** |
|---|---|---|---|---|---|
| HER2 | 4D5.5 | CD8α | CD28 | CD28 | CD3Z |
| Mesothelin | M11 | CD8α | CD28 | CD28 | CD3Z |
| CD19 | A3B1 | CD8α | CD8 | 41BB | CD3Z |

### Cell lines maintenance

All human cancer cell lines were purchased from the American Type Culture Collection (ATCC) except for HEK293FT (human embryonic kidney cells) obtained from ThermoFisher Scientific, #R70007) and Jurkat Clon E6-1 (immortalized human lymphocytes from an acute T-cell leukemia, #88042803-1VL) obtained from Sigma Aldrich. SKOV3 (ovarian cancer cell line, #HTB-77) were cultured in DMEM (Dulbbeco's Modified Eagle's Medium, Gibco, #10741574). CAPAN-2 (pancreatic carcinoma cell line, #HTB-80) were cultured in DMEM/F12 (Gibco, #11320033). L55 (lung cancer cell line), L55-CD19t (L55 cell line overexpressing truncated CD19), L55-HER2t (L55 cell line overexpressing truncated HER2t) and HCC1954 (breast cancer cell line, #CRL-2338) were cultured in RPMI-1640 (Roswell Park Memorial Institute, Gibco, #10741574). HEK293FT were cultured in DMEM supplemented with 1% GlutaMax (ThermoFisher Scientific, #35050-061) and 1% non-essential amino acids (NEAA) (ThermoFisher Scientific, #11140-061). All media were supplemented with 10% FBS (Merk, #9665) and with Penicillin-Streptomycin (P/S) (100mg/mL) (Invitrogen, #15140122). Cell lines were maintained in a humidifier at 37 °C and 5% CO2. All cell lines were regularly tested for mycoplasma.

L55-CD19t and L55-HER2t cell line were generated by transducing the L55 wild-type cell line with lentiviral vectors expressing either CD19t or HER2t (truncated version of CD19 or HER2, respectively) under the control of EF1α promoter. After transduction, tumor cells were stained with CD19-PeCy7 (clone SJ25C1, BD, #557835) or HER2-PE (clone 24D2, Biolegend, #324406). L55 cells expressing CD19 or HER2 were collected using FACSAriall cell sorter (BD).

### CAR-T cell generation

Human primary T lymphocytes were isolated from buffy coats of healthy donors (Banc de Sang i Teixits, Barcelona, Spain) using density gradient centrifugation with Lymphoprep (StemCell, #07801). CD4⁺ or CD8⁺ lymphocytes were separated using RosetteSep for Human T cells (StemCell, #15062 and 15063) and stimulated with CD3/CD28 Human T-Activator Dynabeads (Gibco, #11132D) at a 2:1 bead-to-cell ratio for CD8+ T cell population and at a 1:1 ratio for CD4⁺ T cell population. The CD8⁺ population were cultured with a 10% of CD4+ cells to support T cell proliferation. T lymphocytes were cultured in complete RPMI 1640 media containing 10% heat-inactivated fetal bovine serum (FBS) (Merck, #F4135), 1% Penicillin-Streptomycin (ThermoFisher, #15140122), 1% HEPES (Sigma-Aldrich, #H3375-25G), 1% GlutaMax (ThermoFisher, #35050-061) and 100UI or 50UI of IL-2 (Proleukin^{®}) for CD8 and CD4 T cells respectively. CRISPR-modified CAR-T cells were expanded in the presence of human IL-7 (10 ng/mL, Miltenyi Biotec, # 130-095-362) and human IL-15 (10 ng/mL; Miltenyi Biotec, # 130-095-764) to enhance gene editing.

24h after activation, T lymphocytes were transduced with the appropriate volume of lentiviral vector encoding the CAR construct. Activation beads were magnetically removed on day 3 after isolation. Then, T cells were daily counted from day 5 to day 10 after isolation and maintained at a concentration of 0.8 x10⁶ cells/mL to avoid cell overgrowth, by adding CAR-T medium and transferring the culture to the appropriate flask or plate. On day 10, T cells were frozen in aliquots containing a 1:1 ratio of CD4 and CD8 T cells. Freezing of T cells was performed in a final volume of 1 mL, using 50% freezing solution 80% FBS and 20% DMSO (Merck, #D4551-250ML) and 50% X-VIVO 15 serum-free Hematopoietic cell medium (Lonza, #02-053Q). Cryotubes were placed in a freezing container at -80°C for a 24-h period. Following this initial freezing step, the cryotubes were stored within a liquid nitrogen tank.

To generate a pool of KO CAR-T cells, T cells were co-transduced with two lentiviral vectors: i) one containing the HER2-28Z CAR and ii) a pool of lentiviral vectors at a MOI of 0.2-0.5 to ensure that T cells were transduced with only one vector containing a sgRNA. On day 4 after isolation, T cells were debeaded and GFP-positive cells (as a surrogate of LV integration for the library) were separated by FACS-sorting. After sorting, T cells were rested for 2 h and subsequently electroporated with Cas9 protein. The T cell expansion protocol was then continued as explained from day 5 to day 10-11.

### Genomic disruption of genes by CR/SPR/Cas9-mediated gene editing in primary human T cells

To perform knockout of candidate genes and double knockouts, chemically modified sgRNAs were purchased from Synthego (CRISPRevolution sgRNA EZ Kit, Synthego) and resuspended in nuclease-free water. The sgRNAs used for knocking out candidate genes are indicated in **Table 1.**

To generate CRISPR-mediated KO, we use Neon transfection system (ThermoFisher) or Lonza nucleofection (Lonza). For the Neon, the Cas9 protein was electroporated using Neon Transfection System 100 µL Kit (ThermoFisher Scientific, #MPK10096). Electroporation was performed on day 4 of the T cell expansion protocol. Briefly, T cells were resuspended in Resuspension Buffer R at the desired concentrations in a final volume of 60 µL and kept on ice. 10 µg of TrueCut Cas9 Protein v2 (ThermoFisher Scientific, #A36499) protein were mixed with 60 µL of Resuspension Buffer R per reaction. After this, 100 µL of the mixture were pipetted with the Neon^{™} Transfection System Pipette (ThermoFisher Scientific, #MPP100) into the Neon 100-µL tip provided with the kit. To perform electroporation, the Neon^{™} Transfection System Pipette Station (ThermoFisher Scientific, #MPS100) and the Neon^{™} Transfection System (ThermoFisher Scientific, #MPK5000) were used and the program #24 was applied.

When using the Lonza nucleofection system, knockout of candidate genes was performed on day 3 of the T cell expansion protocol using Lonza 4D Nucleofector X unit (Lonza bioscience #AAF-1003X) in cuvettes from the kit Lonza^{™} P3 Primary Cell 4D-Nucleofector^{™} X Kit L (Lonza^{™} V4XP-3024). RNPs were formed by mixing 2 µL of TrueCut Cas9 Protein v2 (ThermoFisher Scientific, #A36499), 6 µL of P3 buffer with supplement and 2 µL sgRNA, in a final volume of 10 µL per reaction. After incubating the reagents, T cells were then resuspended in 90 µL of supplemented P3 buffer and then, 10 µL of RNPs were mixed with T cells. Samples were transferred to Nucleocuvettes, and nucleofection was performed using the EH-115 program. With both protocols, after electroporation, CAR-T cells were quickly transferred into their conditioned media at a concentration of 1.5 x 10⁶ M/mL to continue with the expansion protocol.

**Table 1. Sequence of sgRNA used for knocking-out candidate genes in T cells.**

| **Gene** | **sgRNA sequence** |
|---|---|
| AAVS1 | GGGGCCACUAGGGACAGGAU (SEQ ID NO: 11) |
| ZC3H12C | UGUGUCCAAUGAUAACUACA (SEQ ID NO: 12) |
| UCP3 | UGUCCAGUGGAAAGGUAACG (SEQ ID NO: 13) |
| LlLRB1 | CUGGCCCCCCUGACACCUGA (SEQ ID NO: 16) |
| TG | GGCCUGGUCACAUUGCACUG (SEQ ID NO: 14) |
| ITGB8 | AGCACAUGGAUGUAUCCAUG (SEQ ID NO: 15) |
| GPR25 | CGCCUCGAGCAGCUUCACCA (SEQ ID NO: 17) |
| LAG-3 | CUCAGCACCGUGUAGCGGCG (SEQ ID NO: 18) |
| PD-1 | CGACUGGCCAGGGCGCCUGU (SEQ ID NO: 19) |

To assess the efficiency of CRISPR/Cas9-mediated KO for candidate genes, a forward and a reverse primer were designed using primer 3 tool in Benchling to amplify approximately 500 bp of the genomic region of the specific gene where the designed sgRNA is expected to hybridize for subsequent Cas9-mediated double strand break. Amplification of the region was performed using Phusion^{®} High-Fidelity DNA Polymerase (NEB, #M0530S). The pairs of forward and reverse primers used to amplify specific regions of candidate genes are indicated in **Table 2.** The efficiency of the knockout using CRISPR/Cas9 system was quantified by using the inference of CRISPR Edits (ICE) CRISPR Analysis tool (Synthego).

**Table 2. Forward (FWD) and reverse (REV) primer for assessing KO of the candidate genes.**

| **Gene** | **Primer sequence** |
|---|---|
| AAVS1 | FWD 5'-AGTTAGAACTCAGGACCAACTTATT (SEQ ID NO: 20) |
| | REV 3'-GAATATAAGGTGGTCCCAGCTC (SEQ ID NO: 21) |
| ZC3H12C | FWD 5'-CAGGGGAGGAGAGTGGTGTGCT (SEQ ID NO: 22) |
| | REV 3'-TACAGGGAAAGGTGGGGCCTGC (SEQ ID NO: 23) |
| UCP3 | FWD 5'-GCTGCAGTGAGCCTGGATTGCA (SEQ ID NO: 24) |
| | REV 3'-GAGGAAGGGCATGTGGGCACAC (SEQ ID NO: 25) |
| LILRB1 | FWD 5'-GGTCCTGGGCTGAGAGCTGGAA (SEQ ID NO: 26) |
| | REV 3'-TAAATGTGGCTCAGACCACC (SEQ ID NO: 27) |
| TG | FWD 5'-CGTGGGTGGTGAGGAGGGTGAT (SEQ ID NO: 28) |
| | REV 3'-TCCCTCCTGCACTTCCTGCCTC (SEQ ID NO: 29) |
| ITGB8 | FWD 5'-CCCTGGCACAAAAGAAGTACTCCC (SEQ ID NO: 30) |
| | REV 3'-GTTCCTCCTTGGAGTGTGGCGC (SEQ ID NO: 31) |
| GPR25 | FWD 5'-TACATCCCCGCGCTCTACCTGG (SEQ ID NO: 32) |
| | REV 3'-TGCGGCAGTAGCAGAAGAGGGT (SEQ ID NO: 33) |
| LAG-3 | FWD 5'-CACCGTTCCTGCCCTTGCTCTG (SEQ ID NO: 34) |
| | REV 3'-ACAATCTCTCCACAGGGCCCCC (SEQ ID NO: 35) |
| PD-1 | FWD 5'-TTTCCCTTCCGCTCACCTCC (SEQ ID NO: 36) |
| | REV 3'-CAAAGAGGGGACTTGGGCCA (SEQ ID NO: 37) |

### In vitro coculture experiments

For real-time cytotoxicity assays, the xCELLigence Real-Time Analyzer System (ACEA Biosciences) was used. 1x10⁴ tumor cells were plated in 50 µL of medium per well of an E-16 plate (Agilent). Approximately 18 h later, effector cells were added in an effector-to-target ratio (E:T) of 5:1 ratio for experiments with SKOV3 tumor cells and 1:1 ratio for experiments with HCC1954 or L55 cells, in a final volume of 100 µL of CAR-T medium. For tumor only control, 100 µL of CAR-T medium were added to each well. Cell index was monitored every 20 min for six days. Relative cell impedance was normalized to the maximum cell index value before T cell plating.

For cytokine production analysis, a total of 1x10⁴ tumor cells were seeded. After overnight incubation, CAR-TILs were added at an effector:target ratio of 3:1. At indicated experiments, anti PD-L1 (Durvalumab) antibody was added to co-cultures at a final concentration of 10 µg/mL. Supernatants were collected 24 h after co-culture. For CAR-independent stimulation of CAR-TILs, a cocktail of PMA and lonomycin (PMA/lo) (Cell stimulation cocktail, eBioscience, #00-4970-93) was added at 1X to the volume of CAR-TIL culture. Supernatants were collected 18 h after stimulation. Co-culture and stimulation experiments were performed in duplicates. The production of IFN-γ and IL-2 were analyzed using the DuoSet ELISA Development Kit (R&D Systems, #DY285B and DY202, respectively) as per the manufacturer's protocol. Optical density of each well was determined using a microplate reading set to 450 nm. The LEGENDplex Human CD8/NK Panel (Biolegend, #741187) was used to assess and quantify the presence of 13 human proteins, including IL-2, IL-4, IL-6, IL-10, IL-17A, IFN-γ, TNF-α, soluble Fas, soluble FasL, Granzyme A, Granzyme B, Perforin, and Granulysin, in supernatants from co-cultures of CAR-TILs and tumor cells or from supernatants of CAR-TILs stimulated with PMA/lo, following manufacturer's instructions. Supernatants were thawed and used undiluted, diluted at 1/5 and diluted 1/10 in a final volume of 50 µL with Assay Buffer. For intracellular staining, 5x10⁵ tumor cells were seeded in 12-well plates. After overnight incubation, T cells were added at indicated effector to target ratio. 20 h later, GolgoPlugTM (BD Bioscience, #555029) was added to each well. 4h later, flow cytometry staining was performed as described below.

### Flow cytometry and antibodies

To assess transduction efficiency, CAR staining was performed on day 9 of T cell expansion. Cells were harvested, washed, and resuspended in FACS buffer (PBS supplemented with 2% FBS) with 2 µL of goat anti-human or goat anti-mouse IgG-biotin. After incubation, cells were washed to ensure removing the unbound antibody and avoid interfering with secondary antibodies. In a second step, T cells were resuspended in FACS buffer containing T cell lineage antibodies, and 1 µL of streptavidin (SA) conjugated to a fluorochrome. SA antibody was either used in PE (ThermoThermoFisher Scientific, #12-4317-87) or eFluo450 (ThermoFisher Scientific, # 48-4317-82) fluorochrome.

For analysis of CAR-TILs phenotype, tumors were collected and disaggregated as explained in section ***Isolation of tumor infiltrating CAR-T cells (CAR-TILs).*** Cell suspensions were then stained with the following antibodies: CD45- PerCP-Cy5.5 (clone HI30; BD Bioscience; #564105), CD4-AF488 (clone RPA-T4; BD Biosciences; # 557695), CD8-APC (clone HIT8a; Biolegend; #300912) or CD8-APC-H7 (clone HIT8a;BD Bioscience; Cat# 300912), PD-1-PeCy7 (clone EH12.1; BD Bioscience; # 561272), LAG-3-BV605 (clone 11C3C65; Biolegend; #369324), TIM3-eFluor710 (clone F38-E2; Biolegend; # 345006) and CD39-PE-Dazzle (clone A1; Biolegend; # 328224). All antibodies were diluted in FACS buffer solution made from phosphate-buffered saline (PBS) and 2% FBS, except for the viability dye, which was resuspended in PBS.

Intracellular staining was performed with the Foxp3/Transcription Factor Staining Buffer set (ThermoFisher, # 00-5523-00) according to the manufacturer's instructions. The following antibodies were used: Ki67-BV605 (clone Ki-67, Biolegend, # 350522), IFNg-PerCP-Cy5.5 (clone B27, BD Biosciences, # 560704) and TNF-α -PE (clone MAb11, BD Biosciences, # 554513). All flow cytometry data were obtained in FacsCanto 3 L, Fortessa 4 L HT and Fortessa 5 L (BD Biosciences) and analyzed with FlowJo software.

### In vitro reestimulation assay

HCC1954 cells were seeded in 6-well plates at 1x10⁶ cells/well in a volume of 4 mL the day preceding T cell seeding. HER2-CAR-T cells were thawed and rested overnight at 4x10⁶ cells/ml with media supplemented with IL-7 and IL-15 cytokines. After overnight resting, T cells were counted by trypan blue exclusion and 3x10⁶ T cells/well were transferred to the HCC1954 plates. Replicates of the co-cultures were established. After 3-4 days, the co-cultures were thoroughly resuspended by frequent pipetting and the T cell number was determined by trypan blue exclusion. Cell suspensions were collected, spun down and resuspended in fresh RPMI media. The resulting cell suspension was transferred into HCC1954-coated plate (3x10⁶ T cell/well) for continuous co-culture. This process was repeated for 14-21 days.

### NSG mouse xenograft models

Tumor cell lines were *in vitro* amplified to the desired number and subsequently trypsinized, washed and resuspended in a solution of 1:1 of PBS with the solubilized extracellular matrix Matrigel (Corning, #11543550), which facilitates tumor engraftment and growth.

Immune-compromised NOD-SCID-Gamma (NSG) mice, either female (for studies with SKOV3 tumors) or male (for studies with L55 and CAPAN-2 tumors) were subcutaneously implanted with 5x10⁶ tumor cells in one or both flanks, in a final volume of 100 µL.

CAR-T cells were administered to tumor-bearing mice using the intravenous route in doses of 1x10⁶-1x10⁷ CAR-T cells per animal, according to the experiment. The day before treatment, tumors were measured and animals were randomized based on tumor size, providing that all treatment groups have similar baseline tumor size. CAR-T cells were thawed and rested overnight in CAR-T medium supplemented with cytokines. The next day, the number of viable CAR-T cells was counted via trypan blue exclusion and washed. After that, CAR-T cells were filtered to eliminate any clumps of cells. Cells were then resuspended in an appropriate volume of PBS to infuse the cell suspension to each animal. Subcutaneous tumors were measured every 3-5 days using a digital caliper and tumor volume was calculated using the following formula: $Tumor volume \left(mm3\right) = \frac{Length \left(L\right) x Width {\left(W\right)}^{2} xπ}{6}$

To analyze T cell persistence, peripheral blood samples were obtained by retro-orbital bleeding at the indicated time points. Blood was examined for T cell persistence using BD Trucount (Cat# 340334, BD Biosciences), and stained for CD45-PerCyP5.5 (BD, Biosciences #564105), CD4-FITC (BD Biosciences, #557695) and CD8-APC (Biolegend, #300912) markers. T-cell numbers were calculated using the following formula: $T cell number \left(cells/μL of blood\right) = \frac{T cell e vents}{Bead events} x \frac{Number of beads per test}{Volume of blood per test}$

All procedures were conducted in compliance with the Animal Ethics Committee of the Universitat de Barcelona.

### Isolation of tumor infiltrating CAR-T cells (CAR-TILs)

To isolate tumor infiltrating CAR-T cells (CAR-TILs), animals were sacrificed, and subcutaneous tumors were collected and then disaggregated using the Tumor Dissociation kit human (Miltenyi Biotec, #130-095-929) following the manufacturer's protocol. After disaggregation of tumor samples, cell suspensions were washed, stained for live and dead and Fc receptors were blocked. To isolate CAR-TILs, we stained for human CD45. FACS-sorting was performed using FACSAriall or FACS Aria SORP (BD) cell sorters. Sorted T cells were collected and resuspended with CAR-T medium supplemented with 100 IU of IL-2. CAR-TILs were rested O/N in a humidified 37 °C, 5% CO₂ incubator. The next day, CAR-TILs were used for functional studies.

In parallel, after disaggregation, a fraction of tumor samples was stained to analyze phenotype of CAR-TILs. Tumor cell suspensions were stained with the L/D fixable viability eFluor 450 (eBioscience, #65-0863-14) in the dark for 30 min at RT. After washing, murine Fc receptor (FcR) blocking reagent (Miltenyi Biotec, #130-092-575) was added to prevent nonspecific labelling.

To analyze the phenopype of CAR-TILs, tumor samples were resuspended in 100 µL of FACS buffer containing a specific amount of previous titrated antibodies detailed in the section ***Flow cytometry and antibodies.*** For intracellular staining, samples were fixed and permeabilized using the True Nuclear and Foxp3/Transcription Factor Staining Buffer set (ThermoFisher Scientific, #00-5523-00), according to the manufacturer's instructions.

### Immunohistochemistry analyzes

Immunohistochemical studies were performed on formalin-fixed, paraffin-embedded tissue sections according to standard protocols. Paraffin sections slides were developed using immunostainer Bond Max (Leyca Biosystems). Low pH retrieval was done (Bond ER1 Buffer solution, Leyca Biosystems) for 20 min followed by 1 h incubation with primary antibody (ErbB2 (HER-2) Recombinant Rabbit Monoclonal Antibody (clone SP3; Invitrogen, #MA5-14509)) or CD8 (CD8 Rabbit Monoclonal (clone SP16; (Invitrogen, #MA5-14548)) and incubated during 10 minutes of anti-rabbit Polymer of Bond Refine Polymer (Leyca Biosystems). 3'-diaminobenzidine (DAB) was used as a chromogen. Then, 12 min incubation with Harris Hematoxylin (Leyca Biosystems) was carried out as a nuclear staining screening. Images were obtained using a Nikon Eclipse E600 inverted microscope and an Olympus DP72 camera.

### Bulk RNA-sequencing (RNA-seq) and analysis

Total RNA was isolated from CAR-TILs for RNA-sequencing analysis using the RNeasy Mini kit (Qiagen, #74104) as per manufacture's protocol. Samples were stored at -80°C until they were used for downstream applications to preserve integrity. Libraries were prepared using standard protocols, and cDNA was sequenced using Illumina NextSeq2000 platform with 1x50-bp paired-end reads per sample. RNA-seq reads were mapped against GRCh38 human reference genome combined with the chimeric protein sequence, with STAR/2.7.8a5 and using ENCODE parameters. Gene quantification was performed with HTSeq v0.11.26.

Differential expression analysis between conditions from RNA-seq data were explored using limma v3.42.3 R package. To perform enrichment analysis, two databases were used: Gene-Set Enrichment Analysis (GSEA) (Subramaniam A, et al. 2005: A knowledge-based approach for interpreting genome-wide expression profiles. Proc Natl Acad Sci U S A. 2005;102(43):15545-50) and Ingenuity Pathway Analysis (IPA). GSEA was performed with the C7 ImmunoSigDB database collection using the t statistic metric obtained from the differential expression analysis for ranking genes. The following parameters were applied to the analysis: 1000 permutations, no collapse, no filter. IPA (Qiagen) was performed with the IPA software, which identifies the most significantly perturbed biological pathways in a given set of genes. The pathways identified are included in the Ingenuity Knowledge Base, a vast curated database of biological and chemical interactions (Reference: https://digitalinsights.qiagen.com/products-overview/discovery-insights-portfolio/analysis-and-visualization/qiagen-ipa/). Two types of analysis were performed with IPA software: pathway analysis and upstream regulator analysis.

### Design, generation and analysis of a custom sgRNA library for CRISPR/Cas9-mediated knockout in T cells

A custom library of sgRNAs targeting 300 gene candidates was ordered and produced by Cellecta (California, United States). After construction of the library, 24 constructs were isolated for full-insert Sanger sequencing to confirm the configuration of the sgRNA expression cassette and ensure correct insertion. All guide sequences were also sequenced by NGS to confirm full representation of the oligo pool. To prepare the DNA libraries for Amplicon-sequencing, we amplified DNA using the high-fidelity DNA polymerase KAPA HiFi included in the KAPA HiFi HotStart ReadyMix PCR Kit (Roche, #KK2602). The amplification process was performed in an Applied Biosystems 2720 Thermal Cycler and PCR products were confirmed by electrophoreses in agarose gel.

Libraries were prepared using standard protocols. DNA libraries were sequenced on Illumina's NextSeq500 using a custom sequencing primer. MAGeCK algorithm10 was used to analyze the results from the Amplicon-Seq.

### Soft clustering analysis of gene expression data

To assess the kinetics of gene expression, soft clustering analysis was used using the fuzzy c-means algorithm 8 with Mfuzz package (v2.52.0) in R For the fuzzy c-means algorithm, the parameter fuzzifier *m* was set using *mestimation* function based on the standardized data. This parameter controls how soft or fuzzy the clustering will be based on a specific dataset. To determine the appropriate number of clusters for our dataset, *cselection* function was used to calculate the minimum distance between clusters and the partition coefficient for a number of clusters. The optimal number of clusters was finally set based on the assessment of their biological relevance.

### Data representation and statistical analysis

Schematic diagrams were generated using Biorender.com.

Hierarchical clustering was performed by calculating euclidean distances between samples and using the hclust function. Boxplot of sgRNA counts in samples were performed using boxplot function. RStudio 2021.09.0+351 for Windows was used.

Overlaps between signatures were performed with Venn diagrams using the online tool: https://bioinformatics.psb.ugent.be/webtools/Venn/

Statistical parameters, including number of events quantified, standard deviation and statistical significance, are reported in the figures and in the figure legends. All statistical analyzes were performed using GraphPad Prism v9.4.1 (GraphPad Software Inc.). For comparisons of two groups, paired or unpaired two-tailed t-tests were used. One-way analysis of variance (ANOVA) with Tukey post hoc test was used for the comparison of three or more groups in a single condition. For the analysis of multiple groups, a two-way ANOVA test with Sidak or Tukey's multiple comparisons correction were performed. Asterisks indicate statistical significance as follows: *p<0.05; **p<0.01; ***p<0.001; ****p<0.0001; ns, not significant.

### Example 1. - Results

### HER2-28Z CAR-T cells display a narrow therapeutic window in a model of SKOV3 xenograft tumors and responding tumors eventually escape

To study the T cell-intrinsic mechanisms that give rise to CAR-T cell dysfunction, we developed an *in vivo* model of xenograft tumors that recapitulates disease recurrence in absence of antigen modulation. We used human CAR-T cells targeting the tumor-associated antigen HER2 with low affinity. This CAR contains a mutated version of the trastuzumab-based 4D5 scFv with a -2000-fold reduced affinity, named 4D5.5, which can discriminate between tumor cells expressing high levels of HER2 and normal tissues expressing physiological levels of the same antigen. We used a CAR of second-generation design, featuring a CD28 costimulation and a CD3z endodomain under the control of the human EF1a promoter. Analysis of CAR expression by flow cytometry confirmed that the anti-HER2-28Z CAR was efficiently expressed in the T cell membrane (**Figure 1A**). To stablish the *in vivo* model, NOD-SCID-Gamma (NSG) animals were engrafted with SKOV3 tumor cells and treated intravenously with a single dose of 2x10⁶ HER2-28Z CAR-positive T cells, or CAR-T cells targeting CD19 as control (**Figure 1B**). Despite the initial antitumor response, tumors were not eliminated and eventually escaped to therapy (**Figure 1C**).

To understand the mechanism of tumor escape, we isolated tumor infiltrating T cells (CAR-TILs) at two different phases: 1) early after T-cell infusion (day 8), in a phase where T-cell trafficking and activation leads to tumor regression (named as "Effective phase") and 2) at a second phase in which persisting antigen leads to loss of T-cell functions and tumor progression (named as "Dysfunctional phase", > day 21). We found that T-cells expanded in numbers in the animals, and tumors at the dysfunctional phase were heavily infiltrated with T-cells (**Figure 1D**) and still expressed the HER2 antigen (**Figure 1E**), excluding loss of CAR-T cell persistence or antigen loss as contributors to tumor escape in this model.

### CAR-TILs isolated at dysfunctional phase express phenotypic markers of T cell exhaustion and lose proliferative capacity

To assess whether CAR-TILs isolated from SKOV3 tumors that escape to therapy recapitulate features of exhausted T cells, we analyzed the inhibitory marker PD-1 and Ki67 proliferation marker. In dysfunctional CAR-TILs, PD-1 expression further increased by 1.5-fold compared to effective CAR-TILs (**Figure 2A**). In contrast, Ki67 was only expressed at high levels in the pre-infusion product and in CAR-TILs at the effective phase, while its expression decreased at the dysfunctional phase (**Figure 2B**). As observed for PD-1, CAR-TILs upregulated the expression of other checkpoints inhibitors, including LAG-3, TIM-3 and CD39, at effective phase compared to pre-infusion, and high expression was maintained during the dysfunctional phase (**Figure 2C**). These results indicate that TILs isolated at the dysfunctional phase recapitulated features of T cell exhaustion and lose proliferative capacity.

### CAR-TILs isolated from relapsing SKOV3 xenograft tumors display impaired effector functions in vitro

To evaluate the functionality of isolated CAR-TILs, overnight resting isolated CAR-TILs were cocultured with SKOV3 tumor cell lines and real-time cytotoxicity was monitored during 100 hours by xCELLigence. Also, cytokine secretion was measured in the supernatant of these effector-target cells cocultures after 24 hours of stimulation. Effective CAR-TILs efficiently eliminated tumor cells *ex vivo,* similar to pre-infusion HER2-CAR-T cells (**Figure 3A**). On the contrary, dysfunctional TILs displayed an impaired killing capacity (**Figure 3B**). Contrary to previous reports, this defective function was not reversible after overnight resting of CAR-TILs. In this line, effective but not dysfunctional CAR-TILs released IFNγ- when co-cultured with tumor cells *ex vivo* (**Figure 3C**). A more thorough characterization of the cytokine secretion profile of CAR-TILs after co-culture with SKOV3 cells showed a markedly decreased capacity to release factors implicated in cell-mediated killing of tumor cells, including perforin, granzyme A and B, and sFasL by CAR-TILs at dysfunctional phase. In contrast, dysfunctional CAR-TILs in co-culture with tumor cells tended to produce higher levels of IL-6, although differences were not statistically significant (**Figure 3E**). Similar results were obtained when analyzing the supernatants of CAR-TILs after stimulation with PMA/lonomycin (**Figure 3F**). Altogether indicates that CAR-T cells progressively lose their effector functions in the tumors.

We have recently reported that low affinity HER2 CAR-T cells display increased sensitivity to PD-L1-mediated inhibition and that PD-1/PD-L1 blockade restored effector functions of CAR-T cells. As T cell inhibition could be mediated by the PD-1/PD-L1 axis and considering the upregulation of PD-1 on dysfunctional CAR-TILs, we analyzed the effect of blocking the PD-1/PD-L1 axis in the dysfunctional CAR-TILs. Despite increased expression of PD-1, the addition of an anti-PD-L1 antibody (Durvalumab) failed to reinvigorate T cell functions (**Figure 3D**).

Altogether, these results suggest that after prolonged exposure to tumors, infused CAR-T cells display defective T cell functions that likely depend on T cell intrinsic mechanisms and that cannot be restored by inhibition of PD-1/L1 axis.

### Dysfunctional and Effective CAR-TILs show markedly distinct gene expression profiles

To understand the molecular mechanisms behind the loss of CAR-T effector functions, we performed bulk RNA-sequencing on CD8+ T cells from pre-infusion product, and from CAR-TILs isolated from SKOV3 tumors at effective and dysfunctional phases. We found 2566 significantly differentially expressed genes (DEG) between pre-infusion and effective, and 2472 genes between effective and dysfunctional (**Figure 4A**)**.** Notably, 4721 genes were differentially expressed between dysfunctional and pre-infusion. To elucidate the biological functions of the DEG identified, Ingenuity Pathway Analysis (IPA) was performed (**Figure 4B-****D**). The IPA software identifies the most significantly perturbed biological pathways included in the Ingenuity Knowledge Base, a vast curated database of biological and chemical interactions. To this end, we selected significantly upregulated genes between the dysfunctional vs pre-infusion and the effective vs pre-infusion comparisons. When analyzing upregulated genes from the dysfunctional vs preinfusion comparison, we observed an enrichment in signatures related to T cell dysfunction, including T cell exhaustion signaling pathway and senescence pathway (**Figure 4B**). Pathways referring to inhibitory checkpoint activation, (PD-1/PD-L1 cancer immunotherapy and CTLA-4 signaling in cytotoxic lymphocytes) were also upregulated. At the top of the list of enriched signatures in dysfunctional CAR-TILs, we also found natural killer-related pathways, which have been associated with T cell dysfunction.

### Design and validation of a sgRNA library for CRISPR/Cas9-mediated knockouts of gene candidates in CAR-T cells

The dysregulated transcriptional profile in dysfunctional CAR-TILs uncovered by the RNA-seq analysis suggests that a set of genes upregulated in this phase could have a key role in orchestrating CAR-T cell dysfunction. In this context, the ablation of specific genes could revert or delay CAR-T cell exhaustion and therefore enhance CAR-T antitumor efficacy *in vivo.* To test this, we designed a library of sgRNA for CRISPR/Cas9-mediated knockouts that allowed us to simultaneously test the impact of knocking out various genes in a single experimental setup. To select the genes for the library while preserving the different kinetics of gene upregulation, we selected (i) 100 genes of late upregulation (i.e. upregulated at dysfunctional phase as compared to effective and pre-infusion), (ii) 100 genes of early upregulation (i.e. upregulated at effective phase as compared to pre-infusion and maintained at dysfunctional phase), and (iii) 100 genes progressively upregulated from pre-infusion to dysfunctional (**Figure 5A**). Based on this list, we generated a custom library consisting of 4 sgRNAs targeting each of the candidate genes cloned into a lentiviral vector plasmid expressing GFP. In addition to sgRNA targeting candidate genes, control sgRNA was added to the library to validate our experimental approach. Negative control sgRNAs included: i) non-targeting sgRNAs that showed no complementary within the T cell genome and therefore are not expected to mediate DNA damage, and ii) intron-targeting and sgRNAs targeting safe harbor sites, which although they are not predicted to affect any protein-coding gene, they can induce Cas9-mediated double strand break and therefore are a control for DNA damage response. sgRNAs targeting essential genes for T cell survival (in this library: BCL2, HSPA9, TCF7, PAFA1HB1, HSPA5, CDC16 and GTF2B) were used as positive controls and were expected to be depleted after the KO. We also included sgRNAs targeting PD-1 and LAG-3 as their genetic ablation has been reported to have a positive impact on CAR-T cell performance.

Briefly, to generate a pool of genetically modified CAR-T cells using our custom library of sgRNAs, CAR-T cells were co-transduced with two lentiviral vectors (LV): the one encoding the HER2-CAR and the one for the sgRNAs included in our library (**Figure 5B**). The LVs for the library were used a low multiplicity of infection (MOI) to assure that each CAR-T cell will contain only one sgRNA and therefore will have only the knockout (KO) of one gene. On day 4 post-activation, T cells transduced with the library were separated by FACS-sorting and Cas9 protein was electroporated to induce CRISPR-Cas9-mediated knockouts in CAR-T cells. A pool of genome-edited CAR-T cells was then expanded *in vitro* and infused to mice bearing SKOV3 tumors. At day 20-28 after treatment, CAR-TILs expressing GFP were isolated from tumors by flow-sorting and genomic T cell DNA was used for amplicon sequencing. sgRNA abundance in T cells before KO and T cells after *in vivo* challenge was analyzed and compared by using the model-based Analysis of Genome-wide CRISPR/Cas9 Knockout (MAGeCK) method.

### Analysis of the CRISPR/Cas9 custom library enables the screening and selection of dysfunction-associated genes

To identify candidate genes whose KO enhanced CAR-T cell efficacy, we expected that T cells exhibiting the most potent antitumor effect would display elevated rates of proliferation and, consequently, greater persistence within tumors. In this context, sgRNA targeting genes that restrain CAR-T cell function should be enriched after the *in vivo* screen. The CRISPR library also included sgRNAs targeting essential genes for T cell survival as positive controls. We observed a depletion of positive control sgRNA following the screening, confirming the reliability of our CRISPR screen and ensuring that our results accurately reflect genes influencing CAR-T cell function.

Of the 300 initial candidate genes, the KO for 14 genes was significantly enriched (**Figure 6A** and **Table 3**). Of note, the ablation of PD-1 and LAG-3 was not positively enriched following our screening approach.

Since the 300 genes in the library consist of those significantly upregulated at the dysfunctional phase in the RNA-seq analysis, we sought to analyze how many of them overlap with published signatures of dysfunctional human TILs and CAR-T cells in cancer. 58 out of 300 genes were identified upregulated in the literature (**Figure 6B**) and from those, only 2 (ZC3H12C and ITGB8) were significantly enriched in our *in vivo* screen.

MAGeCK score along with the fold change of enrichment of sgRNAs, uncovered a ranking of enriched genes. Among the top ones, we shortlisted 5 candidate genes (ZC3H12C, UCP3, LILRB1, TG and ITGB8) based on at least two of the following criteria: (i) highest enrichment ranking using the MAGeCK software algorithm, (ii) at least two out of four sgRNAs positively enriched and (iii) previously identified in public signatures of dysfunctional human TILs and CAR-T cells. In subsequent experiments of gene candidate validation, we also included GPR25 as a control of a gene that was not significantly enriched after the *in vivo* screen.

**Table 3. Enriched gene knockouts after in vivo screen compared to T cells before KO identified using MAGeCK software on Amplicon-seq data.**

| **Gene** | **Ranking** |
|---|---|
| ZC3H12C | 1 |
| UCP3 | 2 |
| AHNAK2 | 3 |
| STON1 | 4 |
| MYH6 | 5 |
| LILRB1 | 6 |
| PTK7 | 7 |
| TG | 8 |
| INSRR | 9 |
| ADAM 12 | 10 |
| COL4A1 | 11 |
| ITGB8 | 12 |
| FN1 | 13 |
| ACTG2 | 14 |

### Candidate genes are grouped in three distinct patterns of expression in CAR-TILs

We assessed the expression kinetics of the 14 candidate genes across preinfusion, effective and dysfunctional phases using the soft clustering analysis on RNAseq data from the SKOV3 *in vivo* model. Although all candidate genes were upregulated at the dysfunctional phase compared to the preinfusion product, the 14 genes were grouped into three of the seven clusters (**Figure 7**). Cluster 1 contains genes that are progressively upregulated upon chronic antigen exposure, and contained ITGB8, LlLRB1 and ADAM12 candidates. These genes were upregulated in CAR-TILs shortly after infusion compared to the preinfusion product. At the dysfunctional phase, when CAR-TILs lose their effector functions, the expression of this gene group further increased, compared to effective CAR-TILs. The kinetic expression of these genes suggests a role of these genes during both early and late stages of CAR-T cell exhaustion. Cluster 6 contained ZC3H12C, UCP3, TG, INSRR, STON1, ACTG2 and FN1 candidates. In this cluster, gene expression was upregulated in CAR-TILs shortly after infusion to mice, compared to the preinfusion product. The high expression of this set of genes was maintained at the dysfunctional phase. This suggests that candidate genes in cluster 6 may play a role in the early stages of CAR-T cell exhaustion. Finally, PTK7, COL4A1, MYH6 and AHNAK2 genes grouped in cluster 7. These genes showed similar expression levels in the preinfusion product and effective CAR-TILs, but their expression increased in dysfunctional CAR-TILs. As a result, this set of genes may contribute to the later stages of exhaustion of CAR-T cells.

Overall, these results indicate that the 14 candidate genes can be grouped based on their expression patterns, and, consequently, whether they promote an early or a late exhaustion phenotype in CAR-T cells. This suggests that the grouped genes may play unique role in promoting or establishing CAR-T cell exhaustion.

### ZC3H12C, UCP3, TG and ITGB8 co-express with genes from the TNF-α signaling pathway.

To identify the mechanisms of action in which the candidate genes are involved, we implemented a pipeline based on the RNAseq data from the SKOV3 *in vivo* model, structured as: 1) a correlation analysis to identify genes that significantly co-expressed with each of the candidate genes, and 2) an enrichment analysis to determine the biological pathways in which these genes were involved. We first performed the correlation analysis in the dysfunctional samples using the significantly upregulated genes between the dysfunctional vs the preinfusion comparison. Then, we selected the genes that had a significant and positive correlation for each individual candidate gene separately. This resulted in 59 genes for ZC3H12C, 352 genes for UCP3, 344 genes for TG and 290 genes for ITGB8. Finally, for each gene set, we performed an enrichment analysis (**Figure 8**). The TNF-α signaling pathway emerged as common feature among the subsets of genes that co-expressed with all the four candidates, followed by the epithelial to mesenchymal transition pathway in UCP3, TG and ITGB8 subsets of genes. These results suggest that ZC3H12C, UCP3, TG and ITGB8 co-express with subsets of genes that share common mechanisms of action and point toward the TNF-α signaling via NF-kB pathway as a driver of CAR-T cell dysfunction.

### Genetic ablation of gene candidates ZC3H12C, UCP3, TG and ITGB8 enhances antitumor efficacy of CAR-T cells in SKOV3 solid tumors

To evaluate the antitumor effect of targeted KO candidate genes in CAR-T cells, we used the SKOV3 *in vivo* model mentioned previously. First, we generated engineered HER2-28Z CAR-T cells with disrupted expression of gene candidates ZC3H12C, TG or UCP3 using CRISPR/Cas9 technology. CAR-T cells knocked-out at the AAVS1 safe harbor site were also generated as control of inducing a double-strand break in a gene which deletion does not enhance or impair CAR-T cell function. LAG-3 was included as a positive control of proved antitumor efficacy in specific settings. Briefly, sgRNA targeting ZC3H12C, TG, UCP3, and LAG-3 or AAVS1 safe harbor site control were delivered as ribonucleoproteins 3 days after primary activation of HER2-28Z CAR-T cells. All groups showed comparable CAR expression on the T cell membrane (around 60%) (**Figure 9A**). KO efficiencies were comparable between groups, with a KO score between 60-80% (**Figure 9B**). NSG mice bearing large pre-established SKOV3 tumors were treated with a single intravenous dose of 2x10⁶ control T cells or KO-CAR T cells (**Figure 9C-D**). Control AAVS1-KO CAR-T cells induced only partial antitumor responses, and, in most cases, CAR-T therapy failed to control tumor growth. In contrast, ZC3H12C- and UCP3-KO CAR-T cells demonstrated significantly enhanced antitumor function (**Figure 9C**). After 45 days of CAR-T therapy, ZC3H12C-KO CAR-T cells induced 92% of complete responses and UCP3-KO CAR-T cells induced 42% of complete responses. Although not statistically significant, genetic ablation of TG showed a clear trend for improved CAR-T cell activity as compared to control CAR-T cells, with a 40% of complete responses (**Figure 9D**).

To test the other candidate gene, we generated CAR-T cells with disrupted expression of ITGB8, GPR25 or LAG-3 and control AAVS1-KO CAR-T cells. CAR expression (**Figure 9E**) and KO efficiencies (**Figure 9F**) were comparable between groups. The antitumor efficacy of CAR-T cells with KO for ITGB8 was evaluated in mice bearing SKOV3 tumors (**Figure 9G-H**). ITGB8-deficient CAR-T cells showed significantly enhanced antitumor activity as compared to control CAR-T cells, inducing 83% of complete responses. In contrast, the ablation of GRP25 failed to enhance the performance of CAR-T cells against SKOV3 solid tumors, similar to AAVS1-KO control. This demonstrates the effectiveness of the CRISPR library in identifying candidate genes whose KO enhances CAR-T cell function.

### LILRB1-KO CAR-T cells show high antitumor efficacy in an in vivo model of solid tumor

To evaluate the antitumor effect of LILRB1-KO CAR-T cells in mice bearing SKOV3 tumors, we generated CAR-T cells with disrupted expression of LILRB1 or LAG-3 genes, and control AAVS1 gene. KO efficiencies (**Figure 10A**) were comparable between LILRB1 and LAG-3 groups, with a score of approximately 60-65%. Likewise, KO-CAR-T cell groups showed comparable CAR expression in terms of percentage of the CAR-positive population (around 40-50%) (**Figure 10B**). LlLRB1-deficient CAR-T cells exhibited high antitumor activity, comparable to that observed in LAG-3-KO and AAVS1-KO groups. (**Figure 10C**).

### Genetic deletion of candidate genes ZC3H12C, UCP3, TG and ITGB8 improves CAR-T cell persistence in mice with SKOV3 tumors

Significant expansion and persistence of adoptively transferred tumor-specific T cells are key predictors of clinical efficacy in patients with cancer. Therefore, the persistence of HER2-CAR-T cells KO for AAVS1, ZC3H12C, UCP3, TG and ITGB8 in the peripheral blood of treated mice was assessed at different timepoints after CAR-T cell treatment (**Figure 11A-B**). ZC3H12C-KO CAR-T cells demonstrated significantly greater persistence than AAVS1-KO control CAR-T cells at both time points (5-fold increase at day 14, and 6-fold increase at day 21). The KO of UCP3 increased CAR-T cell persistence by 2-fold compared to control CAR-T cells at both time points (**Figure 11A**). Similarly, TG-KO CAR-T cells showed a 2- and 5-fold increased persistence versus control CAR-T cells (AAVS1 KO) at days 14 and 21, respectively. Also, ITGB8-KO CAR-T cells showed a 6- and 7-fold increased persistence versus control CAR-T cells at days 14 and 21, respectively (**Figure 11B**). Overall, these results demonstrate that the elimination of candidate genes in CAR-T cells can increase T-cell persistence in treated animals compared to control AAVS1-KO CAR-T cells.

### Validation of T cell persistence in mice with L55-HER2t tumors

We next sought to validate the increased T cell persistence of HER2-CART cells KO for the candidate genes in another *in vivo* model. For that, NSG mice bearing subcutaneous lung xenografts genetically modified to overexpress a truncated form of HER2 (HER2t) were treated with ZC3H12C-KO, UCP3-KO, TG-KO, ITGB8-KO, LILRB1-KO, LAG-3-KO, AAVS1-KO CAR+ T cells or untransduced T cells (UTD) as control (**Figure 12**). All candidate genes showed increased T cell persistence compared with the AAVS1-KO and LAG-3-KO CAR-T cell control groups.

### Deleting candidate genes ZC3H12C, TG and ITGB8 in CAR-T cells enhances survival in mice with SKOV3 tumors

We next aimed to assess the survival rate of mice bearing SKOV3 tumors and treated with CAR-T cells KO for candidate genes (**Figure 13A-B**). Mice treated with ZC3H12C-KO CAR-T or UCP3-KO CAR-T cells exhibited a higher survival rate compared to the AAVS1-KO control group (**Figure 13A**). Similarly, ITGB8-KO CAR-T cells showed a significantly higher survival rate compared to mice treated with AAVS1-KO CAR-T cells (**Figure 13B**). A similar trend was observed for the TG-KO CAR-T cell group. These results correlate with the previously observed reduction in tumor burden in mice treated with this targeted KO candidate genes CAR-T cells.

### Genetic deletion of ZC3H12C and UCP3 improves in vivo CAR-T cell efficacy and persistence in mice with Capan-2 xenografts

We next aimed to determine whether our observations in the *in vivo* model of SKOV3 tumors treated with anti-HER2-CAR T cells could be applied to a different model of solid tumors and to CAR-T cells targeting a distinct antigen. To assess that, we used a second-generation CAR containing CD28 and CD3ζ intracellular domains and a scFv targeting Mesothelin (named M11) with low-intermediate affinity obtained from WO2015090230A1.

We generated CAR-T cells with disrupted expression of ZC3H12C or UCP3 and control AAVS1-KO CAR-T cells. NSG mice bearing pre-established Capan-2 tumors were treated with a single dose of 2x10⁶ total control T cells (UTD) or KO-CAR-T cells (**Figure 14A-B**). Although control AAVS-1 KO CAR-T cells induced a substantial antitumor response in this model, ZC3H12C- and UCP3-KO CAR-T cells demonstrated significantly enhanced antitumor activity at the end of the CAR-T therapy (**Figure 14A**). Next, we analyzed T cell persistence in blood at two different timepoints. Genetic deletion of ZC3H12C or UCP3 genes in CAR-T cells resulted in a 3-fold or 2-fold increase in T cell persistence compared to AAVS1-KO CAR-T cells, respectively, at both time points (**Figure 14B**).

### ZC3H12C-KO CAR-T cells enhance the anti-tumor effect of an anti-CD19 41BB-based CAR.

To continue exploring the potential of eliminating ZC3H12C to enhance CAR-T cell efficacy in different models, we develop an artificial model of aggressive solid tumors expressing CD19. For that, we used ARI-0001 CAR-T cells, which is an academic CAR-T product developed by Hospital Clinic-IDIBAPS that has been approved by the Spanish regulatory authorities for the treatment of patients with r/r CD19+ B-cell ALL. ARI-0001 T cells express a second-generation CAR that contains the 41BB and CD3ζ intracellular domains. To assess the antitumor activity of the ZC3H12C-KO ARI-0001 CAR-T cells, NSG mice bearing subcutaneous lung xenografts genetically modified to overexpress CD19 were treated with control T cells (UTD) or ZC3H12C-KO, LAG-3-KO, AAVS1-KO ARI0001 CAR+ T cells (**Figure 15A-C**). While all animals treated with LAG3-KO CAR-T cells and AAVS1-KO CAR-T groups showed progressive disease, treatment with ZC3H12C-KO CAR-T cells induced complete responses in all treated animals (**Figure 15A**). Moreover, ZC3H12C-KO CAR-T cells showed a significantly greater persistence compared to LAG-3-KO and AAVS1-KO CAR-T cells (**Figure 15B**). By day 21, T cell persistence decreased in all groups, yet ZC3H12C-KO CAR-T cells still demonstrated significantly improved persistence (**Figure 15C**). Altogether, these results demonstrate that knocking out ZC3H12C can enhance the antitumor effect and persistence of T cells modified to express CARs with various specificities or costimulatory domain.

### Deletion of ZC3H12C enhances in vitro effector functions of CD28-based anti-HER2 CAR-T cells upon chronic antigen stimulation.

We next aimed to characterize the mechanism through which the genetic deletion of ZC3H12C enhances CAR-T antitumor effect. To this end, we performed a repeated antigen stimulation assay *in vitro,* where CAR-T cells were stimulated with HCC1954 HER2+ breast cancer cells every 3-4 days. Genetic deletion of ZC3H12C increased CAR-T cell proliferation over time compared to AAVS1-KO CAR-T cells (**Figure 16A-B**). We also evaluated the cytotoxic capacity of restimulated CAR-T cells using a real-time assay. While no differences in cytotoxicity between ZC3H12C-KO and control AAVS1-KO CAR-T cells were detected after the first stimulation with tumor cells (Day 1) (**Figure 16C**), control AAVS1-KO CAR-T cells progressively lost their ability to eliminate tumor cells in vitro after repeated stimulations, whereas ZC3H12C-KO CAR-T showed improved killing abilities.

To further confirm these results, SKOV3-bearing mice were treated with AAVS1-KO or ZC3H12C-KO HER2-CAR-T cells and CAR-TILs were isolated three weeks after treatment. Isolated tumor infiltrating ZC3H12C-KO CAR-TILs showed superior cytotoxic capacity than AAVS1-KO CAR-TILs (**Figure 16D**).

### ZC3H12C-KO CAR-T cells show enhanced cytokine production upon chronic antigen stimulation.

ZC3H12C belongs to an RNA-binding protein (RBP) family involved in restricting T cell inflammatory activity by binding to specific mRNAs encoding inflammatory factors like costimulatory receptors, transcription factors, and cytokines such as TNF-α. To assess if the disruption of ZC3H12C enhanced CAR-T cytokine production, we analyzed cytokine levels following a chronic stimulation assay where ZC3H12C-KO and AAVS1-KO CAR-T cells were stimulated for 24h with fresh HCC1954 tumor cells every 3-4 days (**Figure 17A-C**). ZC3H12C-KO CAR-T cells showed increased production of TNF-α, IFN-γ and IL-2, compared to AAVS1-KO CAR-T cells on day 1 of the restimulation assay. This improvement was sustained over time, demonstrating the regulation of cytokine production by ZC3H12C in the context of CAR-T cells targeting HER2 in solid tumors. Interestingly, ZC3H12C-KO CAR-T cells showed an increased percentage of polyfunctional T cells producing both IFN-γ and TNF-α (**Figure 17D**).

To further confirm these results, SKOV3-bearing mice were treated with AAVS1-KO or ZC3H12C-KO HER2-CAR-T cells. CAR-TILs were isolated three weeks after treatment and cocultured ex vivo with SKOV3 tumor cells. ZC3H12C-KO CAR-TILs showed a superior capacity to secrete factors implicated in cell-mediated killing of tumor cells, compared to AAVS1-KO CAR-TILs. We observed significantly higher levels of perforin, granzyme B, IL-6 and FasL. Also, ZC3H12C-KO CAR-TILs produced more than a 2-fold increase in TNF-α, IFNγ and granulysin levels (**Figure 17E**). These results demonstrate that targeted ZC3H12C deletion enhances CAR-T cell proliferation, cytotoxicity and cytokine release upon chronic antigen exposure, both *in vitro* and *in vivo,* leading to superior antitumor effects in vivo.

### The genetic deletion of other upregulated genes at the dysfunctional phase does not enhance antitumor efficacy in vivo

Alongside the *in vivo* CRISR-based screen, we developed another strategy to identify possible genes whose KO could enhance CAR-T cell function. In this case, we performed a soft clustering analysis on RNAseq data from the SKOV3 in vivo model. Through this analysis, we identified several clusters with distinct kinetic patterns of gene expression during the effector, preinfusion, and dysfunctional phases. Specifically, we identified three clusters that exhibited an upregulated gene expression pattern during the dysfunctional phase. To prioritize genes for the individual analysis within the three identified clusters, we focused on i) genes that were significantly upregulated in the dysfunctional phase in the differential analysis and ii) genes included in previously published gene signatures of dysfunctional human TILs and CAR-T cells in cancer. This approach revealed 7 candidates: KRT86, KRT81, DUSP2, CST7, ZFP36, MYO7A and VCAM1 (**Figure 18A**). Gene expression kinetics was validated by RT-PCR in the pre-infusion, effective, and dysfunctional phase, confirming that the selected genes were significantly upregulated in the dysfunctional phase of CAR-T cells *in vitro* (**Figure 18B**). To obtain individual KOs, sgRNA targeting the candidates, and AAVS1 as safe harbor site control, were delivered as ribonucleoproteins three days after primary activation of HER2-28Z CAR-T cells. All groups obtained comparable KO efficiencies (between 60-80%) (**Figure 18C**) and similar CAR expression (around 50%). In an *in vitro* repeated antigen stimulation assay, where CAR-T cells were stimulated with HCC1954 HER2+ expressing tumor cells every 3-4 days, we observed similar population doublings between the KO-CAR-T cells and AAVS1-KO CAR-T cells (**Figure 18D**), indicating that KO-CAR-T cells were able to expand similarly to control T cells. To assess the antitumor activity of selected individual KO CAR-T cells, NSG mice were implanted with subcutaneous SKOV3 xenografts. After the tumors were established, animals were treated with a single injection of 2x10⁶ of AAVS1-KO CAR-T cells as control, or DUSP2-, CST7-, ZFP36- and MYO7A-KO CAR-T cells (**Figure 18E-F**). While treatment with all CAR-T groups reduced tumour growth compared to treatment with control T-cells (UTD), knocking out candidate genes did not enhance or impair the antitumor effect of convectional HER2-CART cells, and the majority of tumors escaped CAR-T cell therapy.

### The double genetic ablation of ZC3H12C with the other gene candidates exhibit enhanced cytokine production upon stimulation with SKOV3 tumor cells

Next, we aimed to investigate whether simultaneously disrupting two of our candidate genes could further enhance CAR-T cell function. To test this, we generated CAR-T cells with double KO of the candidate genes. Specifically, we combined the KO of ZC3H12C with UCP3, ITGB8, TG, LILRB1 or PD-1 genes. The double KO comprising ZC3H12C and AAVS1 genes was also generated as a control of two DNA double strand breaks. In the primary *in vitro* expansion, we did not observe any differences in the population doublings between single and double KO CAR-T cells (**Figure 19A**). Gene disruptions were comparable when knocking out ZC3H12C alone or in a double KO combination, as well as for the second gene in the double KO, achieving approximately 80% KO efficiency for both genes (**Figure 19B**). At the end of the ex *vivo* T cell expansion, we did not observe differences in the percentage of live T cells or the percentage of CAR expression (**Figure 19C-D**). To evaluate the functionality of double KOs, CAR-T cells were cocultured with SKOV3 tumor cells for 24 h, and cytokine secretion was measured (**Figure 19E-F**). Double KOs of candidate genes, including the combination of ZC3H12C with PD-1, in HER2-CART cells resulted in increased cytokine production compared to the single KO AAVS1 (**Figure 19E-G**). Overall, these results suggest that double KO are feasible, do not have a negative impact CAR-T cell expansion and result in enhanced effector T cell functions.

## Claims

1. An immune T cell expressing an antigen receptor, wherein the immune T cell has been modified to have reduced or eliminated the function and/or the expression of one or more of the target proteins encoded by one or more target genes selected from the list consisting of zinc finger CCCH-type containing 12C (ZC3H12C), uncoupling protein 3 (UCP3), thyroglobulin (TG), and integrin subunit beta-8 (ITGB8), and
wherein said reduction or elimination of the function and/or the expression of one or more of the target proteins is calculated with respect to the same immune T cell without having said modification.

2. The immune T cell according to claim 1, wherein the immune T cell has been modified to have reduced or eliminated the function and/or the expression of the ZC3H12C target protein.

3. The immune T cell according to claim 1 or 2, wherein the immune T cell has been modified to have reduced or eliminated the function and/or the expression of one or more target proteins selected from the group consisting of UCP3, TG, and ITGB8.

4. The immune T cell according to claims 2 or 3, wherein the immune T cell has been modified to have reduced or eliminated the function and/or the expression of the ZC3H12C target protein, and one or more target proteins selected from the group consisting of UCP3, TG, and ITGB8.

5. The immune T cell according to claims 1 to 4, wherein the immune T cell has been modified to have reduced or eliminated the function and/or the expression of the ZC3H12C target protein and one more of the proteins selected from the group consisting of PD-1 and LAG-3.

6. The immune T cell according to claims 1 to 5, wherein the immune T cell has been modified to have eliminated the expression of the target protein, preferably wherein the modification is a genetic modification; further preferably wherein the genetic modification is selected from the group consisting of: an insertion, a frameshift mutation, a missense mutation, a deletion, a knock-in, a knock-down, or a knock-out of the gene or part of the gene, and a deletion of the entire target gene encoding for the target protein.

7. The immune T cell according to claims 1 to 6, wherein the immune T cell is a CD4⁺ T cell, a CD8⁺ T cell or a CD4⁺/CD8⁺ T cell.

8. The immune T cell according to claims 1 to 7, wherein the antigen receptor is a chimeric antigen receptor (CAR) comprising:
- an antigen-binding domain;
- a transmembrane domain, preferably a CD8 or CD28 transmembrane domain;
- an intracellular signaling domain, preferably a CD3ζ endodomain; and
- optionally, a CD28 or a 4-1BB costimulatory domain.

9. The immune T cell according to claims 1 to 8, wherein antigen receptor specifically binds to an antigen selected from the group consisting of HER2, CD19 and mesothelin.

10. An *in vitro* or *ex vivo* method of producing an immune T cell, the method comprising the steps of:
(a) introducing an antigen receptor into an immune T cell and
(b) modifying the immune T cell to reduce or eliminate the function and/or the expression of one or more of the target proteins encoded by the target genes ZC3H12C, UCP3, TG, or ITGB8, wherein the reduction or elimination the function and/or the expression is calculated with respect the same immune T cell without said modification,
wherein steps (a) and (b) may be performed in any order.

11. An immune T cell obtained or obtainable from the method according to claim 10.

12. A pharmaceutical composition comprising the immune T cell according to any one of claims 1 to 9 or 11, and a pharmaceutically acceptable excipient.

13. An immune T cell according to claims 1 to 9 or 11, or the pharmaceutical composition according to claim 12, for use in therapy.

14. The immune T cell according to claims 1 to 9 or 11, or the pharmaceutical composition according to claim 12, for use in the treatment of cancer.

15. The immune T cell for use according to claim 14, wherein the antigen receptor specifically binds to an antigen selected from the group consisting of: HER2, CD19 and mesothelin, and wherein the cancer is selected from the group consisting of: an HER2-positive cancer, a CD19-positive cancer and a mesothelin-positive cancer, respectively.
